# EUROPEAN PATENT SPECIFICATION

(11) **EP 3 207 169 B1**
(45) Date of publication and mention of the grant of the patent: **01.07.2020**
(21) Application number: 15850902.6
(22) Date of filing: 13.10.2015
(51) Int. Cl.: C40B 50/06, C40B 40/06, C12Q 1/68

(54) **MATE PAIR LIBRARY CONSTRUCTION**
PAARTEILBIBLIOTHEKKONSTRUKTION
CONSTRUCTION DE BANQUES "MATE PAIR"

(30) Priority: 14.10.2014 WO PCT/CN2014/088542; 26.11.2014 CN 201410698443
(43) Date of publication of application: 23.08.2017
(73) Proprietor: MGI Tech Co., Ltd., Yantian District Shenzhen Guangdong 518083 (CN)
(72) Inventor: JIANG, Yuan, Shenzhen Guangdong 518083 (CN); DRMANAC, Radoje, Los Altos Hill, California 94022 (US); HUROWITZ, Evan, Mountain View, California 94040 (US); ALEXEEV, Andrei, Woodland, California 95776 (US); ZHAO, Xia, Shenzhen Guangdong 518083 (CN); RUAN, Jie, Shenzhen Guangdong 518083 (CN)
(74) Representative: Hamer, Christopher K.
(86) International application number: PCT/CN2015/091859
(87) International publication number: WO 2016/058517

(56) References cited:
- WO-A1-2012/044847
- WO-A2-2010/003153
- CN-A- 1 230 226
- CN-B- 102 296 065
- US-A1- 2011 033 854
- US-A1- 2012 329 678
- Applied Biosystems: "Applied Biosystems SOLiD 4 system Library Preparation Guide", , 1 April 2010 (2010-04-01), XP055444333, Retrieved from the Internet: URL:http://tools.thermofisher.com/content/ sfs/manuals/SOLiD4_Library_Preparation_man .pdf [retrieved on 2018-01-25]
- EVA C BERGLUND ET AL: "Next-generation sequencing technologies and applications for human genetic history and forensics", INVESTIGATIVE GENETICS, BIOMED CENTRAL LTD, LONDON, UK, vol. 2, no. 1, 24 November 2011 (2011-11-24), page 23, XP021094353, ISSN: 2041-2223, DOI: 10.1186/2041-2223-2-23

## Description

### BACKGROUND OF THE INVENTION

Large-scale genomic sequence analysis is a key step toward understanding a wide range of biological phenomena. The need for low-cost, high-throughput sequencing and re-sequencing has led to the development of new methods for generating libraries of target nucleic acids, as well as new approaches to sequencing that employ parallel analysis of multiple nucleic acid targets simultaneously. However, there remains a need for methods and compositions that increase the efficiency of the process for generating libraries of nucleic acid targets.

### BRIEF SUMMARY OF THE INVENTION

Provided herein are novel ligation methods that are referred to herein as "3' branch ligation" in which a double stranded target polynucleotide is ligated to a 3' branch adapter. The target polynucleotide comprises a ligation site comprising a 3'-hydroxyl selected from the group consisting of a nick, a gap, and a 5' overhang; and the 3' branch adapter comprises a 5' blunt end comprising a 5'-phosphate and a nonligatable 3' end. In these methods the target polynucleotide is contacted with the 3' branch adapter polynucleotide in the presence of a ligase under conditions suitable for ligation at the ligation site of the 3'-hydroxyl group of the target polynucleotide and the 5'-phosphate of the 5' blunt end of the adapter.

According to one embodiment of such 3' branch ligation methods, the 5' blunt end of the 3' branch adapter comprises a 5' terminus comprising the 5'-phosphate and a 3' terminus that is blocked from ligation by a blocking group, e.g., a dideoxynucleotide. According to another embodiment, the 3' end of the 3' branch adapter is protected from self-ligation by a 3' overhang or a ligation blocking group, e.g., a dideoxynucleotide or a 3'-phosphate group. According to another embodiment, the ligation site is a nick, the method comprising treating the target polynucleotide with an enzyme with 5' exonuclease activity to remove one or more nucleotides at the nick to produce a gap. According to another embodiment, the the ligation conditions comprise an amount of PEG or SSB protein or a combination thereof that is effective to detectably increase ligation of the 3' branch adapter to the target polynucleotide at the ligation site.

The 3' branch ligation methods of the invention have a number of applications. One such application is in the context of polynucleotide library construction.

Thus, according to another embodiment of the invention, methods are provided for making a mate pair polynucleotide library. Such methods comprise: providing a plurality of double-stranded target polynucleotides; producing circular constructs, each comprising a target polynucleotide, a first adapter, and a nick or gap in the first adapter; performing controlled nick translation (for example, controlled nick translation, including without limitation ntCNT and ttCNT) to produce nick translation products, each comprising the target polynucleotide, the first adapter, and a nick or gap a first selected distance within the target polynucleotide; performing 3' branch ligation to ligate a 3' branch adapter to each nick translation product at the nick or gap to produce gap ligation products; performing controlled primer extension to produce primer extension products by hybridizing a primer to the 3' branch adapter of the gap ligation products and extending the primer a second selected distance within the target polynucleotides; and adding a 5' adapter to a 5' end of the primer extension products to produce a mate pair library, each member of the library comprising: the 5' adapter, a first end portion of a target polynucleotide, the first adapter, a second end portion of the target polynucleotide, and the 3' branch adapter.

According to one embodiment of such library construction methods, the first adapter comprises two half adapter arms, and the method comprises ligating to each end of the target polynucleotides a half adapter arm of the first adapter to produce a ligation product; and ligating the half adapter arms together to produce the circular construct.

According to another embodiment, the first adapter comprises one or more uracil residues, and the method comprises excising said one or more uracil residues to produce the nick or gap in the first adapter.

According to another embodiment, the method comprises denaturing the gap ligation products to produce linear single strands and hybridizing the primer to the linear single strands.

Such library construction methods may be adapted for use in sequencing by a number of methods, including, for example and without limitation, cPAL sequencing and sequencing by synthesis According to one embodiment, the mate pair library is a double-stranded mate pair library and the method comprises producing single strands from the mate pair library and ligating ends of the single strands to produce single-stranded library circles. Such library circles may be amplified by rolling circle replication to produce DNA nanoballs, which may be disposed in an array on a solid support to produce a DNA nanoball array. According to another embodiment, the mate pair library is a double-stranded mate pair library, and the method comprises: producing single strands from the mate pair library; disposing the single strands on a surface of a solid support in an array; and amplifying the single strands on the array to produce an amplified array, for example, by bridge PCR.

According to another embodiment of the invention, mate pair polynucleotide libraries are provided that are made by any of the methods described above.

According to another embodiment, kits are provided for constructing a mate pair polynucleotide library for performing such library construction methods, such kits comprising: 5' and 3' half adapter arms of a first adapter; a 3' branch adapter; a 5' adapter; and instructions for use. According to one embodiment, at least one of said 5' and 3' half adapter arms of said first adapter comprises at least one uracil residue. According to another embodiment, such kits comprise a single stranded splint oligonucleotide. According to another embodiment, such kits of comprise one or more members of the group consisting of: a uracil-excising enzyme; a DNA ligase; and a DNA polymerase.

### BRIEF DESCRIPTION OF THE DRAWINGS

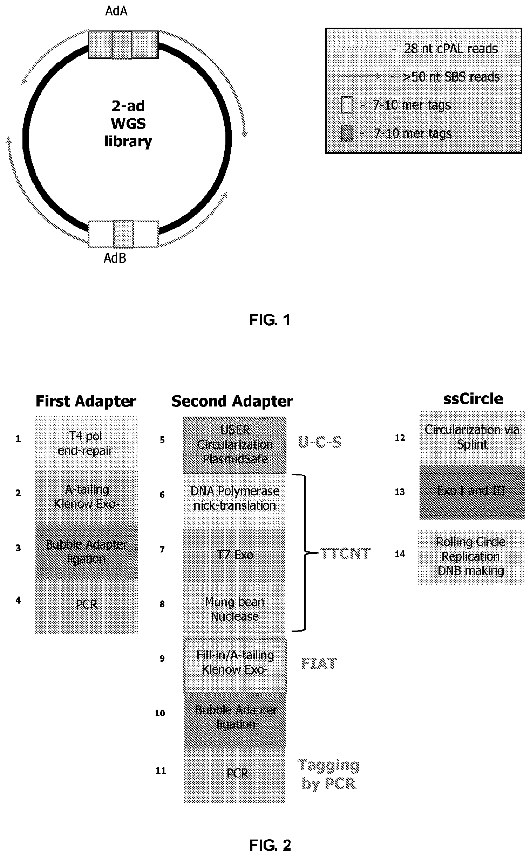

**FIG. 1****. Schematic of two-adapter library configuration.** A two-adapter library, comprising a first adapter ("AdA") and a second adapter ("AdB") can be configured for different applications. For example, a two-adapter library as depicted can be used for sequencing applications utilizing combinatorial probe anchor ligation (cPAL) chemistry, for sequencing applications utilizing sequencing by synthesis (SBS) chemistry, or for sequential sequencing by cPAL and SBS chemistries. These applications can be used, for example, in whole genome sequencing or in whole exome sequencing.
**FIG. 2****. Exemplary flow chart of library construction** - **ttCNT/Exo.** A flow chart for constructing a library in which the first adapter and the second adapter are bubble adapters is shown. Input DNA is added at step 1 and is modified in steps 1 and 2 in preparation for ligation with the first adapter (step 3). The ligation product is amplified by PCR (step 4). The amplification product is subjected to a "USER-Circularization-PlasmidSafe" (U-C-S) process (step 5) that results in the formation of a dsDNA construct having a gap in each strand. A time and temperature controlled nick translation ("ttCNT") reaction is performed on the dsDNA construct (steps 6-8), then the resulting product is end-repaired (step 9) in preparation for ligation with the second adapter (step 10). The ligation product is amplified by PCR (step 11). The amplification reaction can include adding a barcode tag into the second adapter sequence by PCR. Single-stranded circular DNA constructs are formed from the amplification product by circularizing the amplification product in the presence of a splint oligonucleotide (steps 12-13). The ssDNA circular constructs can then be amplified by Rolling Circle Replication to form DNA nanoballs (DNBs).

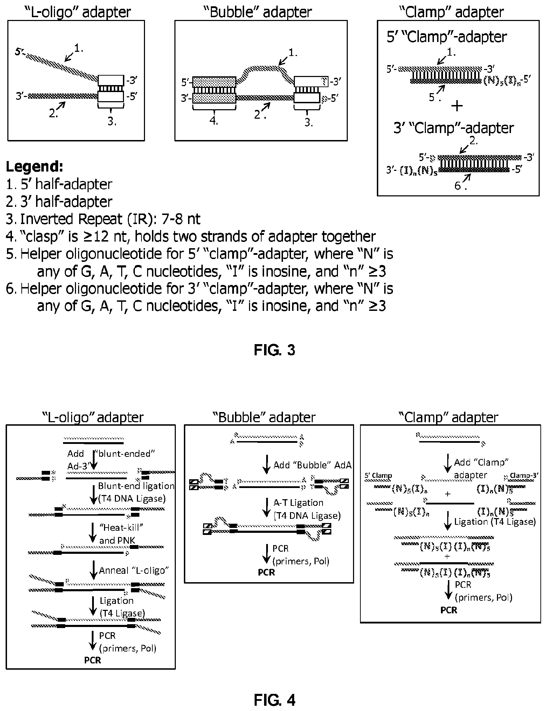

**FIG. 3****. Comparison of structures of "bubble," "L-oligo," and "clamp" adapters.** *Left panel:* Structure of an L-oligo adapter. *Middle panel:* Structure of a bubble adapter. *Right panel:* Structure of a clamp adapter. Legend: 1= 5' half-adapter (in red; also referred to herein as the first oligonucleotide); 2= 3' half-adapter (in blue; also referred to herein as the second oligonucleotide); 3= inverted repeat (IR) sequence of 7-8 nt; 4= clasp region of ≥12 nt that holds the two oligonucleotides together. 5= helper oligonucleotide for 5' clamp adapter, where "N" is any of G, A, T, or C nucleotides, "I" is inosine, and "n"≥3. 6= helper oligonucleotide for a 3' clamp adapter, where "N" is any of G, A, T, or C nucleotides, "I" is inosine, and "n"≥3.
**FIG. 4****. Overview of exemplary methods for attaching L-oligo, bubble, and clamp adapters to a DNA fragment.** *Left panel:* Exemplary method of ligating an L-oligo adapter to a DNA fragment. The second oligonucleotide (blue) of the L-oligo adapter is ligated to a dephosphorylated blunt-ended DNA fragment in the presence of a helper oligonucleotide having a 3'-end modification using T4 DNA ligase. After ligation, a heat-kill step inactivates the ligase and T4 PNK is added to phosphorylate the 5' ends of the ligation product. The first oligonucleotide (red) of the adapter is annealed to the phosphorylated ligation product using T4 DNA ligase. The resulting ligation product is then amplified by PCR. *Middle panel:* Exemplary method of ligating a bubble adapter to a DNA fragment. The first oligonucleotide (red) and the second oligonucleotide (blue) of the bubble adapter are annealed and ligated to a 5'-phosphorylated, 3' dA-tailed DNA fragment using T4 DNA ligase to form a double-stranded construct comprising the DNA fragment flanked on both sides by a duplex of the adapter oligonucleotides. The resulting ligation product is then amplified by PCR. *Right panel:* Exemplary method of ligating a clamp adapter to a DNA fragment. The first oligonucleotide (red) and the second oligonucleotide (blue) of the clamp adapters are ligated to single-stranded and 5' phosphorylated DNA fragments in the presence of helper oligonucleotides and T4 DNA ligase. The helper oligonucleotides have either a 5' or 3' single-stranded overhang consisting of the sequence (N)₅(I)ₙ. The resulting construct is a single-stranded linear DNA fragment flanked on both sides by a duplex comprising the first and second adapter oligonucleotide and a corresponding helper oligonucleotide. The resulting ligation product is then amplified by PCR.

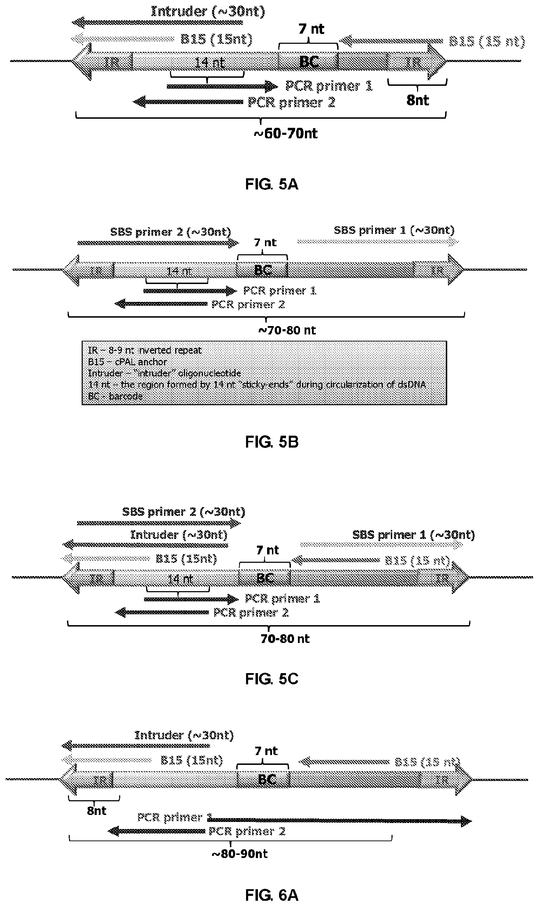

**FIG. 5****. Exemplary adapter architecture for first adapter for sequencing by cPAL and/or SBS.** (A)-(C) Exemplary depictions of a first bubble adapter or a first L-oligo adapter as viewed in the final mate-pair polynucleotide construct. (A) For sequencing by cPAL (reading target nucleotide sequence and barcodes in the 5' direction with cPAL), the first adapter includes two hybridization sequences for a cPAL anchor (B15) and a hybridization sequence for an intruder oligonucleotide. The first adapter has a length of about 60-70 bases. (B) For sequencing by SBS, the first adapter includes a hybridization sequence for a first SBS primer (SBS primer 1) that reads the target nucleotide sequence in the 3' direction, and a hybridization sequence for a second SBS primer (SBS primer 2) that reads barcodes in the 3' direction. The first adapter has a length of about 70-80 bases. (C) For sequencing by both cPAL and SBS, the first adapter includes two hybridization sequences for a cPAL anchor (B15), a hybridization sequence for an intruder oligonucleotide, a hybridization sequence for a first SBS primer (SBS primer 1), and a hybridization sequence for a second SBS primer (SBS primer 2). The target nucleotide sequence can be read in the 5' direction by cPAL or in the 3' direction by SBS with SBS primer 1. The barcodes can be read in the 5' direction by cPAL or in the 3' direction by SBS with SBS primer 2. The first adapter has a length of about 70-80 bases.

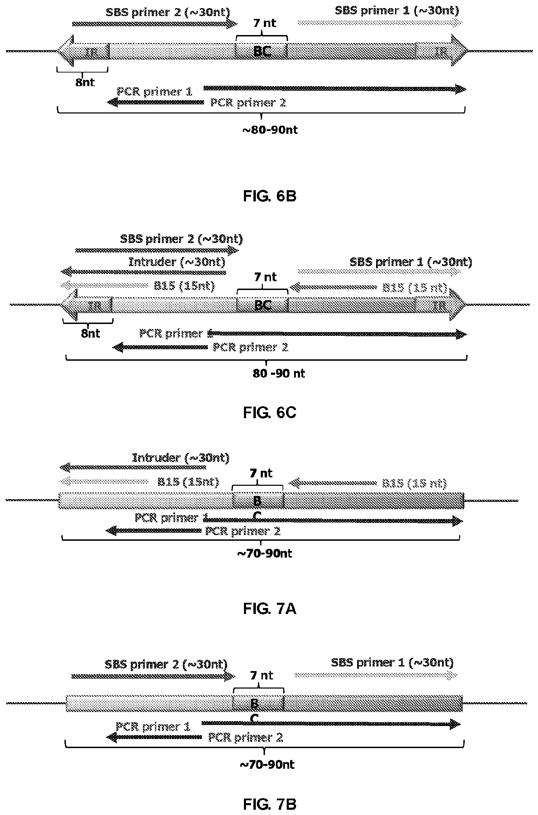

**FIG. 6****. Exemplary adapter architecture for second adapter for sequencing by** cPAL **and/or SBS.** (A)-(C) Exemplary depictions of a second bubble adapter or a second L-oligo adapter as viewed in the final mate-pair polynucleotide construct. (A) For sequencing by cPAL (reading target nucleotide sequence and barcodes in the 5' direction with cPAL), the second adapter includes two hybridization sequences for a cPAL anchor (B15) and a hybridization sequence for an intruder oligonucleotide. The second adapter has a length of about 80-90 bases. (B) For sequencing by SBS, the second adapter includes a hybridization sequence for a first SBS primer (SBS primer 1) that reads the target nucleotide sequence in the 3' direction, and a hybridization sequence for a second SBS primer (SBS primer 2) that reads barcodes in the 3' direction. The second adapter has a length of about 80-90 bases. (C) For sequencing by both cPAL and SBS, the second adapter includes two hybridization sequences for a cPAL anchor (B15), a hybridization sequence for an intruder oligonucleotide, a hybridization sequence for a first SBS primer (SBS primer 1), and a hybridization sequence for a second SBS primer (SBS primer 2). The target nucleotide sequence can be read in the 5' direction by cPAL or in the 3' direction by SBS with SBS primer 1. The barcodes can be read in the 5' direction by cPAL or in the 3' direction by SBS with SBS primer 2. The second adapter has a length of about 80-90 bases.

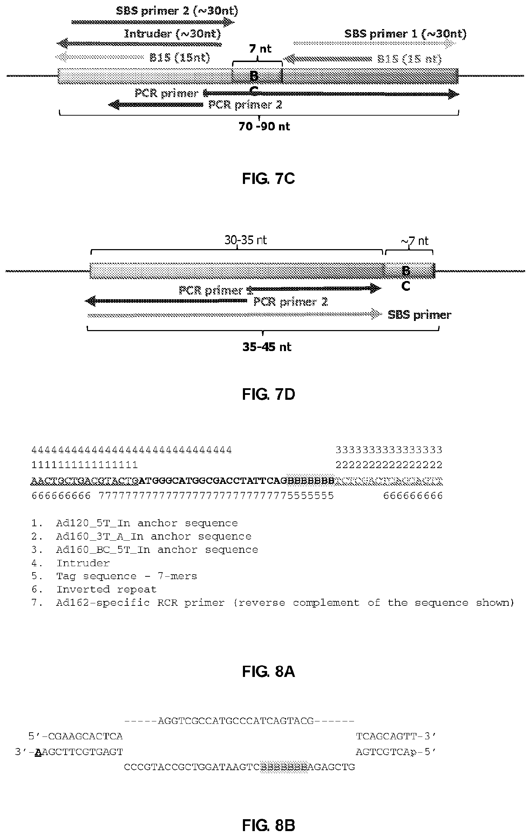

**FIG. 7****. Exemplary adapter architecture for clamp adapter for sequencing by cPAL and/or SBS.** (A)-(D) Exemplary depictions of a clamp adapter as viewed in the final mate-pair polynucleotide construct. (A) For sequencing by cPAL (reading target nucleotide sequence and barcodes in the 5' direction with cPAL), the adapter includes two hybridization sequences for a cPAL anchor (B15) and a hybridization sequence for an intruder oligonucleotide. The adapter has a length of about 70-80 bases. (B) For sequencing by SBS, the second adapter includes a hybridization sequence for a first SBS primer (SBS primer 1) that reads the target nucleotide sequence in the 3' direction, and a hybridization sequence for a second SBS primer (SBS primer 2) that reads barcodes in the 3' direction. The adapter has a length of about 70-90 bases. (C) For sequencing by both cPAL and SBS, the second adapter includes two hybridization sequences for a cPAL anchor (B15), a hybridization sequence for an intruder oligonucleotide, a hybridization sequence for a first SBS primer (SBS primer 1), and a hybridization sequence for a second SBS primer (SBS primer 2). The target nucleotide sequence can be read in the 5' direction by cPAL or in the 3' direction by SBS with SBS primer 1. The barcodes can be read in the 5' direction by cPAL or in the 3' direction by SBS with SBS primer 2. The adapter has a length of about 70-90 bases. (D) An alternative design for sequencing by SBS. The adapter comprises a hybridization sequence for a first SBS primer (SBS primer 1). The target nucleotide sequence and the barcodes are read "in-line" in the 3' direction using the same SBS sequencing primer. The adapter has a length of about 35-45 bases.
**FIG. 8****. Exemplary bubble adapter "Adapter A - Ad203."** (A) Nucleotide sequence of bubble adapter Ad203. Ad203 includes the following features: anchor hybridization sequences (1, 2, 3); an intruder hybridization sequence (4); a 7-mer barcode/tag sequence (5); an inverted repeat (6); and a RCR primer hybridization sequence for specifically amplifying constructs having one orientation of the first adapter (7). (B) The duplex of oligonucleotides that forms the Ad203 bubble adapter. An A-tailed target polynucleotide is ligated to the 3'-T overhang of the duplex of oligonucleotides. B = heptameric barcode/tag. p = 5' phosphate group. A = 3' amino modifier (3AmMO, Integrated DNA Technologies (IDT), Coralville, IA). The 3' amino modifier blocks potential ligations of the 3' end of the oligonucleotide with other DNA molecules.

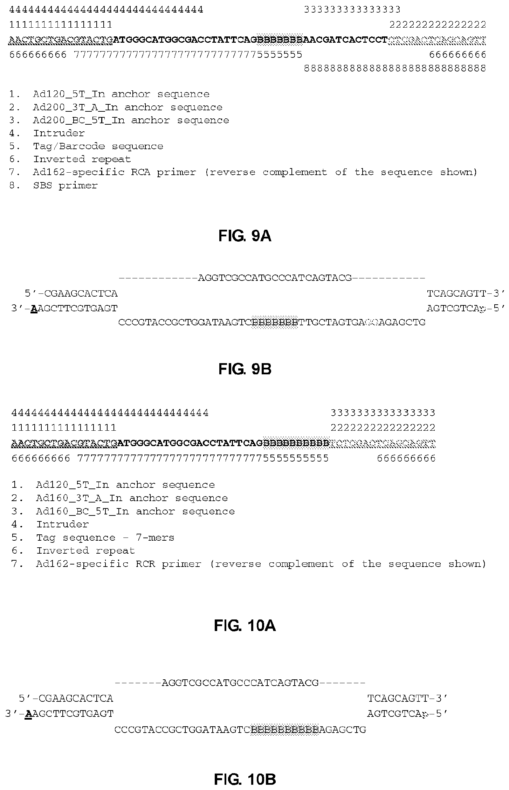

**FIG. 9****. Exemplary bubble adapter "Adapter A - Ad201."** (A) Nucleotide sequence of SBS-enabled bubble adapter Ad201. Ad203 includes the following features: anchor hybridization sequences (1, 2, 3); an intruder hybridization sequence (4); a 7-mer barcode/tag sequence (5); an inverted repeat (6); a RCR primer hybridization sequence for specifically amplifying constructs having one orientation of the first adapter (7); and an SBS primer hybridization sequence. (B) The duplex of oligonucleotides that forms the Ad201 bubble adapter. An A-tailed target polynucleotide is ligated to the 3'-T overhang of the duplex of oligonucleotides. B = heptameric barcode/tag. p = 5' phosphate group. A = 3' amino modifier (3AmMO, Integrated DNA Technologies, Coralville, IA). The 3' amino modifier blocks potential ligations of the 3' end of the oligonucleotide with other DNA molecules.
**FIG. 10****. Exemplary bubble adapter "Adapter A - Ad162."** (A) Nucleotide sequence of cPAL-enabled bubble adapter Ad162. Ad162 includes the following features: anchor hybridization sequences (1, 2, 3); an intruder hybridization sequence (4); a 7-mer barcode/tag sequence (5); an inverted repeat (6); and a RCR primer hybridization sequence for specifically amplifying constructs having one orientation of the first adapter (7). (B) The duplex of oligonucleotides that forms the Ad162 bubble adapter. An A-tailed target polynucleotide is ligated to the 3'-T overhang of the duplex of oligonucleotides. B = heptameric barcode/tag. p = 5' phosphate group. A = 3' amino modifier (3AmMO, Integrated DNA Technologies, Coralville, IA). The 3' amino modifier blocks potential ligations of the 3' end of the oligonucleotide with other DNA molecules.

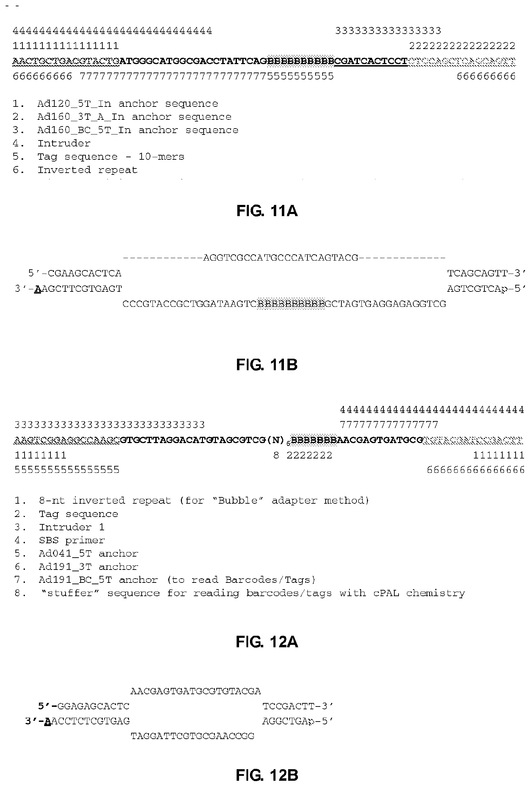

**FIG. 11****. Exemplary bubble adapter "Adapter A - Ad181."** (A) Nucleotide sequence of cPAL-enabled bubble adapter Ad181. Ad181 includes the following features: anchor hybridization sequences (1, 2, 3); an intruder hybridization sequence (4); a 10-mer barcode/tag sequence (5); an inverted repeat (6); and a RCR primer hybridization sequence for specifically amplifying constructs having one orientation of the first adapter (7). (B) The duplex of oligonucleotides that forms the Ad181 bubble adapter. An A-tailed target polynucleotide is ligated to the 3'-T overhang of the duplex of oligonucleotides. B = 10-mer barcode/tag. p = 5' phosphate group. A = 3' amino modifier (3AmMO, Integrated DNA Technologies, Coralville, IA). The 3' amino modifier blocks potential ligations of the 3' end of the oligonucleotide with other DNA molecules.
**FIG. 12****. Exemplary bubble adapter "Adapter B - Ad195."** (A) Nucleotide sequence of SBS-enabled bubble adapter Ad195. Ad195 includes the following features: an 8-nt inverted repeat (1); a tag sequence (2); an intruder hybridization sequence (3); an SBS primer hybridization sequence (4); anchor hybridization sequences (5, 6, 7); and a "stuffer" (N)₆ sequence for reading barcodes or tags with cPAL chemistry (8). (B) The duplex of oligonucleotides that forms the Ad195 bubble adapter. An A-tailed target polynucleotide is ligated to the 3'-T overhand of the duplex of oligonucleotides. B = heptameric barcode/tag. p = 5' phosphate group. A = 3' amino modifier (3AmMO, Integrated DNA Technologies, Coralville, IA). The 3' amino modifier blocks potential ligations of the 3' end of the oligonucleotide with other DNA molecules. The oligonucleotides that form Ad195 do not include a tag sequence; a tag/barcode can be added to the adapter by PCR after the ligation step.

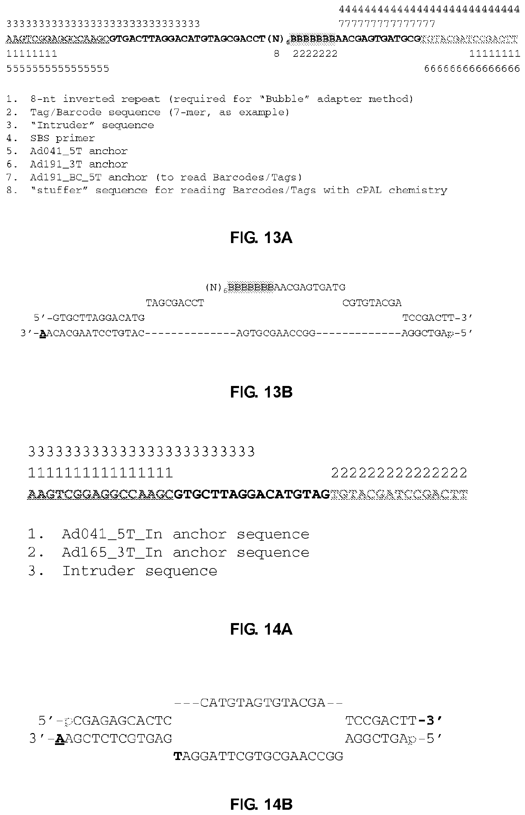

**FIG. 13****. Exemplary bubble adapter "Adapter B - Ad194."** (A) Nucleotide sequence of SBS-enabled bubble adapter Ad194. Ad194 includes the following features: an 8-nt inverted repeat (1); a tag sequence (2); an intruder hybridization sequences (3,4); an SBS primer hybridization sequence (4); anchor hybridization sequences (5, 6, 7); and a "stuffer" (N)₆ sequence for reading barcodes or tags with cPAL chemistry (8). (B) The duplex of oligonucleotides that forms the Ad194 bubble adapter. An A-tailed target polynucleotide is ligated to the 3'-T overhand of the duplex of oligonucleotides. B = heptameric barcode/tag. p = 5' phosphate group. A = 3' amino modifier (3AmMO, Integrated DNA Technologies, Coralville, IA). The 3' amino modifier blocks potential ligations of the 3' end of the oligonucleotide with other DNA molecules.
**FIG. 14****. Exemplary bubble adapter "Adapter B** - **Ad165-Bubble."** (A) Nucleotide sequence of cPAL-enabled bubble adapter Ad165-Bubble. Ad165-Bubble includes the following features: anchor hybridization sequences (1, 2); and an intruder hybridization sequences (3). (B) The duplex of oligonucleotides that forms the Adl65-Bubble bubble adapter. An A-tailed target polynucleotide is ligated to the 3'-T overhand of the duplex of oligonucleotides. p = 5' phosphate group. A = 3' amino modifier (3AmMO, Integrated DNA Technologies, Coralville, IA). The 3' amino modifier blocks potential ligations of the 3' end of the oligonucleotide with other DNA molecules.

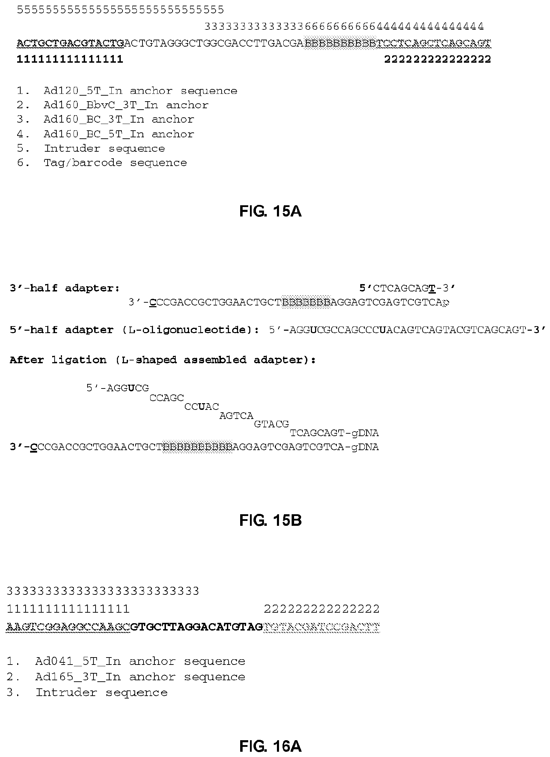

**FIG. 15****. Exemplary L-oligo adapter "Adapter A - Ad169."** (A) Nucleotide sequence of cPAL chemistry-enabled L-oligo adapter Ad169. Ad169 includes the following features: anchor hybridization sequences (1, 2, 3, 4); an intruder hybridization sequence (5); and a tag/barcode sequence (6). (B) The Ad169 L-oligo adapter is ligated to a target polynucleotide in a two-step process using a 3'-half adapter and a 5'-half adapter. After ligation of the 3'-half adapter and the 5'-half adapter, the oligonucleotides form an L-shaped structure. B = barcode. p = 5' phosphate group for ligating the 3'-half adapter to a target polynucleotide. C = ddC (dideoxy-nucleotide to prevent unwanted ligation). T = 3-dT-Q modification (Operon/Eurofins, Huntsville, AL) to prevent ligation to the target polynucleotide. An 8-nucleotide region of complementarity between the oligonucleotides is highlighted.

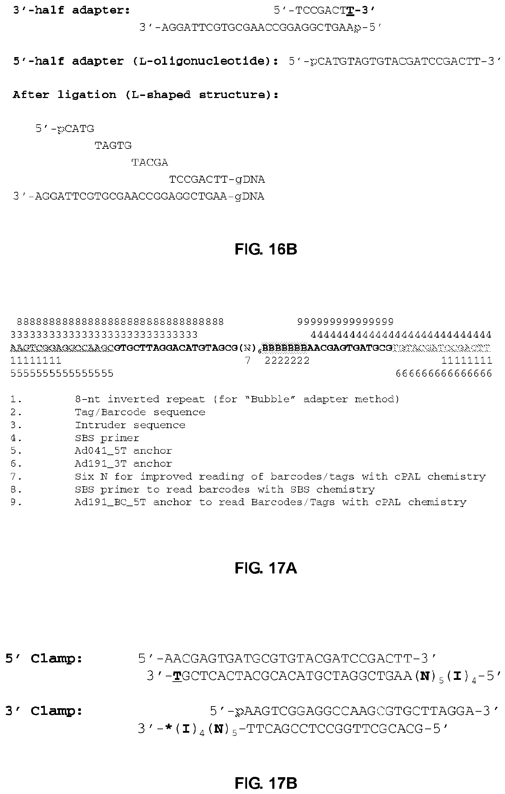

**FIG. 16****. Exemplary L-oligo adapter "Adapter B** - **Ad165."** (A) Nucleotide sequence of cPAL chemistry-enabled L-oligo adapter Ad165. Ad165 includes the following features: anchor hybridization sequences (1, 2); and an intruder hybridization sequence (3). (B) The Ad165 L-oligo adapter is ligated to a target polynucleotide in a two-step process using a 3'-half adapter and a 5'-half adapter. After ligation of the 3'-half adapter and the 5'-half adapter, the oligonucleotides form an L-shaped structure. T = 3-dT-Q modification (Operon/Eurofins, Huntsville, AL) to prevent ligation to the target polynucleotide. An 8-nucleotide region of complementarity between the oligonucleotides is highlighted.
**FIG. 17****. Exemplary clamp adapter "Adapter B** - **Ad191."** (A) Nucleotide sequence of SBS-enabled clamp adapter Ad191. Ad191 includes the following features: an inverted repeat sequence (1); a tag/barcode sequence (2); an intruder hybridization sequence (3); an SBS primer hybridization sequence (4); two anchor hybridization sequences (5, 6); a "stuffer" (N)₆ sequence for reading barcodes or tags with cPAL chemistry; an SBS primer hybridization sequence for reading barcodes or tags with SBS chemistry (8); and an anchor hybridization sequence for reading barcodes or tags with cPAL chemistry. (B) The Ad191 clamp adapter is ligated to a target polynucleotide by ligating a 3' clamp and a 5' clamp to the target polynucleotide that is in single-stranded form. The 5' clamp comprises an oligonucleotide that forms the 5' portion of the clamp adapter; the 3' clamp comprises an oligonucleotide that forms the 3' portion of the clamp adapter; and each of the 5' clamp and 3' clamp comprise a helper oligonucleotide comprising an (N)₅(I)ₙ sequence. The oligonucleotides that form Ad191 do not include a tag sequence; a tag/barcode can be added to the adapter by PCR after the ligation step. p = 5' phosphate group for ligating to a single-stranded polynucleotide. T = modified with 3' C3 spacer (3SpC3, Integrated DNA Technologies, Coralville, IA). * = last inosine is modified with 3' amino modifier (3AmMO, Integrated DNA Technologies, Coralville, IA). N = mix of all 4 nucleotides (A, T, C, G) at 1:1:1:1 ratio in each position. I = inosine.

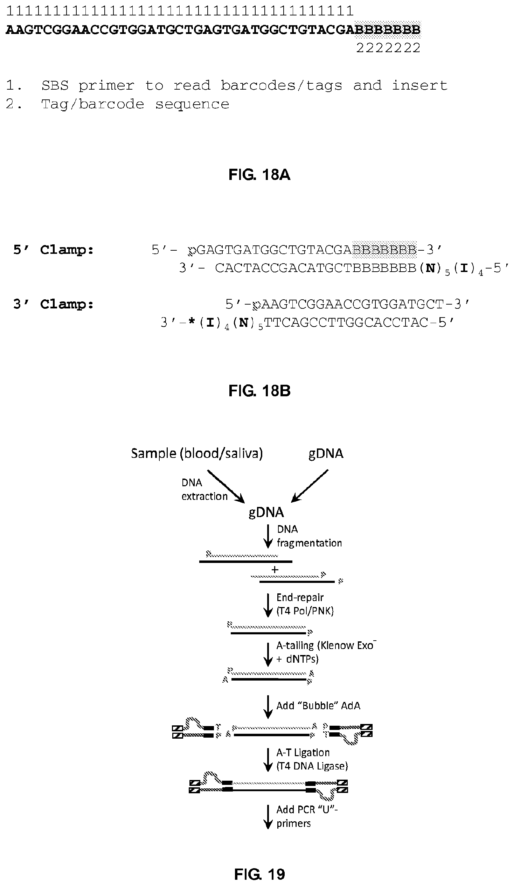

**FIG. 18****. Exemplary clamp adapter "Adapter B - Ad212."** (A) Nucleotide sequence of clamp adapter Ad212 for sequencing by SBS with "in-line" barcode reading. Ad212 includes the following features: an SBS primer hybridization sequence for reading barcodes or tags and for reading target polynucleotide sequence ("insert") (1); and a tag/barcode sequence (2). (B) The Ad212 clamp adapter is ligated to a target polynucleotide by ligating a 3' clamp and a 5' clamp to the target polynucleotide that is in single-stranded form. The 5' clamp comprises an oligonucleotide that forms the 5' portion of the clamp adapter; the 3' clamp comprises an oligonucleotide that forms the 3' portion of the clamp adapter; and each of the 5' clamp and 3' clamp comprise a helper oligonucleotide comprising an (N)₅(I)ₙ sequence. p = 5' phosphate group for ligating to a single-stranded polynucleotide, and for direct single-stranded ligation-circularization without amplification. C = modified with 3' amino modifier (3AmMO, Integrated DNA Technologies, Coralville, IA). * = last inosine is also modified with 3AmMO. N = mix of all 4 nucleotides (A, T, C, G) at 1:1:1:1 ratio in each position. I = inosine.

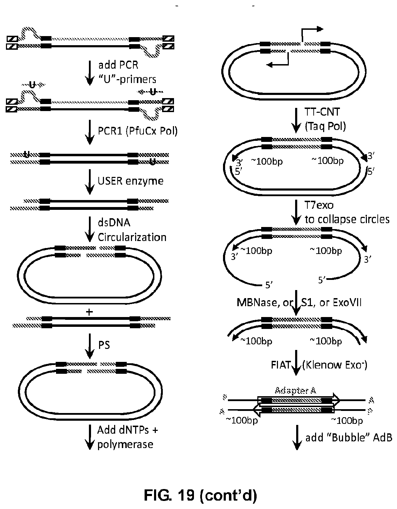

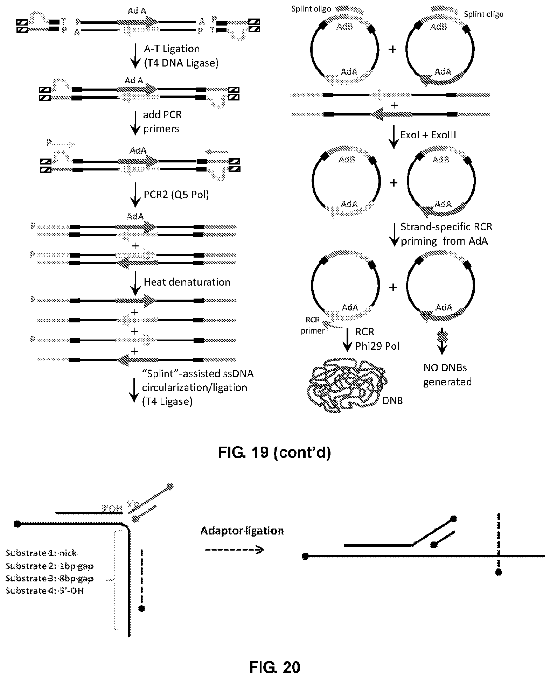

**FIG. 19****. Exemplary flow chart for construction of library comprising two bubble adapters.** An exemplary process for constructing a mate-pair polynucleotide construct comprising two bubble adapters is shown.
**FIG. 20****. 3' branch ligation.** This illustration shows ligation of an adapter to various substrates. The adapter is a synthetic dsDNA with a blunt 5' end and a 3' overhang at the 3' end to prevent adapter self-ligation. To further prevent self-ligation of the adapter, the 3' termini of the adapters are dideoxynucleotides (shown as solid circles). The phosphorylated 5' terminus of the long adapter strand (top strand) is joined with the 3' terminus of the substrate DNA. The substrate DNA molecules contain one of the following structures: Substrate 1, a nick (3'-OH, i.e., without 3' phosphate); Substrate 2, a 1 bp gap; Substrate 3, an 8 bp gap; and Substrate 4, a 5' OH, i.e., overhang end with excess 5' termini.

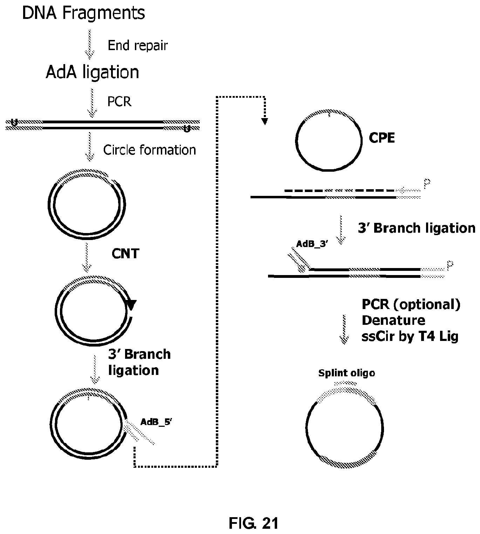

**FIG. 21****. Exemplary flow chart of library construction - ntCNT/CPE.** A flow chart is shown for constructing a library involving nick translation controlled by nucleotide amount (ntCNT) coupled with Controlled Primer Extension (CPE). The genome to be characterized is fragmented into pieces and then 500-100bp genomic DNA fragments are isolated. After end-repair and A-tailing, Ad1 half-adapter arms are ligated to ends of the fragments and the resulting Ad1 ligated fragments are amplified. The USER reaction removed 5' ends of primers, creating Ad1 arm complements. The fragment ends then become complementary to each other and the fragment with ligated Ad1 arms is circularized. A 1 bp gap is created on one strand of the circularized DNA, then nick translated for 80 bp by controlling the dNTP amount. If the DNA polymerase used for ntCNT is Taq DNA polymerase, a gapping reaction optionally is performed is to increase the size of the gap to facilitate ligation of an adapter by 3' branch ligation. Adapter Ad2_5' is then ligated to the gap by 3' branch ligation (specifically, gap ligation). The linear strand is selected as a template to synthesize the complementary strand by CPE with a specific length by controlling the dNTP ratio (i.e., ntCPE). Adapter Ad2_3' is ligated to the 5' overhang end by 3' branch ligation. Large scale PCR is used to make a copies of the resulting linear dsDNA, which are then denatured to produce ssDNA. A splint oligo is annealed to join the ends of the ssDNA and T4 ligase is used to ligate the ends to create single strand circles, which are subsequently amplified by rolling circle amplification to make DNBs for sequencing.

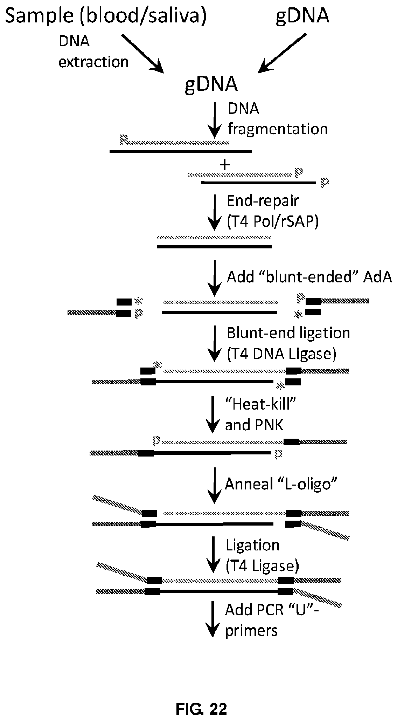

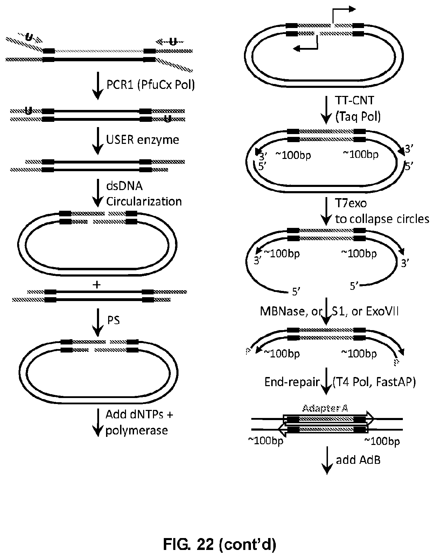

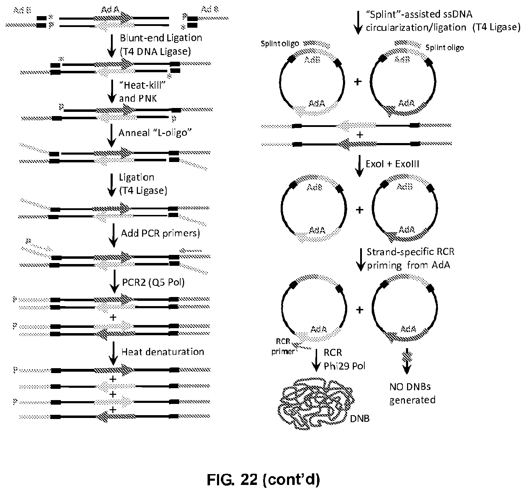

**FIG. 22****. Exemplary flow chart for construction of library comprising two L-oligo adapters.** An exemplary process for constructing a mate-pair polynucleotide construct comprising two L-oligo adapters is shown.

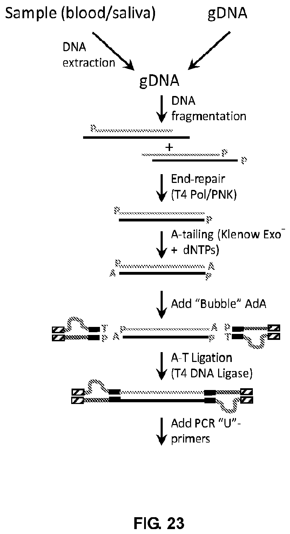

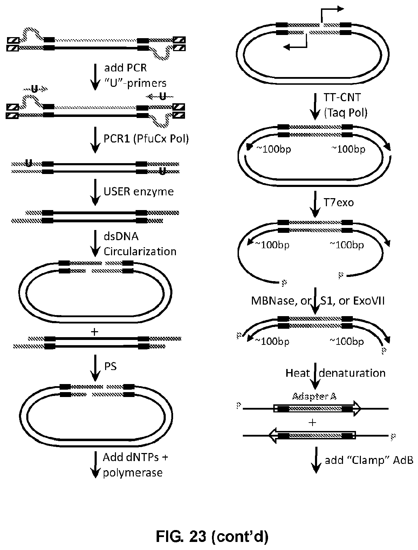

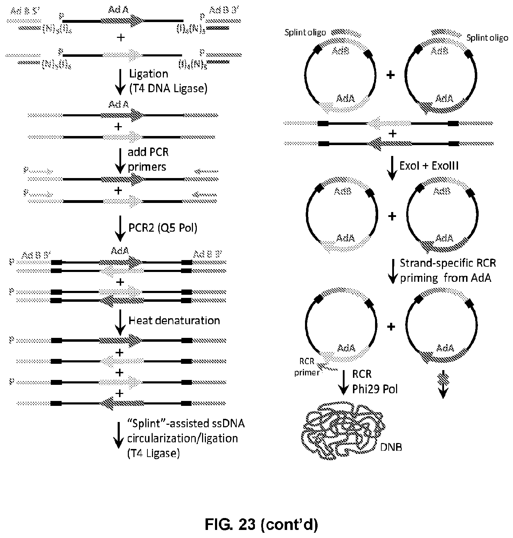

**FIG. 23****. Exemplary flow chart for construction of library comprising a bubble adapter and a clamp adapter.** An exemplary process for constructing a mate-pair polynucleotide construct comprising a first adapter that is a bubble adapter and a second adapter that is a clamp adapter is shown.

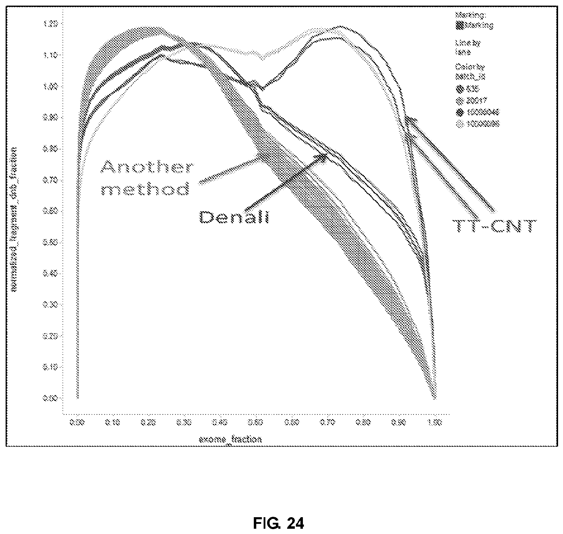

**FIG. 24****. Exome GC curves for libraries constructed using time and temperature controlled nick translation (TT-CNT) as compared to libraries constructed using other methods.** GC curves for libraries constructed according to the method of Example 1 (Batch 10000046) and Batch 10000096) were compared to the GC curves for libraries constructed using a nick translation method ("Denali") and libraries constructed according to another method.

### DETAILED DESCRIPTION OF THE INVENTION

### 1. Overview

The invention is defined by the claims. In one aspect, polynucleotide constructs and libraries for nucleic acid sequencing, and methods of generating polynucleotide constructs and libraries, are provided. The polynucleotide constructs described herein comprise mate-pair polynucleotide sequences that are produced from larger nucleic acid fragments, and further comprise adapter sequences. As used herein, the term "mate-pair polynucleotide construct" refers to a construct comprising a mate-pair of polynucleotide sequences, or " polynucleotide arms," that are produced from a larger nucleic acid (e.g., genomic DNA) fragment and further comprising a first adapter and a second adapter, wherein each polynucleotide arm is attached to the first adapter on one end and the second adapter on the other end A schematic of a mate-pair polynucleotide construct is depicted in Figure 1. A flow chart showing an exemplary process for generating a mate-pair polynucleotide construct comprising two bubble adapters is shown in Figure 2.

In some embodiments, the polynucleotide constructs or libraries described herein can be subjected to amplification methods to form polynucleotide concatemers, or "[DNA] nanoballs," that can be disposed on a surface. Sequencing methods can then be performed on the polynucleotide constructs, or on nanoballs comprising concatemers of the polynucleotide constructs, in order to detect and identify a target nucleic acid sequence. In some embodiments, the polynucleotide constructs and libraries can be sequenced using techniques such as sequencing by ligation methods, for example, combinatorial probe anchor ligation ("cPAL") methods, or sequencing by synthesis methods.

The mate-pair constructs and libraries as described herein are useful in determining the lengths and/or nucleotide sequences of repeating sequences within a target polynucleotide, a genome, an exome, a nucleotide library, and so forth. For example, many sequencing techniques have relatively short read lengths, and because these shorter read lengths may not be able to sequence through long stretches of repeating sequences (for example, repeating sequences that extend for 20, 30, 40, or 50 bases or more), it can be difficult to assemble a complete sequence from short read lengths, in part because the endpoints of the repeating sequences cannot be determined. By using mate-pair constructs and mate-pair libraries as described herein, in which the size of the starting polynucleotide fragment and the length of the deleted portion of the fragment is known or can be predicted, even a short read length can be used to identify the length and/or nucleotide sequence of a region of interest in a target polynucleotide.

The mate-pair constructs and libraries as described herein are also useful in reducing GC bias that traditionally results in low coverage of GC-rich sequences. The improvements in coverage of GC-rich sequences that can be obtained using the methods and compositions as described herein allows for higher quality of data or the ability to sequence certain gene, genome, or exome regions.

Additionally, the methods and compositions as described herein possess multiple features that significantly reduce costs for library construction. In one aspect, the methods described herein require relatively small amounts of nucleic acid input (for example, a starting genomic DNA input of about 3 µg unfragmented DNA, or 0.3 to 1.2 pmoles of fragmented and size-selected DNA). Thus, the methods described herein decrease the amount of input nucleic acid that is required for generating libraries, as compared to methods of library construction that are known in the art, without sacrificing yield or coverage. Additionally, the methods described herein reduce the total number of steps required for library construction, optimize various enzymatic and non-enzymatic steps, and scale down the reaction volumes that are required for various steps, as compared to library construction methods known in the art, without sacrificing yield or coverage. The methods described herein make the library construction process amenable to automation to increase sequencing throughput.

### 2. Genomic Nucleic Acid for Library Construction

In general, the mate-pair libraries produced according to the methods described herein comprise target nucleic acid sequences (e.g., genomic DNA, although as discussed herein, other types of nucleic acids can be used) with known synthetic polynucleotide sequences (called "adapters") between target nucleic acid sequences. The adapters can act as starting points for reading bases for a number of positions beyond each adapter-genomic DNA junction, and optionally bases can be read in both directions from the adapter.

Target nucleic acids for generating mate-pair libraries as described herein may be single stranded or double stranded, as specified herein, or may contain portions of both double stranded and single stranded sequences. For example, target nucleic acids may be genomic DNA, cDNA, mRNA, or a combination or hybrid of DNA and RNA. In some embodiments, the target nucleic acids for generating mate-pair libraries are genomic DNA.

Target nucleic acids (e.g., genomic DNA) for generating mate-pair libraries can be obtained from any organism of interest. Organisms of interest include, for example, plants; animals (e.g., mammals, including humans and non-human primates); and pathogens, such as bacteria and viruses. In some embodiments, the target nucleic acids (e.g., genomic DNA) are human nucleic acids.

Target nucleic acids are obtained from samples from an organism of interest. Non-limiting examples of samples include bodily fluids (including, but not limited to, blood, urine, serum, lymph, saliva, anal and vaginal secretions, perspiration and semen); cells; environmental samples (for example, air, agricultural, water and soil samples); biological warfare agent samples; research samples (e.g., products of nucleic acid amplification reactions, such as PCR amplification reactions); purified samples, such as purified genomic DNA; RNA preparations; and raw samples (bacteria, virus, genomic DNA, etc.). Methods of obtaining target nucleic acids (e.g., genomic DNA) from organisms are well known in the art. See, e.g., Sambrook et al., Molecular Cloning: A Laboratory Manual (1999); Ausubel et al., eds., Current Protocols in Molecular Biology, (John Wiley and Sons, Inc., NY, 1999), or the like.

In some embodiments, target nucleic acids comprise genomic DNA. In some embodiments, target nucleic acids comprise a subset of a genome (e.g., a subset of interest for a particular application, e.g., selected genes that may harbor mutations in a particular subset of a population such as individuals predisposed to get cancer at an early age). In some embodiments, target nucleic acids comprise exome DNA, i.e., a subset of whole genomic DNA enriched for transcribed sequences which contains the set of exons in a genome. In some embodiments, target nucleic acids comprise all or part of a transcriptome, i.e., the set of all mRNA or "transcripts" produced in a cell or population of cells. In some embodiments, target nucleic acids comprise all or part of a methylome, i.e., the population of methylated sites and the pattern of methylation in a genome or in a particular cell.

In some embodiments, target nucleic acids (e.g., genomic DNA) are processed by fragmentation to produce fragments of one or more specific sizes. Any method of fragmentation can be used. For example, in some embodiments, the target nucleic acids are fragmented by mechanical means (e.g., ultrasonic cleavage, acoustic shearing, needle shearing, or sonication); by chemical methods; or by enzymatic methods (e.g., using endonucleases). Methods of fragmentation are known in the art; see e.g., US 2012/0004126. In some embodiments, fragmentation is accomplished by ultrasound (e.g., Covaris or Sonicman 96-well format instruments).

In some embodiments, fragmented target nucleic acids (e.g., fragmented genomic DNA) is subjected to a size selection step to obtain nucleic acid fragments having a certain size or range of sizes. Any methods of size selection can be used. For example, in some embodiments, fragmented target nucleic acids are separated by gel electrophoresis and the band corresponding to a fragment size or range of sizes of interest is extracted from the gel. In some embodiments, a spin column can be used to select for fragments having a certain minimum size. In some embodiments, paramagnetic beads can be used to selectively bind DNA fragments having a desired range of sizes. In some embodiments, a combination of size selection methods can be used.

In some embodiments, the fragmented polynucleotides are about 50 to about 2000 bases in length, e.g., from about 50 to about 600 bases in length, from about 300 to about 1000 bases in length, from about 300 to about 600 bases in length, or from about 200 to about 2000 bases in length. In some embodiments, the fragments are 10-100, 50-100, 50-300, 100-200, 200-300, 50-400, 100-400, 200-400, 400-500, 400-600, 500-600, 50-1000, 100-1000, 200-1000, 300-1000, 400-1000, 500-1000, 600-1000, 700-1000, 700-900, 700-800, 800-1000, 900-1000, 1500-2000, or 1750-2000 bases in length. In some embodiments, the fragmented polynucleotides (e.g., genomic DNA) are about 50, about 100, about 150, about 200, about 250, about 300, about 350, about 400, about 450, about 500, about 550, about 600, about 650, about 700, about 750, about 800, about 850, about 900, about 950, about 1000, about 1100, about 1200, about 1300, about 1400, about 1500, about 1600, about 1700, about 1800, about 1900, or about 2000 bases in length.

### 3. Adapters

In one aspect, the polynucleotide constructs as described herein comprise adapters. As used herein, adapters are synthetic polynucleotides having a known sequence. Typically, the adapters are shorter in length than the polynucleotide sequences (e.g., genomic DNA fragments) into which they are inserted. The adapters can act as starting points for reading bases for a number of positions beyond each adapter-genomic DNA junction, and optionally bases can be read in both directions from the adapter.

### 3.1 Adapter Features

The architecture of the adapter that is used with the methods of the present invention can include multiple features. In some embodiments, the adapter includes one or more of the following features: an inverted repeat sequence at both the 5' and 3' ends of the adapter, for configuring the oligonucleotides that form the adapter during attachment to DNA fragments; one or more restriction endonuclease recognition sequences; one or more amplification (e.g., PCR) primer hybridization sequences; one or more sequencing primer hybridization sequences (e.g., a hybridization sequence for an SBS primer or a hybridization sequence for a cPAL primer, also referred to herein as an "anchor probe"); one or more sequences for hybridizing a splint oligonucleotide used to circularize single-stranded DNA; one or more Rolling Circle Replication (RCR) primer hybridization sequences; one or more tag or barcode sequences or "stuffer" sequences for reading a tag or barcode by cPAL; and one or more "intruder" hybridization sequences (for oligonucleotides used to wash away an anchor during cPAL sequencing).

In some embodiments, the adapter includes one or more inverted repeat sequences at the 5' and/or 3' ends of the adapter. In some embodiments, the adapter comprises a first inverted repeat sequence at its 5' end and a second inverted repeat sequence at its 3' end. In some embodiments, the inverted repeat sequences are used during the ligation of an adapter to a target nucleic acid. During ligation, the inverted repeat sequences allow for the oligonucleotides that form the adapter to transiently form an oligonucleotide duplex that is ligated to the target nucleic acid.

In some embodiments, an adapter comprises one or more restriction endonuclease recognition sequences that allows for an endonuclease binding at a recognition site within the adapter and cutting close to or within the recognition sequence. In some embodiments, the restriction endonuclease recognition sequences are recognition sites for Type IIs endonucleases. Type IIs endonucleases recognize specific sequences of nucleotide base pairs within a double-stranded polynucleotide sequence, and generally cleave outside of the recognition site, generally leaving an overhang of one strand of the sequence, or "sticky end." Type IIs endonucleases are generally commercially available and are well known in the art.

In some embodiments, an adapter comprises one or more primer hybridization sequences, such as one or more binding sites for a primer or primers for an amplification reaction (e.g., a PCR primer or an RCR primer), or one or more binding sites for a primer or primers for a sequencing reaction (e.g., for sequencing by synthesis). In some embodiments, an adapter comprises multiple primer hybridization sequences, e.g., two, three, four, five, or more primer hybridization sequences.

In some embodiments, an adapter comprises one or more sequencing primer hybridization sequences, such as one or more sequences for hybridizing with an SBS sequencing primer, or one or more sequences for hybridizing with an "anchor" probe. Anchor probes can be used in sequencing methods, for example, in cPAL sequencing methods as described herein. Anchor probes for use in cPAL sequencing are described in US 9,023,769. In some embodiments, an adapter comprises multiple sequencing primer hybridization sequences, e.g., two, three, four, five, or more sequencing primer hybridization sequences. In some embodiments, an adapter comprises sequencing primer hybridization sequences for each or two or more sequencing methods (e.g., one or more sequences for hybridizing with an SBS sequencing primer and one or more sequences for hybridizing with a cPAL anchor probe).

In some embodiments, an adapter comprises one or more "intruder" sequences. As used herein, intruder sequences are binding sites for oligonucleotides that are used for washing away anchor probes during sequencing methods that use anchor probes (e.g., in cPAL sequencing).

In some embodiments, an adapter comprises one or more sequences for hybridizing a "splint" oligonucleotide. As used herein, a splint oligonucleotide is an oligonucleotide that is used in the circularization of single-stranded linear polynucleotide constructs (e.g., a linear construct comprising mate-pair polynucleotide arms, a first adapter, and a second adapter). The splint oligonucleotide hybridizes to the single-stranded circle at the site of ligation in order to stabilize the circle long enough for ligation to be carried out.

In some embodiments, an adapter comprises one or more tag or barcode sequences or "stuffer" (placeholder) sequences for improved quality of barcode sequencing with cPAL chemistry. As used herein, the term "barcode" refers to a unique oligonucleotide sequence that allows a corresponding nucleic acid sequence (e.g., an oligonucleotide fragment) to be identified, retrieved and/or amplified. In some embodiments, a barcode is introduced that is unique to each sample from which polynucleotide fragments are obtained. In some embodiments, barcodes can each have a length within a range of about 4 to about 30 bases, of about 6 to about 20 bases, or of about 5 to about 10 bases. In some embodiments, a barcode comprises a "unique molecular identifier" (UMI) sequence (e.g., a sequence used to label a population of nucleic acid molecules such that each molecule in the population has a different identifier associated with it). Barcode and UMI technologies are known in the art; see, e.g., Winzeler et al. (1999) Science 285:901; Parameswaran et al (2007) Nucleic Acids Res 35(19):e130; Tu et al. (2012) BMC Genomics 13:43; Kivioja et al., Nat Methods 9:72-74 (2012); US 5,604,097; US 7,537,897; US 8,715,967; US 8,835,358; and WO 2013/173394. In some embodiments, a barcode sequence is introduced into an adapter sequence by including the barcode sequence in an oligonucleotide that forms the adapter (e.g., bubble adapter, L-oligo adapter, or clamp adapter). In some embodiments, a barcode sequence is introduced into an adapter sequence through an amplification reaction (e.g., PCR) with one or more primers containing the barcode sequence.

### 3.2 Adapter Structures

In some embodiments, the adapter is a "bubble" adapter. In some embodiments, the adapter is an "L-oligo" adapter. In some embodiments, the adapter is a "clamp" adapter. Exemplary structures of the oligonucleotides that form the bubble adapter, L-oligo adapter, and clamp adapter are shown in Figure 3. Exemplary schematics depicting the method of ligating the bubble adapter, L-oligo adapter, and clamp adapter to a DNA fragment are shown in Figure 4.

In some embodiments, each mate-pair polynucleotide construct in the library of mate-pair constructs that is generated comprises two adapters. In some embodiments, the first adapter and the second adapter in the polynucleotide molecule are the same type of adapter (e.g., each of the first adapter and the second adapter are bubble adapters, or each of the first adapter and the second adapter are L-oligo adapters). In some embodiments, the first adapter and the second adapter in the polynucleotide molecule are different types of adapters (e.g., the first adapter is a bubble adapter and the second adapter is a clamp adapter).

### 3.3 Bubble Adapters

In some embodiments, one or both of the adapters that are ligated to a polynucleotide (e.g., genomic DNA fragment) of interest is a "bubble adapter." The bubble adapter is formed from two oligonucleotide sequences, a "first oligonucleotide" and a "second oligonucleotide." The two oligonucleotides are partially complementary to each other at their 5' and 3' ends, such that the 5' end of the first oligonucleotide is complementary to the 3' end of the second oligonucleotide, and the 3' end of the first oligonucleotide is complementary to the 5' end of the second oligonucleotide. The intervening sequence of each oligonucleotide (i.e., the sequence in the middle region of each oligonucleotide) is not substantially complementary to the other oligonucleotide, such that the middle regions of the oligonucleotides do not hybridize with each other, thus forming a "bubble." A schematic depicting a duplex of oligonucleotides and the bubble structure formed by the duplex is shown in Figure 3 (middle panel).

The bubble adapter may include one or more features such as inverted repeat sequences, restriction endonuclease recognition sequences, PCR primer hybridization sequences, sequencing primer hybridization sequences (e.g., for sequencing with cPAL chemistry and/or for sequencing with SBS chemistry), anchor probe hybridization sequences, RCR primer hybridization sequences, intruder hybridization sequences, tag or barcode sequences, splint oligonucleotide hybridization sequences, and stuffer sequences.

In some embodiments, a mate-pair polynucleotide construct comprises two bubble adapters, a first bubble adapter and a second bubble adapter. The first bubble adapter and the second bubble adapter can include the same features or at least some of the same features (e.g., inverted repeat sequences, restriction endonuclease recognition sequences, PCR primer hybridization sequences, sequencing primer hybridization sequences, anchor probe hybridization sequences, RCR primer hybridization sequences, intruder hybridization sequences, tag or barcode sequences, splint oligonucleotide hybridization sequences, and stuffer sequences). In some embodiments, the first bubble adapter and the second bubble adapter include some, but not all, of the same features.

As viewed in a circular mate-pair polynucleotide construct, the bubble adapter typically has a length of about 50 to about 100 bases (e.g., about 50 to about 90 bases in length, about 60 to about 80 bases in length, about 60 to about 70 bases in length, or about 70-80 bases in length). The first bubble adapter and the second bubble adapter can be the same length or can be different lengths. In some embodiments, the first bubble adapter is longer than the second bubble adapter. In some embodiments, the second bubble adapter is longer than the first bubble adapter.

In some embodiments, the length of the bubble adapter can vary depending on the method or methods of sequencing to be used. For example, in some embodiments, a first bubble adapter and/or a second bubble adapter may contain primer hybridization sequences for sequencing by one type of chemistry (e.g., sequencing with cPAL chemistry only, or sequencing with SBS chemistry only). In some embodiments, a bubble adapter comprising primer hybridization sequences for sequencing with only one type of chemistry has a length of about 60-90 bases, about 60-70 bases, about 60-80 bases, about 70-80 bases, or about 80-90 bases. In some embodiments, a first bubble adapter and/or a second bubble adapter may contain primer hybridization sequences for sequencing with "mixed" chemistry (e.g., sequencing a construct or DNA with cPAL chemistry and SBS chemistry in a sequential manner). In some embodiments, a bubble adapter comprising primer hybridization sequences for sequencing with mixed chemistry has a length of about 70-90 bases, about 70-80 bases or about 80-90 bases. Exemplary embodiments of bubble adapters comprising primer hybridization sequences for sequencing with cPAL chemistry only, for sequencing with SBS chemistry only, or for sequencing with both cPAL chemistry and SBS chemistry are shown in Figure 5A-C and Figure 6A-C.

Typically, the first oligonucleotide (also referred to in Figure 3 as the "5' half-adapter") has a structure as follows. The 5' end of the first oligonucleotide has a region (also referred to in Figure 3 as the "clasp" region) that is complementary to and forms a duplex with a 3' region of the second oligonucleotide. In some embodiments, the clasp region is ≥12 bases in length; in some embodiments, the clasp region is about 12 to about 20 bases in length. Following the clasp region is a region that is not complementary to the second oligonucleotide, which can be from about 15 to about 60 bases in length (e.g., about 15, about 20, about 25, about 30, about 35, about 40, about 45, about 50, about 55, or about 60 bases in length). Following this region of non-complementarity is an inverted repeat region that is complementary to and forms a duplex with a 5' region of the second oligonucleotide. This inverted repeat region can be about 6 to about 14 bases in length; in some embodiments, the inverted repeat region is about 7 to 9 bases in length. Following the inverted repeat region is a 3' "T" overhang of one or more bases that is complementary to an A-tail in a DNA fragment. In some embodiments, the entire length of the first oligonucleotide is from about 35 to about 80 bases in length (e.g., about 35, about 40, about 45, about 50, about 55, about 60, about 65, about 70, about 75, or about 80 bases in length).

Typically, the second oligonucleotide (also referred to in Figure 3 as the "3' half-adapter") has a structure as follows. The 5' end of the second oligonucleotide has a phosphate group for ligating the oligonucleotide to the DNA fragment. Following the 5' phosphate group, the second oligonucleotide has an inverted repeat region that is complementary to and forms a duplex with a 3' region of the first oligonucleotide. This inverted repeat region can be about 6 to about 14 bases in length (e.g., about 6, 7, 8, 9, 10, 11, 12, 13, or 14 bases in length). Following the inverted repeat region is a region that is not complementary to the first oligonucleotide, which can be from about 10 to about 60 bases in length (e.g., about 10, about 15, about 20, about 25, about 30, about 35, about 40, about 45, about 50, about 55, or about 60 bases in length). The lack of complementarity between the first oligonucleotide and the second oligonucleotide results in the formation of a bubble-like structure in the oligonucleotide duplex. Following this region of non-complementarity is a region (also referred to in Figure 3 as the "clasp" region) that is complementary to and forms a duplex with a 5' region of the first oligonucleotide. In some embodiments, the clasp region is ≥12 bases in length; in some embodiments, the clasp region is about 12 to about 20 bases in length (e.g., about 12, about 13, about 14, about 15, about 16, about 17, about 18, about 19, or about 20 bases in length). Following the clasp region, the second oligonucleotide has a 3' modification or blocking group that is used to block any potential ligations of this 3' end with other polynucleotide molecules (e.g., DNA fragments or other bubble adapter oligonucleotides); non-limiting examples of 3' modifications or blocking groups include a 3' amino modifier (3AmMO, Integrated DNA Technologies (IDT), Coralville, IA), 3' spacer (e.g., C3 spacer 3SpC3, IDT), a dideoxynucleotide (e.g. ddC), an inverted dT (invdT, IDT), or any of 3-dT-Q/3-dA-Q/3-dC-Q/3-dG-Q (Operon/Eurofins, Huntsville, AL). In some embodiments, the entire length of the first oligonucleotide is from about 35 to about 80 bases in length (e.g., about 35, about 40, about 45, about 50, about 55, about 60, about 65, about 70, about 75, or about 80 bases in length).

The first oligonucleotide and the second oligonucleotide that form the bubble adapter can be the same length or can be different lengths. In some embodiments, the first oligonucleotide is longer than the second oligonucleotide. In some embodiments, the second oligonucleotide is longer than the first oligonucleotide.

A bubble adapter is ligated to a polynucleotide (e.g., DNA fragment) by annealing a duplex of the first oligonucleotide and the second oligonucleotide and ligating the formed bubble adapter to both ends of the polynucleotide (e.g., DNA fragment). In some embodiments, the resulting bubble adapter that is present in a mate-pair polynucleotide construct is shorter in length than the sum total of the first oligonucleotide and the second oligonucleotide; for example, in some embodiments, a first bubble adapter is shorter than the sum total of the first oligonucleotide and the second oligonucleotide that form the first bubble adapter, due to the overlap of complementary sequences in the first oligonucleotide and the second oligonucleotide that is used to stabilize the open double-stranded DNA circle during the step of generating mate-pair polynucleotide arms. In some embodiments, the resulting bubble adapter that is present in a mate-pair polynucleotide construct is longer in length than the sum total of the first oligonucleotide and the second oligonucleotide; for example, in some embodiments, a second bubble adapter is longer than the sum total of the first oligonucleotide and the second oligonucleotide that form the second bubble adapter, due to the addition of nucleotides in splint-assisted ssDNA circularization or due to the addition of a barcode sequence by PCR.

One embodiment of a first bubble adapter is illustrated in Figure 8 and in SEQ ID NO:1. This first adapter, referred to as "Ad203," has a length of 61 nucleotides and includes the following features: inverted repeat sequences at the 5' and 3' ends of the adapter; anchor probe hybridization sequences; an intruder hybridization sequence; a tag sequence; and a strand-specific RCR primer hybridization sequence. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:1. In some embodiments, an adapter has the polynucleotide sequence of SEQ ID NO:1.

Another embodiment of a first bubble adapter is illustrated in Figure 9 and in SEQ ID NO:2. This first adapter, referred to as "Ad201," has a length of 73 nucleotides and includes the following features: inverted repeat sequences at the 5' and 3' ends of the adapter; anchor probe hybridization sequences; an intruder hybridization sequence; a tag/barcode sequence; a strand-specific RCR primer hybridization sequence; and an SBS primer hybridization sequence. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:2. In some embodiments, an adapter has the polynucleotide sequence of SEQ ID NO:2.

Yet another embodiment of a first bubble adapter is illustrated in Figure 10 and in SEQ ID NO:3. This first adapter, referred to as "Ad162," has a length of 64 nucleotides and includes the following features: inverted repeat sequences at the 5' and 3' ends of the adapter; anchor probe hybridization sequences; an intruder hybridization sequence; a tag/barcode sequence; and a strand-specific RCR primer hybridization sequence. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:3. In some embodiments, an adapter has the polynucleotide sequence of SEQ ID NO:3.

Still another embodiment of a first bubble adapter is illustrated in Figure 11 and in SEQ ID NO:4. This first adapter, referred to as "Ad201," has a length of 75 nucleotides and includes the following features: inverted repeat sequences at the 5' and 3' ends of the adapter; anchor probe hybridization sequences; an intruder hybridization sequence; a tag/barcode sequence; and a strand-specific RCR primer hybridization sequence. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:4. In some embodiments, an adapter has the polynucleotide sequence of SEQ ID NO:4.

One embodiment of a second bubble adapter is illustrated in Figure 12 and in SEQ ID NO:5. This second adapter, referred to as "Ad195," has a length of 79 nucleotides and includes the following features: inverted repeat sequences at the 5' and 3' ends of the adapter; a 7-mer tag sequence; an intruder hybridization sequence; an SBS sequencing primer hybridization sequence; anchor probe hybridization sequences; and a 6-mer "stuffer" sequence for reading barcodes or tags with cPAL chemistry. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:5. In some embodiments, an adapter has the polynucleotide sequence of SEQ ID NO:5.

Another embodiment of a second bubble adapter is illustrated in Figure 13 and in SEQ ID NO:6. This second adapter, referred to as "Adl94," has a length of 81 nucleotides and includes the following features: inverted repeat sequences at the 5' and 3' ends of the adapter; a 7-mer tag sequence; an intruder hybridization sequence; an SBS sequencing primer hybridization sequence; anchor probe hybridization sequences; and a 7-mer "stuffer" sequence for reading barcodes or tags with cPAL chemistry. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:6. In some embodiments, an adapter has the polynucleotide sequence of SEQ ID NO:6.

Yet another embodiment of a second bubble adapter is illustrated in Figure 14 and in SEQ ID NO:7. This second adapter, referred to as "Ad165-Bubble," has a length of 48 nucleotides and includes the following features: inverted repeat sequences at the 5' and 3' ends of the adapter; anchor probe hybridization sequences; and an intruder hybridization sequence. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:7. In some embodiments, an adapter has the polynucleotide sequence of SEQ ID NO:7.

### 3.4 L-oligo Adapters

In some embodiments, one or both of the adapters that are ligated to a polynucleotide (e.g., genomic DNA fragment) of interest is an "L-oligo adapter." The L-oligo adapter is formed from two oligonucleotide sequences, a "first oligonucleotide" (also referred to herein as a "5'-half adapter") and a "second oligonucleotide" (also referred to herein as a "3'-half adapter"). The two oligonucleotides are partially complementary to each other, such that 3' end of the first oligonucleotide is complementary to the 5' end of the second oligonucleotide. The remaining 5' sequence of the first oligonucleotide is not substantially complementary to the remaining 3' sequence of the second oligonucleotide, such that these regions do not hybridize with each other; as a result, the first oligonucleotide forms an "L" shape. A schematic depicting a duplex of oligonucleotides and the L-oligo structure formed by the duplex is shown in Figure 3 (left panel).

The L-oligo adapter may include one or more features such as inverted repeat sequences, restriction endonuclease recognition sequences, PCR primer hybridization sequences, sequencing primer hybridization sequences (e.g., for sequencing with cPAL chemistry and/or for sequencing with SBS chemistry), anchor probe hybridization sequences, RCR primer hybridization sequences, intruder hybridization sequences, tag or barcode sequences, and stuffer sequences.

In some embodiments, a mate-pair polynucleotide construct comprises two L-oligo adapters, a first L-oligo adapter and a second L-oligo adapter. The first L-oligo adapter and the second L-oligo adapter can include the same features or at least some of the same features (e.g., inverted repeat sequences, restriction endonuclease recognition sequences, PCR primer hybridization sequences, sequencing primer hybridization sequences, anchor probe hybridization sequences, RCR primer hybridization sequences, intruder hybridization sequences, tag or barcode sequences, splint oligonucleotide hybridization sequences, and stuffer sequences). In some embodiments, the first L-oligo adapter and the second L-oligo adapter include some, but not all, of the same features. In some embodiments, the first L-oligo adapter comprises a barcode sequence that is introduced into the L-oligo adapter via the second oligonucleotide of the first L-oligo adapter, which is ligated to a polynucleotide fragment prior to ligating the first oligonucleotide of the first L-oligo adapter; because the second oligonucleotide is ligated to the polynucleotide fragment prior to the first oligonucleotide, including a barcode sequence in the second oligonucleotide allows for the possibility of pooling together different samples which are tagged by barcodes and continuing the library construction process as a multiplexed process (e.g., for Whole Exome Sequence (WES) and Long Fragment Read (LFR) sequencing applications).

As viewed in a circular mate-pair polynucleotide construct, the L-oligo adapter typically has a length of about 50 to about 100 bases (e.g., about 50 to about 90 bases in length, about 60 to about 80 bases in length, about 60 to about 70 bases in length, or about 70-80 bases in length). The first L-oligo adapter and the second L-oligo adapter can be the same length or can be different lengths. In some embodiments, the first L-oligo adapter is longer than the second L-oligo adapter. In some embodiments, the second L-oligo adapter is longer than the first L-oligo adapter.

In some embodiments, the length of the L-oligo adapter can vary depending on the method or methods of sequencing to be used. For example, in some embodiments, a first L-oligo adapter and/or a second L-oligo adapter may contain primer hybridization sequences for sequencing by one type of chemistry (e.g., sequencing with cPAL chemistry only, or sequencing with SBS chemistry only). In some embodiments, an L-oligo adapter comprising primer hybridization sequences for sequencing with only one type of chemistry has a length of about 60-90 bases, about 60-70 bases, about 60-80 bases, about 70-80 bases, or about 80-90 bases. In some embodiments, a first L-oligo adapter and/or a second L-oligo adapter may contain primer hybridization sequences for sequencing with "mixed" chemistry (e.g., sequencing a construct or DNA with cPAL chemistry and SBS chemistry in a sequential manner). In some embodiments, an L-oligo adapter comprising primer hybridization sequences for sequencing with mixed chemistry has a length of about 70-90 bases, about 70-80 bases or about 80-90 bases. Exemplary embodiments of L-oligo adapters comprising primer hybridization sequences for sequencing with cPAL chemistry only, for sequencing with SBS chemistry only, or for sequencing with both cPAL chemistry and SBS chemistry are shown in Figure 5A-C and Figure 6A-C.

Typically, the first oligonucleotide (also referred to in Figure 3 as the "5' half-adapter") has a structure as follows. The 5' region of the first oligonucleotide is a region that is not complementary to the 3' region of the second oligonucleotide. In some embodiments, this region that is not complementary is from about 20 to about 60 bases in length (e.g., about 20, about 25, about 30, about 35, about 40, about 45, about 50, about 55, or about 60 bases in length). Following this region of non-complementarity is an inverted repeat region that is complementary to and forms a duplex with the 5' region of the second oligonucleotide. This inverted repeat region can be about 6 to about 12 bases in length (e.g., about 6, about 7, about 8, about 9, about 10, about 11, or about 12 bases in length); in some embodiments, the inverted repeat region is about 7 to 9 bases in length. In some embodiments, the entire length of the first oligonucleotide is from about 25 to about 75 bases in length (e.g., about 25, about 30, about 35, about 40, about 45, about 50, about 55, about 60, about 65, about 70, or about 75 bases in length).

Typically, the second oligonucleotide (also referred to in Figure 3 as the "3' half-adapter") has a structure as follows. The 5' end of the second oligonucleotide, after being annealed to the first oligonucleotide, forms a blunt end. Following the 5' blunt end is an inverted repeat region that is complementary to and forms a duplex with the 3' region of the first oligonucleotide. This inverted repeat region can be about 6 to about 12 bases in length (e.g., about 6, about 7, about 8, about 9, about 10, about 11, or about 12 bases in length); in some embodiments, the inverted repeat region is about 7 to 9 bases in length. Following the inverted repeat region is a region that is not complementary to the 5' region of the first oligonucleotide. In some embodiments, this region that is not complementary is from about 20 to about 60 bases in length (e.g., about 20, about 25, about 30, about 35, about 40, about 45, about 50, about 55, or about 60 bases in length). In some embodiments, the entire length of the first oligonucleotide is from about 25 to about 75 bases in length (e.g., about 25, about 30, about 35, about 40, about 45, about 50, about 55, about 60, about 65, about 70, or about 75 bases in length).

The two oligonucleotide sequences that form the L-oligo adapter can be the same length or can be different lengths. In some embodiments, the first oligonucleotide is longer than the second oligonucleotide. In some embodiments, the second oligonucleotide is longer than the first oligonucleotide.

An L-oligo adapter is ligated to a polynucleotide (e.g., DNA fragment) by a two-step ligation process. In the first ligation step, the 3' half-adapter (second oligonucleotide) is ligated to the 3' end of a blunt-ended polynucleotide (e.g., a genomic DNA fragment) in the presence of a short (about 8-9 nucleotide) helper oligonucleotide that has a 3'-end modification (e.g., a 3-dN-Q modification, available from Operon/Eurofins). As used with respect to ligation of an L-oligo adapter, a "helper oligonucleotide" refers to an oligonucleotide that hybridizes to a portion of the second oligonucleotide (e.g., the 5' region of the second oligonucleotide) to facilitate ligation of the second oligonucleotide to the target polynucleotide fragment in blunt-end ligation. The 5' half-adapter (first oligonucleotide) is then ligated to the 5' ends in a second ligation reaction. In some embodiments, the resulting L-oligo adapter that is present in a mate-pair polynucleotide construct (e.g., a circular mate-pair construct suitable for concatemerization) is shorter in length than the sum total of the first oligonucleotide and the second oligonucleotide, (e.g., due to the overlap of complementary sequences in a first oligonucleotide and a second oligonucleotide that is used to stabilize the open double-stranded DNA circle during the step of generating mate-pair polynucleotide arms).

One embodiment of a first L-oligo adapter is illustrated in Figure 15 and in SEQ ID NO:8. This first adapter, referred to as "Ad169," has a length of 66 nucleotides and includes the following features: an inverted repeat sequence; anchor probe hybridization sequences; an intruder hybridization sequence; and a tag sequence. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:8. In some embodiments, an adapter has the polynucleotide sequence of SEQ ID NO:8.

One embodiment of a second L-oligo adapter is illustrated in Figure 16 and in SEQ ID NO:9. This second adapter, referred to as "Ad165," has a length of 48 nucleotides and includes the following features: an inverted repeat sequence; an intruder hybridization sequence; anchor probe hybridization sequences; and a sequence for hybridizing a splint oligonucleotide. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:9. In some embodiments, an adapter has the polynucleotide sequence of SEQ ID NO:9.

### 3. 5 Clamp Adapters

In some embodiments, one or both of the adapters that are ligated to a polynucleotide (e.g., genomic DNA fragment) of interest is a "clamp adapter." The clamp adapter is ligated to a target polynucleotide by ligating a "3' clamp" and a "5' clamp" to a single-stranded target polynucleotide of interest (e.g., a DNA fragment). The 5' clamp comprises a first oligonucleotide and a first "helper oligonucleotide," and the 3' clamp comprises a second oligonucleotide and a second "helper oligonucleotide." As used with respect to ligation of a clamp adapter, a "helper oligonucleotide" refers to an oligonucleotide that hybridizes to a portion of a first oligonucleotide or second oligonucleotide that forms the clamp adapter in order to facilitate ligation of the first oligonucleotide and the second oligonucleotide to the target polynucleotide. The helper oligonucleotide is removed following ligation, and thus is not part of the final clamp adapter as viewed in the mate-pair polynucleotide construct. The helper oligonucleotides comprise a sequence of random nucleotides (A, T, C, or G) and universal (inosine) nucleotides that is able to hybridize to the target polynucleotide of interest (e.g., DNA fragment). Thus, the helper oligonucleotides help "clamp" the first oligonucleotide and second oligonucleotide to the target polynucleotide. An example of the formation of a clamp adapter from a 5' clamp (comprising a first oligonucleotide) and a 3' clamp (comprising a second oligonucleotide) is shown in Figure 3.

The clamp adapter may include one or more features such as restriction endonuclease recognition sequences, PCR primer hybridization sequences, sequencing primer hybridization sequences (e.g., for sequencing with cPAL chemistry and/or for sequencing with SBS chemistry), anchor probe hybridization sequences, RCR primer hybridization sequences, intruder hybridization sequences, splint oligonucleotide hybridization sequences, tag or barcode sequences, and stuffer sequences.

In some embodiments, a mate-pair polynucleotide construct comprises two clamp adapters, a first clamp adapter and a second clamp adapter. The first clamp adapter and the second clamp adapter can include the same features or at least some of the same features (e.g., restriction endonuclease recognition sequences, PCR primer hybridization sequences, sequencing primer hybridization sequences, anchor probe hybridization sequences, RCR primer hybridization sequences, intruder hybridization sequences, tag or barcode sequences, and stuffer sequences). In some embodiments, the first clamp adapter and the second clamp adapter include some, but not all, of the same features.

As viewed in a circular mate-pair polynucleotide construct, the clamp adapter typically has a length of about 35 to about 100 bases (e.g., about 35 to about 50 bases in length, about 60 to about 90 bases in length, about 70 to about 90 bases in length, or about 70-80 bases in length). The first clamp adapter and the second clamp adapter can be the same length or can be different lengths. In some embodiments, the first clamp adapter is longer than the second clamp adapter. In some embodiments, the second clamp adapter is longer than the first clamp adapter.

In some embodiments, the length of the clamp adapter can vary depending on the method or methods of sequencing to be used. For example, in some embodiments, a first clamp adapter and/or a second clamp adapter may contain primer hybridization sequences for sequencing by one type of chemistry (e.g., sequencing with cPAL chemistry only, or sequencing with SBS chemistry only). In some embodiments, a clamp adapter comprising primer hybridization sequences for sequencing with only one type of chemistry has a length of about 60-90 bases, about 70-90 bases, about 70-80 bases, or about 80-90 bases. Alternatively, in some embodiments, a clamp adapter comprising primer hybridization sequences for sequencing with only SBS sequences has a length of about 35-50 bases or about 35-45 bases. In some embodiments, a first clamp adapter and/or a second clamp adapter may contain primer hybridization sequences for sequencing with "mixed" chemistry (e.g., sequencing a construct or DNA with cPAL chemistry and SBS chemistry in a sequential manner). In some embodiments, a clamp adapter comprising primer hybridization sequences for sequencing with mixed chemistry has a length of about 70-90 bases, about 70-80 bases, or about 80-90 bases. Exemplary embodiments of clamp adapters comprising primer hybridization sequences for sequencing with cPAL chemistry only, for sequencing with SBS chemistry only, or for sequencing with both cPAL chemistry and SBS chemistry are shown in Figure 7A-D.

The first oligonucleotide (corresponding to the 5' portion of the final clamp adapter) and the second oligonucleotide (corresponding to the 3' portion of the final clamp adapter) can be the same length or can be different lengths. In some embodiments, the first oligonucleotide is longer than the second oligonucleotide. In some embodiments, the first oligonucleotide and/or the second oligonucleotide is from about 20 to about 75 bases in length (e.g., about 20, about 25, about 30, about 35, about 40, about 45, about 50, about 55, about 60, about 65, about 70, or about 75 bases in length).

In some embodiments, a first helper oligonucleotide is used for aiding ligation of a first oligonucleotide corresponding to the 5' portion of the final clamp adapter, and a second helper oligonucleotide is used for aiding ligation of a second oligonucleotide corresponding to the 3' portion of the final clamp adapter. In some embodiments, the first helper oligonucleotide comprises a 5' (N)₅(I)ₙ sequence followed by a region that hybridizes to the first oligonucleotide. In the (N)₅(I)ₙ sequence, N can be any of G, A, T, or C nucleotides, I is inosine, and n≥3. In some embodiments, the first helper oligonucleotide further comprises a modification at the 3' end to prevent intramolecular ligation. In some embodiments, the first helper oligonucleotide has a length of about 20-40 bases.

In some embodiments, the second helper oligonucleotide comprises a 5' region that hybridizes to the second oligonucleotide, followed by a (N)₅(I)ₙ sequence. In the (N)₅(I)ₙ sequence, N can be any of G, A, T, or C nucleotides, I is inosine, and n≥3. In some embodiments, the second helper oligonucleotide further comprises a modification at the 3' end to prevent intramolecular ligation. In some embodiments, the second helper oligonucleotide has a length of about 20-40 bases.

A clamp adapter is ligated to a polynucleotide (e.g., DNA fragment) that is in single-stranded form by ligating the first oligonucleotide and second oligonucleotide in the presence of the helper oligonucleotide sequences described above. In some embodiments, the resulting clamp adapter that is present in a mate-pair polynucleotide construct (e.g., a circular mate-pair construct suitable for concatemerization) is shorter in length than the sum total of the first oligonucleotide and the second oligonucleotide, (e.g., due to the overlap of complementary sequences in a first oligonucleotide and a second oligonucleotide that is used to stabilize the open double-stranded DNA circle during the step of generating mate-pair polynucleotide arms).

One embodiment of a clamp adapter is illustrated in Figure 17 and in SEQ ID NO:10. This adapter, referred to as "Ad191," has a length of 76 nucleotides and includes the following features: inverted repeat sequences; a tag or barcode sequence; a "stuffer" sequence for reading barcodes or tags with cPAL chemistry; anchor probe hybridization sequences; an intruder hybridization sequence; an SBS sequencing primer hybridization sequence; an RCR primer hybridization sequence, and an SBS primer hybridization sequence for reading barcodes or tags with SBS chemistry. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:10. In some embodiments, an adapter has the polynucleotide sequence of SEQ ID NO:10.

Another embodiment of a clamp adapter is illustrated in Figure 18 and in SEQ ID NO:11. This adapter, referred to as "Ad212," has a length of 44 nucleotides and includes the following features: an SBS primer for reading barcodes/tags and target polynucleotide; and a tag/barcode sequence. In some embodiments, an adapter has a polynucleotide sequence that is substantially identical (e.g., is at least 70%, 75%, 80%, 85%, 90%, 91%, 92%, 93%, 94%, 95%, 96%, 97%, 98%, or 99% identical) to SEQ ID NO:11. In some embodiments, an adapter has the polynucleotide sequence of SEQ ID NO: 11.

### 3.6 Combinations of Different Types of Adapters

In some embodiments, a mate-pair polynucleotide construct (e.g., a circular mate-pair construct suitable for concatemerization) comprises two adapters that are different types of adapters as described herein. In some embodiments, a mate-pair polynucleotide construct comprises a first adapter that is a clamp adapter and a second adapter that is a bubble adapter. In some embodiments, a mate-pair polynucleotide construct comprises a first adapter that is a bubble adapter and a second adapter that is a clamp adapter. The first adapter and the second adapter can include the same features or at least some of the same features (e.g., restriction endonuclease recognition sequences, PCR primer hybridization sequences, sequencing primer hybridization sequences, anchor probe hybridization sequences, RCR primer hybridization sequences, intruder hybridization sequences, tag or barcode sequences, and stuffer sequences). In some embodiments, the first adapter and the second adapter include some, but not all, of the same features. As a non-limiting example, in some embodiments, the bubble adapter comprises an inverted repeat sequence while the clamp adapter does not include an inverted repeat sequence.

### 4. First Adapter Ligation and Circularization

### 4.1 Modification of Polynucleotide Fragments

In some embodiments, prior to ligating the first adapter to the polynucleotide fragments, the polynucleotide fragments are modified in order to make their ends compatible for ligation with the first adapter. As a non-limiting example, in some embodiments, the polynucleotide fragments may contain 5' and/or 3' protruding ends, and phosphate groups may be present or may be absent at the 5' and/or 3' ends. In some embodiments, prior to ligating the first adapter to fragmented DNA, the ends of the DNA fragments can be modified by generating sticky ends for use in A-T ligation. As another non-limiting example, in some embodiments, prior to ligating the first adapter to fragmented DNA, the ends of the DNA fragments can be modified by generating blunt dephosporylated ends for use in blunt-end ligation. As yet another non-limiting example, in some embodiments, prior to ligating the first adapter to fragmented DNA, the DNA is denatured into single-stranded form.

In some embodiments, modification of the polynucleotide fragments results in DNA fragments having 5' phosphorylated blunt-ends. One of skill in the art will understand how to generate 5' phosphorylated blunt-ended DNA (e.g., by adding phosphate groups to 5' ends of the DNA fragments, regenerating hydroxyl groups to 3' ends of DNA, filling in recessed 3' ends, and/or removing protruding 3' ends as necessary). One of skill in the art can identify suitable enzymes (e.g., kinases and polymerases) for making 5' phosphorylated blunt-ended DNA, e.g., T4 Polynucleotide Kinase (T4 PNK), T4 DNA Polymerase, Klenow Large Fragment, *E. coli* DNA Polymerase I, *E. coli* DNA Polymerase I Large Fragment, Taq Polymerase, Bst Polymerase Full Length, Bst Polymerase Large Fragment, Bsu DNA Polymerase Large Fragment, and combinations thereof. In some embodiments, one or more deoxyadenosines ("dA") are then added to the 3' ends of the 5' phosphorylated blunt-end DNA fragments, using a DNA polymerase, to produce a 3' overhang or "tail." In some embodiments, a single dA is added to the 3' ends. In some embodiments, Taq polymerase, Klenow exo⁻, Bsu DNA Polymerase Large Fragment, or a combination thereof is used for dA-tailing the DNA fragments. In some embodiments, the 3' overhang modified DNA fragments are used for ligating with a first adapter that is a bubble adapter.

In some embodiments, modification of the polynucleotide fragments results in DNA fragments having dephosphorylated blunt-ends. DNA fragments having dephosphorylated blunt-ends can be useful, e.g., for preventing the ligation of DNA fragments to each other rather than to the first adapter. One of skill in the art will understand how to generate dephosphorylated blunt-ended DNA (e.g., by removing phosphate groups from 5' and/or 3' ends, filling in recessed 3' ends, and/or removing protruding 3' ends as necessary). One of skill in the art can identify suitable enzymes (e.g., phosphatases and polymerases) for making dephosphorylated blunt-ended DNA, e.g., shrimp alkaline phosphatase, T4 DNA polymerase, Klenow Large Fragment, *E. coli* DNA Polymerase I, *E. coli* DNA Polymerase I Large Fragment, Taq Polymerase, Bst Polymerase Full Length, Bst Polymerase Large Fragment, Bsu DNA Polymerase Large Fragment, and combinations thereof. In some embodiments, the dephosphorylated blunt-end DNA fragments are used for ligating with a first adapter that is an L-oligo adapter.

In some embodiments, modification of the polynucleotide fragments comprises denaturing a double-stranded DNA fragment into single strands (e.g., by heat denaturation). In some embodiments, the 5' ends of single-stranded DNA fragments are phosphorylated. One of skill in the art will recognize suitable enzymes (e.g., kinases, e.g., T4 PNK) for phosphorylating 5' ends. One of skill in the art will also recognize that double-stranded DNA fragments can be denatured after end-repair of the DNA fragments (e.g., after blunt-end repair using a combination of T4 Polymerase and T4 PNK to produce 5' phosphorylated ends), or that double-stranded DNA fragments can be denatured prior to end-repair of the DNA fragments (e.g., denaturing the DNA fragments into single-stranded DNA, then sequentially treating the single-stranded DNA with a phosphatase and a kinase to remove 3' phosphates and add 5' phosphates). In some embodiments, the 5' phosphorylated single-stranded DNA fragments are used for ligating with a first adapter that is a clamp adapter.

### 4.2 Ligation

### 4.2.1 Bubble Adapter Ligation

In some embodiments, the first adapter that is ligated to the polynucleotide fragments is a bubble adapter. For ligating a DNA fragment with a first adapter that is a bubble adapter, the first oligonucleotide and the second oligonucleotide of the first bubble adapter are annealed to the modified (e.g., dA-tailed DNA) fragment to form a double-stranded linear construct comprising the DNA fragment flanked on both sides by a duplex of the first adapter oligonucleotides. The ligation reaction is performed using a suitable ligase enzyme. In some embodiments, T4 DNA ligase is used. An exemplary schematic depicting the ligation of a bubble adapter to a DNA fragment is shown in Figure 4.

### 4.2.2 L-oligo Adapter Ligation

In some embodiments, the first adapter that is ligated to the polynucleotide fragments is an L-oligo adapter. For ligating a DNA fragment with a first adapter that is an L-oligo adapter, a two-step process is used. First, the second oligonucleotide of the first L-adapter is ligated to the modified (e.g., dephosphorylated blunt-ended) fragment in the presence of a short (about 8-9 bases in length) helper oligonucleotide having a 3'-end modification (e.g., a 3-dN-Q modification, Eurofin-MWG-Operon, where N is any base). The ligation reaction is performed using a suitable ligase enzyme. In some embodiments, T4 DNA ligase is used. The ligase is inactivated (e.g., in a heat-kill step) and the helper oligonucleotide is removed from the ligation product, as it has a low melting temperature. A phosphate group is then added to the 5' ends of the ligation product. The phosphorylation is carried out using any suitable enzyme. In some embodiments, T4 PNK is used to phosphorylate the 5' ends. A second ligation step is then carried out to ligate the phosphorylated ligation product to the first oligonucleotide of the first L-oligo adapter, to form a double-stranded linear construct comprising the DNA fragment flanked on both sides by a duplex of the first adapter oligonucleotides. The ligation reaction is performed using a suitable ligase enzyme (e.g., T3 DNA ligase, T4 DNA ligase, T7 DNA ligase, Chlorella virus DNA ligase (SplintR®, New England Biolabs, Inc., Ipswich, MA), or Taq DNA ligase). In some embodiments, T4 DNA ligase is used. An exemplary schematic depicting the ligation of an L-oligo adapter to a DNA fragment is shown in Figure 4.

### 4.2.3 Clamp Adapter Ligation

In some embodiments, the first adapter that is ligated to the polynucleotide fragments is a clamp adapter. For ligating a DNA fragment with a first adapter that is a clamp adapter, the first oligonucleotide and the second oligonucleotide of the first clamp adapter are annealed to the modified (e.g., single-stranded and 5' phosphorylated) DNA fragment in the presence of a first helper oligonucleotide and a second oligonucleotide. Each helper oligonucleotide has the sequence (N)₅(I)ₙ, and the first helper oligonucleotide and the second helper oligonucleotide sequence have different sequences. The resulting construct is a single-stranded linear construct comprising the DNA fragment flanked on one side by a duplex comprising the first adapter oligonucleotide and a helper oligonucleotide, and flanked on the other side by a duplex comprising the second adapter oligonucleotide and a helper oligonucleotide. The ligation reaction is performed using a suitable ligase enzyme (e.g., T3 DNA ligase, T4 DNA ligase, T7 DNA ligase, Chlorella virus DNA ligase (SplintR®, New England Biolabs, Inc., Ipswich, MA), or Taq DNA ligase). In some embodiments, T4 DNA ligase is used. An exemplary schematic depicting the ligation of a clamp adapter to a DNA fragment is shown in Figure 4.

### 4.3 Amplification and Circularization

Following the ligation step, the resulting linear construct comprising the DNA fragment flanked on both sides by the first adapter oligonucleotides is amplified by PCR. The amplification is performed using primers that contain uracil residues and that hybridize within the adapter region. The polymerase that is used for the amplification reaction is a polymerase that tolerates that presence of uracils in a template. In some embodiments, PfuTurbo® Cx DNA polymerase or KAPA HiFi HotStart Uracil+ DNA polymerase is used for amplifying the double-stranded oligonucleotide duplex-DNA fragment construct. The resulting amplification product is a double-stranded construct comprising the DNA fragment and the first oligonucleotide and second oligonucleotide of the first adapter, wherein each strand of the DNA fragment is flanked by the first oligonucleotide of the first adapter on one end and the second oligonucleotide of the first adapter one the other end. In some embodiments, the amplification product further comprises one or more uracil residues in each strand of the double-stranded construct.

Optionally, one or more tags or barcodes can be added to the first adapter during the amplification reaction. Typically, a tag or barcode sequence is added using a primer that comprises the tag or barcode sequence. In some embodiments, the tag or barcode sequence is about 4 to about 15 bases in length (e.g., 4, 5, 6, 7, 8, 9, 10, 11, 12, 13, 14, or 15 bases in length). Methods of introducing tag or barcode sequences during an amplification reaction are known in the art See, e.g., US 8,691,509; US 8,841,071; and US 8,921,076.

The amplified product is then treated with an enzyme that specifically excises uracil bases, which results in the creation of a single nucleotide gap at the location of each uracil in the double-stranded construct. In some embodiments, the enzyme that is used to create gaps at the uracil sites is uracil DNA glycosylase or USER™ (Uracil-Specific Excision Reagent) enzyme.

The amplified and uracil-specific excising enzyme-treated products subsequently circularize to form a circular double-stranded polynucleotide fragment with "sticky" ends in the region of the first adapter where the uracil residues were excised (referred to herein as an "open double-stranded circular polynucleotide construct"). In some embodiments, the excising of uracils results in a nick in each polynucleotide strand or a gap in each polynucleotide strand that is from about 1 to about 10 bases in length. In some embodiments, the gap in each polynucleotide strand is about 2 bases in length.

An exemplary schematic depicting the amplification and formation of the open double-stranded circular polynucleotide construct is shown in Figure 19. As shown in Figure 19, the structure of the open double-stranded circular polynucleotide construct is such that the gap on one polynucleotide strand does not overlap with the gap on the other polynucleotide strand, and in between the regions of the first adapter that have the gaps, there is a region of overlapping (complementary) sequence that is sufficient to stabilize the open double-stranded circle. The region of overlapping sequence can be from about 8 to about 20 bases in length. In some embodiments, the region of overlapping sequence is from about 12 to about 14 bases in length.

In some embodiments, the reaction product of the DNA circularization reaction is purified to remove contaminating non-circularized linear DNA fragments. In some embodiments, the reaction product is treated with a DNase that specifically digests linear double-stranded DNA but not circular or nicked circular double-stranded DNA. In some embodiments, the reaction product is treated with Plasmid-Safe™ ATP-Dependent DNase (Epicentre, Madison, WI) or Exonuclease V (RecBCD) (New England Biolabs, Inc.).

### 5. Generation of Mate-Pair Library Arms (ttCNT/Exo)

The open double-stranded circular polynucleotide construct comprising the first adapter is used as a template for the generation of polynucleotide "arms" that extend from each end of the first adapter. In the open double-stranded circular polynucleotide construct, the ends of the fragmented DNA, i.e., the "mate-pair," are separated by the first adapter. Polynucleotide arms are synthesized from each end of the first adapter, into a portion of the fragmented DNA sequence starting at the ends of the fragmented DNA, and the middle portion of the fragmented DNA sequence is removed, thereby generating mate-pair polynucleotide arms that are attached to each end of the first adapter.

In some embodiments, each polynucleotide arm comprises a length of about 50-150 bases, about 60-120 bases, or about 80-100 bases (e.g., about 50, about 60, about 70, about 80, about 90, about 100, about 110, about 120, about 130, about 140, about 150 bases).

In some embodiments, for a construct comprising a mate-pair of polynucleotide arms attached to a first adapter, each polynucleotide arm has a length of about 40-150 bases, about 60-120 bases, or about 80-100 bases (e.g., about 40, about 50, about 60, about 70, about 80, about 90, about 100, about 110, about 120, about 130, about 140, or about 150 bases); and the first adapter has a length of about 50-100 bases, about 60-90 bases, about 70-80 bases, about 60-70 bases, or about 80-90 bases (e.g., about 50, about 60, about 70, about 80, about 90, or about 100 bases). In some embodiments, the construct comprising a mate-pair of polynucleotide arms attached to a first adapter has a length of about 150-400 bases, about 150-300 bases, about 180-300 bases, about 180-280 bases, about 180-250 bases, about 200-300 bases, about 200-280 bases, about 250-350 bases, about 230-330 bases, or about 200-250 bases.

### 5.1 Time and Temperature Controlled Nick Translation

In some embodiments, the generation of polynucleotide arms extending from each end of the first adapter is carried out by a process of time and temperature controlled nick translation (ttCNT). Typically, the process involves a DNA polymerase-driven synthesis reaction on the open double-stranded circular polynucleotide construct. For each strand of the construct, this polymerase reaction results in moving the nick, in a 5' to 3' direction, from the gap in the region of the first adapter towards and then along the DNA fragment that is ligated to the first adapter. As the nick moves along the DNA fragment, the DNA polymerase synthesizes a polynucleotide arm that is attached to the first adapter. *See, e.g.,* Figure 19.

In time and temperature controlled nick translation, polymerase-driven DNA synthesis in the 5' to 3' direction is controlled by optimizing the time and temperature of the nick translation reaction in a non-limiting concentration of dNTPs. The time and temperature conditions are optimized for the particular polymerase being used for the nick translation reaction. Thus, in time and temperature controlled nick translation, the length of each polynucleotide arm attached to the first adapter can be controlled by modulating the progression of DNA synthesis.

In some embodiments, time and temperature controlled nick translation is carried out using Taq Polymerase, *E. coli* DNA Polymerase I, Bst DNA Polymerase Full Length, LongAmp® Taq DNA Polymerase (New England Biolabs, Inc.), or OneTaq® DNA Polymerase (New England Biolabs, Inc.). In some embodiments, Taq Polymerase, LongAmp® Taq DNA Polymerase, or OneTaq® DNA Polymerase is used. The optimal time and temperature for the nick translation reaction can vary based on the polymerase that is used. In some embodiments, the nick translation reaction occurs at a temperature of about 37°C to about 72°C (e.g., about 37°, about 40°, about 45°, about 50° about 55°, about 60°, about 65°, about 70°, or about 72°C). In some embodiments, the nick translation reaction is carried out for about 10 to about 120 seconds (e.g., about 10, about 20, about 30, about 40, about 50, about 60, about 70, about 80, about 90, about 100, about 110, or about 120 seconds). In some embodiments, time and temperature controlled nick translation is carried out using Taq Polymerase for about 10 to about 120 seconds, at a temperature of about 45°C.

DNA synthesis by time and temperature controlled nick translation can be stopped by incubating the reaction on ice, by chelating the available magnesium in the reaction with a chelator (e.g., EDTA at a concentration of at least about 20 mM), and/or by adding a salt (e.g., sodium chloride at a concentration of at least about 800 mM) to the reaction. In some embodiments, the time and temperature controlled nick translation reaction is stopped by adding about 20 mM EDTA to the reaction.

At the end of the DNA synthesis reaction by time and temperature controlled nick translation, the open double-stranded circular polynucleotide construct is "collapsed" by initiating nucleotide removal at the sites of the nicks in the construct and proceeding in the 5' to 3' direction of each strand, thereby creating a linear construct that is partially double-stranded (at the region where the first adapter is located and where the polynucleotide arms were synthesized) and that has single-stranded tails on either 5' end. In some embodiments, T7 exonuclease is used to remove the nucleotides and create the 5' single-stranded tails.

The 5' single-stranded DNA tail is then removed from the construct using a nuclease that degrades single-stranded nucleic acids. In some embodiments, Mung Bean Nuclease, S1 nuclease, Exonuclease VII, or T7 Endonuclease I may be used for removing the 5' single-stranded ends. The resulting construct is a double-stranded linear construct in which each strand comprises the first adapter flanked by polynucleotide arms that are a mate pair of nucleic acid sequences (referred to herein as a "linear mate-pair construct").

The optimal reaction conditions (e.g., time, temperature, and units) for removing the 5' single-stranded DNA tail can vary based on the nuclease that is used. For example, for S1 nuclease, exemplary conditions include: 5-20 U/pmol at about 23°C for about 15 minutes; 5-20 U/pmol at about 12°C for about 30 minutes; or 5-20 U/pmol at about 4°C for about 60 minutes. For Exonuclease VII, exemplary conditions include: 0.4-12 U/pmol at about 37°C for about 30 minutes. For Mung Bean Nuclease, exemplary conditions include: 1-7 U/pmol at about 22°C for about 30 minutes; or about 4-32 U/pmol at about 37°C for about 15 minutes. For T7 Endonuclease I, exemplary conditions include: 1-4 U/pmol at about 23°C for about 30 minutes; 1-4 U/pmol at about 30°C for about 30 minutes; or 1-4 U/pmol at about 37°C for about 15 minutes.

### 5.2 Controlled Extension

In some embodiments, the generation of polynucleotide arms extending from each end of the first adapter is carried out by a process of controlled extension. Typically, the process involves conducting first an exonuclease reaction at the nick or gap on each strand of the open double-stranded circular polynucleotide construct to generate a construct that is single-stranded except for a region of overlapping sequence in the region of the first adapter. Subsequently, a polymerase-driven nucleic acid strand extension is conducted starting at the 3' end of the first adapter on each strand which uses the single-stranded tails as templates. The extension reaction moves in a 5' to 3' direction to synthesize a polynucleotide arm that is attached to the first adapter.

### 5.2.1 Time and Temperature Controlled Extension

In some embodiments, a mate pair construct is generated by the method of "time and temperature controlled extension." In time and temperature controlled extension, the open double-stranded circular polynucleotide construct is "collapsed" by initiating nucleotide removal by nuclease at the sites of the nicks in the construct and proceeding in the 5' to 3' direction of each strand, thereby creating a linear construct that is mostly single-stranded except for a short region of overlapping sequence (about 8 to about 20 bases in length, e.g., about 12 to 14 bases in length) in the first adapter region. In some embodiments, T7 exonuclease is used to remove the nucleotides and create the 5' single-stranded tails. In some embodiments, each single-stranded polynucleotide tail extending from the 5' end of the first adapter is about 150 to about 500 bases in length.

Polymerase-driven DNA extension from the 3' end of the first adapter on each strand is then carried out in order to extend the polynucleotide arm on each strand, resulting in a construct that comprises a double stranded first adapter and double-stranded polynucleotide arms extending from each end of the first adapter, and which further comprises single-stranded tails at the 5' end of each strand. The polymerase-driven DNA synthesis is controlled by optimizing the time and temperature of the extension reaction in a non-limiting concentration of dNTPs. The time and temperature conditions are optimized for the particular polymerase being used for the nick translation reaction. Thus, in time and temperature controlled extension, the length of each polynucleotide arm attached to the first adapter can be controlled by modulating the progression of DNA synthesis. In some embodiments, time and temperature controlled extension is carried out using *E. coli* DNA Polymerase I, *E. coli* DNA Polymerase I Large Fragment, Taq Polymerase, Bst DNA Polymerase Large Fragment, Bst DNA Polymerase Full Length, Bsu DNA Polymerase Large Fragment, T4 DNA Polymerase Exo-, phi29 WT, phi29 M1 mutant, phi29 M6 mutant, phi29 M8 mutant, Sulfolobus DNA Polymerase IV, Bst 2.0 DNAPolymerase, Bst 2.0 WarmStart® DNA Polymerase (New Englands Biolabs, Inc.), LongAmp® Taq DNA Polymerase (New England Biolabs, Inc.), or OneTaq® DNA Polymerase (New England Biolabs, Inc.). In some embodiments, Taq Polymerase, Sulfolobus DNA Polymerase IV, LongAmp® Taq DNA Polymerase, or OneTaq® DNA Polymerase is used.

The optimal time and temperature for the controlled extension reaction can vary based on the polymerase that is used. In some embodiments, the controlled extension reaction occurs at a temperature of about 4°C to about 60°C (e.g., about 4°, about 10°, about 15°, about 20°, about 25°, about 30°, about 35°, about 37°, about 40°, about 45°, about 50° about 55°, about 60°C). In some embodiments, the nick translation reaction is carried out for about 10 to about 120 seconds (e.g., about 10, about 20, about 30, about 40, about 50, about 60, about 70, about 80, about 90, about 100, about 110, or about 120 seconds). Exemplary conditions include: *E. coli* DNA Polymerase I at about 4° to about 25°C for about 15 to about 120 seconds; *E. coli* DNA Polymerase I Large Fragment at about 4° to about 25°C for about 15 to about 60 seconds; Taq Polymerase, LongAmp® Taq DNA Polymerase, or OneTaq® DNA Polymerase at about 37° to about 55°C for about 10 to about 90 seconds; Bst DNA Polymerase Large Fragment, Bst DNA Polymerase Full Length, or Bst 2.0 DNA Polymerase at about 37° to about 45°C for about 10 to about 30 seconds; Bsu DNA Polymerase Large Fragment or T4 DNA Polymerase Exo- at about 4° to about 25°C for about 15 to about 60 seconds; phi29 WT, phi29 M1 mutant, phi29 M6 mutant or phi29 M8 mutant at about 4°C for about 10 to about 60 seconds; Sulfolobus DNA Polymerase IV at about 37°C for about 30 to about 90 seconds; Bst 2.0 WarmStart® DNA Polymerase at about 45°C for about 10 to about 30 seconds.

DNA synthesis by time and temperature controlled extension can be stopped by chelating the available magnesium in the reaction with a chelator (e.g., EDTA at a concentration of at least about 20 mM), and/or by adding a salt (e.g., sodium chloride at a concentration of at least about 800 mM) to the reaction.

Following the extension reaction, the 5' single-stranded tails are removed using a nuclease that degrades single-stranded nucleic acids. In some embodiments, mung bean nuclease, S1 nuclease, Exonuclease VII, or T7 Endonuclease I is used for removing the 5' single-stranded ends. The resulting construct is a double-stranded linear construct in which each strand comprises the first adapter flanked by polynucleotide arms that are a mate pair of nucleic acid sequences (referred to herein as a "linear mate-pair construct").

The optimal reaction conditions (e.g., time, temperature, and units) for removing the 5' single-stranded DNA tail can vary based on the nuclease that is used. For example, for S1 nuclease, exemplary conditions include: 5-20 U/pmol at about 23°C for about 15 minutes; 5-20 U/pmol at about 12°C for about 30 minutes; or 5-20 U/pmol at about 4°C for about 60 minutes. For Exonuclease VII, exemplary conditions include: 0.4-12 U/pmol at about 37°C for about 30 minutes. For Mung Bean Nuclease, exemplary conditions include: 1-7 U/pmol at about 22°C for about 30 minutes; or about 4-32 U/pmol at about 37°C for about 15 minutes. For T7 Endonuclease I, exemplary conditions include: 1-4 U/pmol at about 23°C for about 30 minutes; 1-4 U/pmol at about 30°C for about 30 minutes; or 1-4 U/pmol at about 37°C for about 15 minutes.

### 5.2.2 Reversible Terminator Controlled Extension

In some embodiments, a mate pair construct is generated by the method of "reversible terminator controlled extension." In reversible terminator controlled extension, as in time and temperature controlled extension, the open double-stranded circular polynucleotide construct is "collapsed" by initiating nucleotide removal at the sites of the nicks or gaps in the construct and proceeding in the 5' to 3' direction of each strand, thereby creating a linear construct that is mostly single-stranded except for a short region of overlapping sequence (about 8 to about 20 bases in length, e.g., about 12 to 14 bases in length) in the first adapter region. In some embodiments, T7 exonuclease is used to remove the nucleotides and create the 5' single-stranded tails. In some embodiments, each single-stranded polynucleotide tail extending from the 5' end of the first adapter is about 150 to about 500 nucleotides in length.

Polymerase-driven DNA extension from the 3' end of the first adapter on each strand is then carried out in order to extend the polynucleotide arm on each strand, resulting in a construct that comprises a double stranded first adapter and double-stranded polynucleotide arms extending from each end of the first adapter, and which further comprises single-stranded tails at the 5' end of each strand. In reversible terminator controlled extension, the polymerase-driven DNA synthesis is controlled by optimizing the ratio of reversible terminators to dNTPs. The reversible terminators can be, for example, from the group of 3'-OH blocked reversible terminators (e.g., 3'-O-azidomethyl reversible terminators; 3'-O-NH2 reversible terminators, and 3'-O-allyl reversible terminators) or from the group of 3'-OH unblocked reversible terminators (e.g., "virtual terminators," developed by Helicos BioSciences Corporation, and "lightning terminators," 2-nitrobenzyl alkylated terminators developed by Michael L. Metzker's group). DNA synthesis stops when all growing chains are terminated by the incorporation of the reversible terminators. DNA synthesis can be reinitiated by treatment with THPP (Tris(3-hydroxypropyl)phosphine), which makes the 3' hydroxyl groups available for further polynucleotide extension. Thus, in reversible terminator controlled extension, the length of each polynucleotide arm attached to the first adapter can be controlled by modulating the progression of DNA synthesis. In some embodiments, reversible terminator controlled extension is carried out using Thermo Sequenase™ (GE Healthcare, Pittsburg, PA), T7 Sequenase™ 2.0 (GE Healthcare), Therminator™ (New England Biolabs, Inc.), Therminator™ IX, or custom polymerase. The DNA synthesis reaction is stopped automatically when the polymerase incorporates a reversible terminator nucleotide.

The optimal conditions (e.g., the ratio of reversible terminators to natural nucleotides, time, and temperature) for the reversible terminator controlled extension reaction can vary based on the polymerase that is used. In some embodiments, a ratio of about 1:20 to about 1:500 reversible terminators to natural nucleotides (e.g., about 1:20, about 1:30, about 1:40, about 1:50, about 1:60, about 1:70, about 1:80, about 1:90, about 1:100, about 1:150, about 1:200, about 1:250, about 1:300, about 1:350, about 1:400, about 1:450, or about 1:500 reversible terminators to natural nucleotides) is used. Exemplary conditions include: Thermo Sequenase™ with a 1:200-1:600 ratio of reversible terminators:natural nucleotides, at about 72°C for about 1-5 minutes; T7 Sequenase™ 2.0 with a 1:20-1:100 ratio of reversible terminators:natural nucleotides, at about 37°C for 30 seconds-2 minutes; Therminator™ at a 1:5-1:20 ratio of reversible terminators:natural nucleotides, at about 72°C for 1-5 minutes; Therminator™ IX with a 1:40-1:400 ratio of reversible terminators:natural nucleotides, at about 72°C for 1-5 minutes; or custom polymerase with a 1:50-1:300 ratio of reversible terminators:natural nucleotides, at about 37°C for about 5 minutes or at about 60°C for about 5 minutes.

Following the controlled extension reaction, the 5' single-stranded tails are removed using a nuclease that degrades single-stranded nucleic acids. In some embodiments, mung bean nuclease, S1 nuclease, Exonuclease VII, or T7 Endonuclease I is used for removing the 5' single-stranded ends. The optimal reaction conditions (e.g., time, temperature, and units) for removing the 5' single-stranded DNA tail can vary based on the nuclease that is used. For example, for S1 nuclease, exemplary conditions include: 5-20 U/pmol at about 23°C for about 15 minutes; 5-20 U/pmol at about 12°C for about 30 minutes; or 5-20 U/pmol at about 4°C for about 60 minutes. For Exonuclease VII, exemplary conditions include: 0.4-12 U/pmol at about 37°C for about 30 minutes. For Mung Bean Nuclease, exemplary conditions include: 1-7 U/pmol at about 22°C for about 30 minutes; or about 4-32 U/pmol at about 37°C for about 15 minutes For T7 Endonuclease I, exemplary conditions include: 1-4 U/pmol at about 23°C for about 30 minutes; 1-4 U/pmol at about 30°C for about 30 minutes; or 1-4 U/pmol at about 37°C for about 15 minutes.

The resulting construct is a double-stranded linear construct in which each strand comprises the first adapter flanked by polynucleotide arms that are a mate pair of nucleic acid sequences (referred to herein as a "linear mate-pair construct"). This linear mate-pair construct has 3' terminators that need to be chemically treated with THPP (Tris(3-hydroxypropyl)phosphine) to generate 3' hydroxyls required for the ligation to the second adapter. In some embodiments, about 4-20 mM THPP is added to the reaction, followed by treatment at 55°C for about 10 minutes. Following this treatment, the linear mate-pair construct can be ligated to the second adapter or modified in preparation for ligation to the second adapter.

### 6. Second Adapter Ligation

### 6.1 Modification of Polynucleotide Fragments

In some embodiments, prior to ligating the second adapter to the linear mate-pair construct, the linear mate-pair construct is modified in order to make the ends compatible for ligation with the second adapter. For example, in some embodiments, modifications result in a linear mate-pair construct having "sticky" ends for use in A-T ligation. One of skill in the art will understand how to end-repair and add A-tails to constructs for use in A-T ligation (e.g., by filling in recessed 3' ends and removing protruding 3' ends as necessary, and by adding one or more deoxyadenosines to the 3' ends). One of skill in the art can identify suitable enzymes for end repair and A-tailing (e.g., polymerases, e.g., T4 DNA polymerase and/or Klenow Large Fragment; or Klenow Exo⁻). In some embodiments, the tail of the modified construct comprises a single dA. In some embodiments, end-repair and A-tailing processes are carried out in separate reactions. In some embodiments, end-repair and A-tailing processes are carried out in a single reaction. In some embodiments, end-repair and A-tailing processes are carried out in a single reaction using one enzyme (e.g., Klenow Exo⁻). In some embodiments, the A-tailed modified DNA fragments are used for ligating with a second adapter that is a bubble adapter.

In some embodiments, prior to ligating the second adapter to the linear mate-pair construct, modified constructs have dephosphorylated blunt ends that are suitable for use in blunt-end ligation. One of skill in the art will understand how to generate dephosphorylated blunt-ended DNA (e.g., by removing phosphate groups from 5' and/or 3' ends, filling in recessed 3' ends, and/or removing protruding 3' ends as necessary). One of skill in the art can identify suitable enzymes (e.g., phosphatases and polymerases) for making dephosphorylated blunt-ended DNA, e.g., shrimp alkaline phosphatase, T4 DNA polymerase, Klenow Large Fragment, *E. coli* DNA Polymerase I, *E. coli* DNA Polymerase I Large Fragment, Taq Polymerase, Bst Polymerase Full Length, Bst Polymerase Large Fragment, Bsu DNA Polymerase Large Fragment, and combinations thereof. In some embodiments, the dephosphorylated blunt-end DNA fragments are used for ligating with a second adapter that is an L-oligo adapter.

In some embodiments, the linear mate-pair construct is modified by denaturing the construct into a single-stranded form (e.g., by heat denaturation) prior to ligating the second adapter. In some embodiments, the single-stranded construct is used directly, without prior DNA repair, for ligating with a second adapter that is a clamp adapter, as the post-nick translation nuclease trimming of the nick translation products results in linear mate-pair constructs having 5' phosphates and 3' hydroxyls.

### 6.2 Ligation

### 6.2.1 Bubble Adapter Ligation

In some embodiments, the second adapter that is ligated to the modified linear mate-pair construct is a bubble adapter. The first oligonucleotide and the second oligonucleotide of the second bubble adapter are annealed and ligated to the modified (e.g., A-tailed) linear mate-pair construct to form a double-stranded linear construct comprising the mate pair of polynucleotide arms separated at by the first adapter and flanked on both sides by a duplex of the second adapter oligonucleotides. The ligation reaction is performed using a suitable ligase enzyme. In some embodiments, T4 DNA ligase is used.

### 6.2.2 L-oligo Adapter Ligation

For ligating the modified linear mate-pair construct to a second adapter that is an L-oligo adapter, a two-step process is used. First, the second oligonucleotide of the second L-adapter is ligated to the modified (e.g., dephosphorylated blunt-ended) fragment in the presence of a short (about 8-9 nucleotide) helper oligonucleotide having a 3'-end modification (e.g., a 3-dN-Q modification, Eurofin-MWG-Operon, wherein N is any of A, T, G, or C). The ligation reaction is performed using a suitable ligase enzyme. In some embodiments, T4 DNA ligase is used. The ligase is inactivated (e.g., in a heat-kill step) and the helper oligonucleotide is removed from the ligation product. A phosphate group is then added to the 5' ends of the ligation product. The phosphorylation is carried out using any suitable enzyme. In some embodiments, T4 PNK is used to phosphorylate the 5' ends. A second ligation step is then carried out to ligate the phosphorylated ligation product to the first oligonucleotide of the second L-oligo adapter, to form a double-stranded linear construct comprising the mate pair of polynucleotide arms separated by the first adapter and flanked on both sides by a duplex of the second adapter oligonucleotides. The ligation reaction is performed using a suitable ligase enzyme. In some embodiments, T4 DNA ligase is used.

### 6.2.3 Clamp Adapter Ligation

In some embodiments, the second adapter that is ligated to the polynucleotide fragments is a clamp adapter. The first oligonucleotide and the second oligonucleotide of the second clamp adapter are annealed to the modified (e.g., single-stranded) linear mate-pair construct in the presence of a first helper oligonucleotide and a second helper oligonucleotide. Each helper oligonucleotide has the sequence (N)₅(I)ₙ, and the first helper oligonucleotide and the second helper oligonucleotide sequence are different sequences. The resulting construct is a single-stranded linear construct comprising the mate pair of polynucleotide arms separated by the first adapter and flanked on both sides by the second adapter oligonucleotides. The ligation reaction is performed using a suitable ligase enzyme (e.g., T3 DNA ligase, T4 DNA ligase, T7 DNA ligase, or Chlorella virus DNA ligase (SplintR®, New England Biolabs, Inc.). In some embodiments, T4 DNA ligase is used.

### 6.3 Amplification

After the ligation reaction, the linear mate-pair construct, comprising the mate pair of polynucleotide arms separated by the first adapter and flanked on both sides by the second adapter oligonucleotides, is amplified by PCR. In some embodiments, the PCR polymerase is a polymerase that produces blunt-ended PCR products. In some embodiments, the PCR polymerase Q5® DNA polymerase is used. In some embodiments, one of the primers that is used in the amplification reaction is 5' phosphorylated in order to allow for strand-specific circularization and ligation of the amplification product (e.g., in order to select for strands having a desired adapter orientation). For example, in some embodiments, the primer that is 5'-phosphorylated is a primer that hybridizes to a 5' region of the second adapter.

Optionally, one or more tags or barcodes can be added to the second adapter during the amplification reaction. Typically, a tag or barcode sequence is included in the PCR primer that comprises the tag or barcode sequence. In some embodiments, the tag or barcode sequence is about 4 to about 15 bases in length (e.g., 4, 5, 6, 7, 8, 9, 10, 11, 12, 13, 14, or 15 bases in length). Methods of introducing tag or barcode sequences during an amplification reaction are known in the art. See, e.g., US 8,691,509; US 8,841,071; and US 8,921,076.

### 6.4 Circularization of Amplification Product

Following amplification of the double-stranded linear construct, the amplification products are denatured to separate the products into single-stranded polynucleotides. Denaturation can be accomplished, for example, by heat denaturation, chemical denaturation, or by the use of biotin/streptavidin labeling to specifically capture one of the two strands of an amplified product. In some embodiments, the amplification products are heat denatured by heating the amplification product at 95°C for about 3 minutes, followed by snap-cooling on ice for about 2 minutes or fast-ramp (4°C/second) down to 4°C for about 10 minutes. In some embodiments, the amplification products are chemically denatured by treatment with 75 mM potassium hydroxide or 110 mM sodium hydroxide. In some embodiments, the amplification products are separated into single-stranded polynucleotides by biotinylating one strand of a PCR product (e.g., biotinylating an unwanted strand and leaving a desired strand carrying a 5' phosphate unlabeled) and capturing the biotinylated strand with streptavidin magnetic beads.

The single-stranded polynucleotides are then circularized. In some embodiments, a DNA ligase (e.g., T4 DNA ligase) is used to circularize the single-stranded polynucleotides. In some embodiments, the single-stranded polynucleotides are denatured and circularized in the presence of a "splint" oligonucleotide that serves as a template to covalently close the single-stranded polynucleotides. The splint oligonucleotide comprises a first portion that is complementary to the first oligonucleotide of the second adapter and a second portion that is complementary to the second oligonucleotide of the second adapter. In some embodiments, each of the first portion and the second portion of the splint oligonucleotide is at least 10 bases in length (e.g., at least 10, at least 11, at least 12, at least 13, at least 14, at least 15, at least 16, at least 17, at least 18, at least 19, or at least 20 bases in length). In some embodiments, each of the first portion and the second portion of the splint oligonucleotide is at least 12 bases in length.

Following the circularization of the single-stranded polynucleotides, the products of the circularization reaction can be treated with one or more exonucleases to remove non-circularized linear strands, to remove splint oligonucleotides that remain annealed to the single-stranded circular constructs, and to remove excess free (non-annealed) splint oligonucleotides. Suitable enzymes for removing the components other than single-stranded circular constructs can be determined by one of skill in the art. In some embodiments, Exonuclease I, Exonuclease III, Exonuclease VII, T7 Exonuclease, or RecJ Exonuclease can be used. In some embodiments, Exonuclease I, Exonuclease III, or a combination thereof is used. In an exemplary embodiment, Exonuclease I and Exonuclease III are added to the single-stranded circularization reaction for a final concentration of 0.5-2 U/µl, followed by incubation at 37°C for about 30 minutes, then 20 mM EDTA is added to stop the reaction.

The single-stranded circular polynucleotide construct that is formed comprises the mate pair of polynucleotide arms, the first adapter, and the second adapter. In this circular single-stranded mate-pair construct, each polynucleotide arm is attached to the first adapter on one end and the second adapter on the other end. In some embodiments, the circular constructs that are generated comprise a mixture of adapter orientations within the circle (i.e., some single-stranded circular constructs will comprise one orientation of the first adapter relative to the second adapter, and other single-stranded circular constructs will comprise the reverse orientation of the first adapter relative to the second adapter). As discussed below, it is possible to select for a single orientation of the first adapter relative to the second adapter, in order to generate concatemers of circular mate-pair constructs that all have the same orientation of the first adapter and the second adapter.

In some embodiments, the circular polynucleotide construct comprising the mate pair of polynucleotide arms, the first adapter, and the second adapter has a length of about 180-550 bases, about 180-500 bases, about 180-450 bases, about 180-400 bases, about 180-350 bases, about 180-330 bases, about 200-550 bases, about 200-500 bases, about 200-450 bases, about 200-400 bases, about 200-350 bases, about 200-330 bases, about 230-550 bases, about 230-500 bases, about 230-450 bases, about 230-400 bases, about 230-350 bases, about 230-330 bases, about 250-550 bases, about 250-500 bases, about 250-450 bases, about 250-400 bases, or about 250-350 bases.

### 7. Mate-Pair Library Construction, Method Two: Two Adapter Mate Pair Libraries by Controlled Nick Translation and Controlled Primer Extension

One embodiment of the invention is a method for mate pair library construction that is termed Controlled Nick Translation (for example, nick translation controlled by nucleotide amount, ntCNT) coupled with Controlled Primer Extension (ntCNT/CPE).

As detailed below, after adding a first adapter (AdA) to genomic DNA and forming a double stranded circle (dsCir) with a nick or a gap, CNT moves the nick or gap with a selected length into the genomic DNA. 3' branch ligation (or gap ligation) is used to ligate a 5' arm of the second adapter at the resulting. Note that due to the low efficiency of ligation to a nick, either ntCNT is used or a gapping step is included after nick translation to create a gap of a few basepairs for gap ligation The two strands of dsCir DNA resulting from 3' branch ligation are optionally separated, and a single stranded DNA (ssDNA) strand is generated that includes an AdA sequence surrounded by genomic DNA (specifically, the ends of a starting genomic DNA fragment) and AdB-5' sequence at the 3' end of the genomic DNA. This ssDNA strand is used as template in a CPE reaction, resulting in a construct with a mate pair derived from the starting genomic DNA fragment. Each arm of the mate pair has a selected length (resulting from the CNT and CPE reactions, respectively), separated by AdA sequence, with AdB_5' sequences at one end of the construct. An AdB_3' sequence is then added to the other end of the construct by 3' branch ligation (in this case, a 5' overhang ligation), resulting in an amplifiable template with AdB primers at each end.

Such a construct can be used as a template for bridge PCR (as in Illumina's sequencing-by-synthesis [SBS] process), assuming the use of appropriate AdB 5' and 3' sequences. Such a construct can also be circularized and used to generate DNA nanoballs for sequencing for sequencing by cPAL, SBS or other sequencing methods.

### 7.2 3' Branch Ligation

After ntCNT, 3' branch ligation is performed to add a 3' arm of the second adapter (AdB_3').

It is well known that nicks in double stranded DNA fragments and double stranded DNA fragments with sticky or blunt ends can be joined at 5' phosphate and 3' hydroxyl groups. The ligation of sticky ends or nicks is generally faster and less dependent on enzyme concentration than blunt end ligation. Both processes can be catalyzed by bacteriophage T4 DNA ligase. T4 ligase is reported to mediate certain unconventional ligations: it seals dsDNA substrates containing an abasic site or a gap at the ligation junction, joins branched DNA strands, and forms a stem-loop product with partially double stranded DNA (Nilsson and Magnusson, Nucleic Acids Res 10:1425-1437, 1982; Goffin et al., Nucleic Acids Res 15:8755-8771, 1987; Mendel-Hartvig et al., Nucleic Acids Res. 32:e2, 2004; Western and Rose, Nucleic Acids Res., 19:809-813, 1991).

We have discovered that T4 ligase can be used to join DNA fragments at dephosphylated nicks, gaps or 5' overhang regions to form an Okazaki fragment-like structure. As illustrated in Figure 20, the insert DNA can be a synthetic linker or adapter DNA consisting of double-stranded DNA with one blunt end and one 3' overhang. Both 3' termini of the adaptors are dideoxynucleotides, which prevents self-ligation of the adapter. The 5' terminus of the long adaptor strand is phosphorylated and ligates to the 3' terminus of the substrate DNA at the gap.

The substrate DNA molecule (i.e., the target polynucleotide) contains one of the following structures: (1) a nick or (2) gap with a 3'-hydroxyl terminus (i.e., one or more missing nucleotide bases), or (3) a 5' overhang (5'-OH) (that is, 3' branch ligation encompasses nick ligation, gap ligation, and 5' overhang ligation). T4 ligase joins the 5'-phospharylated adaptor strand to the 3'-hydroxylated substrate DNA strand to form a branched DNA structure. Therefore, we name this novel ligation event a "3' branch ligation." The adapter ligated to the substrate DNA at the nick, gap or 5' overhang may be referred to as a "3' branch adapter."

We examined numerous factors that affect general ligation efficiency including: adaptor: :DNA ratio, the amount of T4 ligase, final ATP concentration, Mg²⁺ concentration, pH, incubation time and various additives. Adding polyethylene glycol (PEG) to a final concentration of 10% substantially increased the ligation efficiency from less than 10% to more than 80%. Ligation is efficient to gaps (e.g., 1, 2, 3, 4, 5, 6, 7, 8 or more bp gaps) and 5'-OH DNA. In fact, ligation to 5'-OH ligation is almost 100% complete, even higher than for blunt end ligations. Substrates with a 1 bp gap had a ligation efficiency of about 50%, and ligation efficiency is higher for longer gaps (e.g., 2 bp or longer). However, the nick ligation efficiency occurs, but at a low efficiency (less than 10%) even under optimized conditions. It is possible that the longer ssDNA region makes the 3'-OH of the substrate more accessible for ligation and therefore results in higher ligation efficiency.

Practically speaking, if the ntCNT reaction uses a DNA polymerase that has 3' exonuclease (exo) activity such as DNA Polymerase I, a 5' arm of a second adapter (AdB) can be added by ligation directly to the 3' end of the resulting gap region. If the CNT reaction uses a DNA polymerase that lacks 3' exo activity (or if ttCNT is used), a less processive exonuclease, e.g., T7 exo or Bst polymerase (Bst polymerase has exonucleoase activity; for this purpose, we use it in the absence of dNTPs), can be used to remove a few nucleotides from the 5' end of the nicks and create a gap region for AdB 3' gap ligation for more effective 3' branch ligation.

SSB (Single Strand Binding) protein (e.g., at a final concentration of 10-20 ng per microliter) also increases 3' branch ligation efficiency for an 8 bp gap and 5'-OH DNA, but has no effect on nicked or 1 bp gapped DNA. It appears that SSB proteins bind to the single stranded region and stabilizes ssDNA.

Therefore, according to one embodiment of the invention, 3' branch ligation is performed with ligation conditions that include an amount of PEG or SSB protein or a combination thereof that is effective to detectably increase ligation of the 3' branch adapter to the target polynucleotide at the ligation site. For PEG, such an effective amount includes without limitation a final concentration of 5, or 10, or 15, or 20 percent, for example. For SSB protein, such an effective amount includes without limitation a final concentration of 5, or 10, or 15, or 20 ng/µl.

### 7.3 Controlled primer extension (CPE)

Next, controlled primer extension (CPE) is carried out. As for CNT, this reaction employs a DNA polymerase, and the extent to which the primer is extended can be controlled by time and temperature (ttCPE), nucleotide amount (ntCPE), etc. The DNA synthesis is extended from a primer that hybridizes to Ad2_5' through genomic sequence, then Ad1, and finally a selected distance into genomic sequence on the other side of Ad1 from Ad2_5', resulting in a double stranded construct that includes mate-pair arms separated by Ad1 and, at the 3' end, Ad2-5'.

### 7.4 Overhang ligation (OH ligation)

The 3' half-adapter arm of Ad2, Ad2_3', can be added at the 5' end of the construct resulting from CPE by 3' branch ligation, as shown in Figure 21. The OH ligation product is then PCR amplified using AdB 5' and AdB 3' primers to produce a double stranded construct that includes mate-pair arms separated by Ad1 and half adapter arms at each end (i.e., Ad2 5' and Ad2_3').

### 7.5 Making Single Stranded Circles (ssCir)

It would be possible to use this construct for bridge PCR and sequencing by synthesis using Illumina's protocols, particularly if the appropriate Ad2 sequences were used. However, to form DNA nanoballs, the follow steps can be used. First, strand separation is performed on the double stranded PCR product, Then, ends of the single strands are joined using a splint oligonucleotide, which has sequences that hybridize to Ad2-5' and Ad2-3', then ligated using T4 ligase to create a single-stranded circle that can be used as a substrate for rolling circle replication to produce DNA nanoballs.

### 7.6 Alternative Approaches for Adding AdB

There are several alternative approaches to the addition of the second adapter (AdB). The ntCNT step could be achieved by: (a) ntCNT using E. coli DNA polymerase I ("Pol I"), or using a mixture of Pol I plus another polymerase (as discussed above); (b) ntCNT using Taq following by a gapping step mediated by Bst Pol or T7 exo; (c) ttCNT using Taq following by a gapping step; (d) nt-ttCNT using both time and temperature and limited dNTP amount by a single polymerase like Taq or combinations of polymerases. The CPE step could be achieved by (a) ttCPE using PfuCx or another single polymerase; (b) ntCPE using Taq or another single polymerase; (d) nt-ttCPE using both time and temperature and limited dNTP amount by a single polymerase like Taq or combinations of polymerases.

### 7.7 Controlled reactions using a DNA polymerase (CNT/CPE/CSD)

We have discussed various ways to control the pace and/or extent of reactions involving DNA polymerases, including without limitation control by time and temperature, nucleotide amount, reversible terminators, etc. Such controlled reactions include, without limitation, nick translation (CNT), extension from a 3' end of a strand or primer (CE and CPE) and strand displacement (SD). The methods described in detail herein for control of one of these reactions apply generally to all.

An issue in these reactions reactions is the uniformity of amplification of all sequences. DNA Pol I tends to pause at certain DNA regions, which can stop the nick translation process and result in GC bias in the resulting library. In order to solve this problem, we have used several approaches:
1. For ntCNT reactions, instead of using dNTPs in an equal ratio, we have used two dNTPs in a sufficient or excess amount and two dNTPs in a limited amount. ntCNT reactions with excess A and T (i.e., using G and C as the limiting nucleotides) result in better amplification of GC-rich regions. For moving a pmol of DNA for about 50-100 bp, a 60 ul reaction was supplemented with 17 to 19 pmol of dGTP and dCTP each, and 34 to 38 pmol of dATP and dTTP each. One can also use additives that are known to suppress polymerase pausing and enhance the amplification of GC-rich regions, such as betaine, ethylene glycol, 1,2-propanediol, SSB, etc.
2. Mixing DNA Pol I or DNA Pol I, large (Klenow) Fragment with one or more different DNA polymerases (e.g., Taq or Bst polymerase) can bypass pausing sites that interfere with amplification mediated by DNA Pol I.
3. The nick translation reaction is composed of two enzymatic steps: degrading the old strand then synthesizing the new strand. In addition to biased polymerase activity, the exonuclease activity of DNA Pol I for DNA degradation may lead to biased amplification. This bias can be mitigated in CNT reactions by adding a less processive enzyme that has 5' to 3' exonuclease activity before or within nick translated DNA to degrade the old strand before or along with Pol I's exonuclease step.

### 8. Concatamerization

In one aspect, the circular mate-pair polynucleotide construct comprising a mate pair of polynucleotide arms, a first adapter, and a second adapter are used to generate concatemers of the circular construct. These concatemers are also referred to herein as "nucleic acid nanoballs," "DNA nanoballs," and "DNBs." Methods of generating DNBs are known in the art and are described, e.g., in US 8,445,194; US 8,592,150; US 9,023,769; and WO 2007/120208.

The concatemers comprise multiple copies, in tandem, of the mate-pair polynucleotide construct comprising the mate-pair polynucleotide arms, first adapter, and second adapter. In some embodiments, the concatemer comprises tens to hundreds of copies of the mate-pair polynucleotide construct, e.g., about 100 to about 500 copies, about 100 to about 400 copies, about 150 to about 400 copies, about 150 to about 300 copies, or about 150 to about 250 copies.

Concatemers of the mate-pair constructs may be produced by any of a variety of methods, including but not limited to, Rolling Circle Replication (RCR) and Circle Dependent Amplification (CDA). Methods of amplifying circular polynucleotide constructs, such as by RCR or CDA, are described in the art. *See, e.g.,* WO 2006/1199066; US 2008/0213771; US 8,445,194; and US 9,023,769.

### 8.1 Rolling Circle Replication

In some embodiments, RCR is used to generate concatemers of the mate-pair constructs as described herein. The RCR process relies upon the desired target polynucleotide being in a circular form. RCR uses the original circular polynucleotide, not copies of a copy, which ensures fidelity of sequence. Furthermore, as a circular entity, the circular mate-pair construct acts as an endless template for a strand-displacing polymerase that extends a primer complementary to a portion of the circle (e.g., in an adapter region). The continuous strand extension creates a long, single-stranded polynucleotide consisting of multiple (e.g., tens or hundreds) of concatemers comprising multiple copies of sequences complementary to the circular polynucleotide. The single-stranded polynucleotide comprising the concatemers can fold upon itself to form a three-dimensional ball (the DNB), which can subsequently be disposed on a surface for making DNB arrays.

Typically, RCR reaction components include a single-stranded circular polynucleotide template, one or more primers that anneal to the single-stranded circular polynucleotide, a DNA polymerase having strand displacement activity to extend the 3' ends of primers annealed to the circular polynucleotides, and nucleotides. In some embodiments, the DNA polymerase is the bacteriophage phi29 DNA polymerase. The RCR reaction components are combined under conditions that permit primers to anneal to the circular polynucleotide template (e.g., in a region within the first adapter) and to be extended by the DNA polymerase to form concatemers of sequences complementary to the circular polynucleotide. In some embodiments, the RCR reaction is allowed to continue until depletion of the reaction components. In some embodiments, the RCR reaction is terminated after a certain timepoint (e.g., after about 10 minutes, about 20 minutes, about 30 minutes, about 40 minutes, about 50 minutes, or about 1 hour). Guidance regarding conditions and reagents for RCR reactions is available, e.g., in US 5,854,033; US 6,143,495; and 8,722,326.

In some embodiments, the concatemers produced by RCR are approximately uniform in size; accordingly, in some embodiments, methods of the invention may include a step of size-selecting concatemers. For example, in some embodiments, concatemers are selected that as a population have a coefficient of variation in molecular weight of less than about 30%; and in another embodiment, less than 20%. In some embodiments, size uniformity is further improved by adding low concentrations of chain terminators, such ddNTPs, to the RCR reaction mixture to reduce the presence of very large concatemers, e.g., produced by DNA circles that are synthesized at a higher rate by polymerases. In some embodiments, concentrations of ddNTPs are used that result in an expected concatemer size in the range of from 50-250 Kb, or in the range of from 50-100 Kb. In another aspect, concatemers may be enriched for a particular size range using conventional separation techniques, e.g., size-exclusion chromatography, membrane filtration, or the like. See, e.g., US 2012/0004126.

### 8.2 Controlling Orientation of the Adapters in the Circular Mate-Pair Construct

In some embodiments, only a subset of circular mate-pair constructs, having a single orientation of the first adapter relative to the second adapter, is concatemerized. The control of adapter orientation relative to each other can be advantageous, for example, for maximizing the amount of signal that can be detected, such as when an anchor that is specific for the first adapter is used in a sequencing reaction.

In some embodiments, for selecting circular mate-pair constructs having a single orientation of the first adapter relative to the second adapter, a strand-specific RCR primer is used that is specific for one orientation of the first adapter in the circular mate-pair construct. This strand-specific primer hybridizes to one orientation of the first adapter, but does not hybridize to the other orientation (which is a reverse complement of the orientation being selected for). Accordingly, the RCR reaction only occurs for the circular mate-pair constructs in which the strand-specific RCR primer can bind.

In some embodiments, an "annealing-free" method for selecting circular mate-pair constructs having a single orientation of the first adapter relative to the second adapter is used. The annealing-free method uses a "pre-annealed" strand- and adapter-specific RCR primer, which is pre-annealed during the splint circularization/ligation step, and not right before the RCR reaction. Thus, the annealing-free method couples the steps of single-stranded DNA circularization and amplification through the use of a strand-specific amplification primer (e.g., a strand-specific RCR primer for replication by RCR) and a splint oligonucleotide having a 3' end blocked for extension by polymerase. Linear single-stranded polynucleotides (e.g., linear single-stranded polynucleotide constructs comprising a mate-pair of polynucleotide arms, a first adapter, and a second adapter) are circularized in the presence of both the 3' end-blocked splint and the strand-specific amplification primer using a suitable ligase (e.g., T4 DNA ligase) The ligation products are then treated with an exonuclease (e.g., Exonuclease I) to remove non-circularized linear strands and excess non-annealed splint oligonucleotides. Single-stranded DNA circles are then purified from the free oligonucleotides and nuclease(s) using magnetic beads. The RCR reaction components are then combined with the purified ligation products under conditions that permit a DNA polymerase to extend the pre-annealed strand-specific primer to form concatemers of sequences complementary to the circular polynucleotide.

### 9. Generation of Arrays

In one aspect, DNBs comprising concatemers of mate-pair constructs as described herein are disposed on a surface to form a random array of molecules. Polynucleotide molecules, including DNA concatemers such as DNBs, can be fixed to a substrate by a variety of techniques. Methods of generating arrays of DNBs are described, for example, in US 7,910,354; US 8,133,719; US 8,440,397; US 8,445,196; US 8,772,326; US 9,023,769; and in US 2013/01 78369.

In some embodiments, patterned substrates with two dimensional arrays of spots can be used to produce DNB arrays. The spots are activated to capture and hold the DNBs, while the DNBs do not remain in the areas between spots. In general, a DNB on a spot will repel other DNBs, resulting in one DNB per spot. Because DNBs are three-dimensional, arrays comprising DNBs result in more DNA copies per square nanometer of binding surface than traditional DNA arrays comprising short linear pieces of DNA This three-dimensional quality further reduces the quantity of sequencing reagents required, resulting in brighter spots and more efficient imaging. Occupancy of DNB arrays often exceeds 90%, but can range from 50% to 100% occupancy.

In some embodiments, the patterned surfaces are produced using standard silicon processing techniques. Such patterned arrays achieve a higher density of DNBs than unpatterned arrays, leading to fewer pixels per base read, faster processing, and increased efficiency in reagent use.

In some embodiments, a surface may have reactive functionalities that react with complementary functionalities on the polynucleotide molecules to form a covalent linkage. Long DNA molecules, e.g., several nucleotides or larger, may also be efficiently attached to hydrophobic surfaces, such as a clean glass surface that has a low concentration of various reactive functionalities, such as -OH groups. In some embodiments, polynucleotide molecules can be adsorbed to a surface through non-specific interactions with the surface, or through non-covalent interactions such as hydrogen bonding, van der Waals forces, and the like.

Attachment of the polynucleotides to the substrate may also include wash steps of varying stringencies to remove incompletely attached single molecules or other reagents present from earlier preparation steps whose presence is undesirable or that are nonspecifically bound to surface.

Upon attachment to a surface, single stranded polynucleotides generally fill a flattened spheroidal volume that on average is bounded by a region which is approximately equivalent to the diameter of a concatemer in random coil configuration. How compact a single stranded polynucleotide can be disposed on a surface can be affected by a number of factors, including the attachment chemistry used, the density of linkages between the polynucleotide and the surface, the nature of the surface, and the like. Preserving the compact form of the macromolecular structure of polynucleotides (including concatemers) on a surface can increase the signal to noise ratio; for example, a compact concatemer can result in a more intense signal from probes (e.g., fluorescently labeled oligonucleotides) that are specifically directed to components of the concatemer.

A wide range of densities of circular mate-pair constructs and/or DNBs can be arrayed on a surface. In some embodiments, each discrete region may comprise from about 1 to about 1000 molecules. In further embodiments, each discrete region may comprise from about 10 to about 900, about 20 to about 800, about 30 to about 700, about 40 to about 600, about 50 to about 500, about 60 to about 400, about 70 to about 300, about 80 to about 200, and about 90 to about 100 molecules. In some embodiments, arrays of circular mate-pair constructs and/or DNBs are provided in densities of at least 0.5, 1, 2, 3, 4, 5, 6, 7, 8, 9, or 10 million molecules per square millimeter.

### 10. Sequencing

In some embodiments, the mate-pair constructs as described herein, or the arrays comprising mate-pair constructs or concatemers thereof (e.g., DNBs) are used to identify a nucleotide sequence of one or more target polynucleotides. Techniques that can be used with the constructs and/or arrays described herein for identifying polynucleotide sequences of interest include, but are not limited to, techniques that rely on traditional hybridization methods to distinguish nucleotides at the detection position; extension techniques that add a base to basepair with the nucleotide at the detection position (e.g., sequencing by synthesis methods such as pyrosequencing); ligation techniques that rely on the specificity of ligase enzymes, such that ligation reactions occur preferentially if perfect complementarity exists at the detection position; and cleavage techniques that rely on enzymatic or chemical specificity such that cleavage occurs preferentially if perfect complementarity exists; and combinations thereof.

In some embodiments, a sequencing method as described herein is used to determine at least about 10 to about 200 bases in target nucleic acids, e.g., about 10, about 20, about 30, about 40, about 50, about 60, about 70, about 80, about 90, about 100, about 110, about 120, about 130, about 140, about 150, about 160, about 170, about 180, about 190, or about 200 bases in target nucleic acids. In some embodiments, a sequencing method described herein is used to identify at least 5, 10, 15, 20, 25, 30 or more bases adjacent to one or both ends of each adapter in a nucleic acid construct as described herein.

In some embodiments, the constructs and/or arrays described herein are used in conjunction with combinatorial probe-anchor ligation ("cPAL") sequencing techniques. In some embodiments, the constructs and/or arrays described herein are used in conjunction with sequencing by synthesis ("SBS") sequencing techniques. In some embodiments, the constructs, DNBs, and/or arrays described herein are used in conjunction with a combination of sequencing techniques, for example, with a combination of cPAL and SBS sequencing techniques that can be used on the constructs, DNBs, and/or arrays in a sequential manner.

### 10.1 cPAL Sequencing

In some embodiments, the constructs, libraries, or DNBs described herein are used in cPAL sequencing methods. cPAL sequencing involves identifying a nucleotide at a particular detection position in a target nucleic acid by detecting a probe ligation product formed by ligation of at least one anchor probe that hybridizes to all or part of an adapter and a sequencing probe that contains a particular nucleotide at an "interrogation position" that corresponds to (e.g. will hybridize to) the detection position. A "sequencing probe," as used herein, refers to an oligonucleotide that is designed to provide the identity of a nucleotide at a particular detection position of a target nucleic acid. Sequencing probes will generally comprise a number of degenerate bases and a specific nucleotide at a specific location within the probe to query the interrogation position. The sequencing probe contains a unique identifying label. If the nucleotide at the interrogation position is complementary to the nucleotide at the detection position, ligation can occur, resulting in a ligation product containing the unique label which is then detected. In any given cycle, the sequencing probes used are designed such that the identity of one or more of bases at one or more positions is correlated with the identity of the label attached to that sequencing probe. Once the ligated sequencing probe (and hence the base(s) at the interrogation position(s)) is detected, the ligated complex is stripped off of the construct or DNB, and a new cycle of adapter and sequencing probe hybridization and ligation is conducted. Multiple cycles of cPAL will identify multiple bases in the regions of the target nucleic acid adjacent to the adapters.

Additionally, sequencing reactions can be done at one or both of the termini of each adapter, e.g., the sequencing reactions can be "unidirectional" with detection occurring 3' or 5' of the adapter or the other, or the reactions can be "bidirectional" in which bases are detected at detection positions 3' and 5' of the adapter. Bidirectional sequencing reactions can occur simultaneously--i.e., bases on both sides of the adapter are detected at the same time--or sequentially in any order.

cPAL sequencing methods have many of the advantages of sequencing by hybridization methods known in the art, including DNA array parallelism, independent and non-iterative base reading, and the capacity to read multiple bases per reaction. Additionally, cPAL resolves two limitations of sequencing by hybridization methods, specifically, the inability to read simple repeats and the need for intensive computation. In some embodiments, the cPAL sequencing method comprises the use of one, two, three or more anchor probes in every hybridization-ligation cycle. In some embodiments, the cPAL sequencing method comprises the use of at least two ligated anchor probes in every hybridization-ligation cycle. In some embodiments, the first anchor probe hybridizes to a first anchor site in an adapter and the second anchor probe hybridizes to a second anchor site. In some embodiments, one anchor probe is fully complementary to an adaptor and the second anchor probe is fully degenerate, and thus able to hybridize to the unknown nucleotides of the region of the target nucleic acid that is adjacent to the adapter. In some embodiments, the second, fully degenerate, anchor probe is from about 5 to about 20 bases in length (e.g., about 5 to about 10 bases in length).Upon ligation to the first anchor probe, the formation of the longer ligated anchor probe construct provides the stability needed for subsequent steps of the cPAL process.

A detailed description of different exemplary embodiments of cPAL methods, as well as reagents and conditions for carrying out sequencing by cPAL, is provided in US 6,309,824; US 6,401,267; US 6,864,052; US 7,906,285; US 7,910,304; US 7,910,354; US 7,960,104; US 8,105,771; US 8,278,039; US 8,415,099; US 8,445,194; US 8,445,197; US 9,023,769; US 2008/0213771; US 2009/0264299; US 2012/0135893; and U.S. Patent Application Ser. Nos. 60/992,485; 61/026,337; 61/035,914 61/061,134; and 61/102,586.

### 10.2 SBS Sequencing

In some embodiments, the constructs, libraries, or DNBs described herein are used in sequencing by synthesis (SBS) methods. Sequencing by synthesis reactions can be performed on DNB arrays, which provide a high density of sequencing targets as well as multiple copies of monomeric units.

Any method of SBS sequencing can be used. Examples of SBS sequencing include, but are not limited to, pyrosequencing, sequencing by primer extension, and single molecule real time (SMRT) sequencing. SBS methods are described, for example, in US 6,210,891; US 6,828,100; US 6,833,246; US 6,911,345; US 7,858,311; US 8,399,188; and US 9,017,973.

### 10.3 Sequencing with Both cPAL and SBS Chemistries

In some embodiments, the constructs, libraries, or DNBs described herein are used in a combination of sequencing methods. For example, in some embodiments, the constructs and libraries described herein are sequenced using both cPAL chemistry and SBS chemistry in a sequential manner (e.g., first by cPAL chemistry, followed by SBS chemistry). In some embodiments, the first adapter and second adapter comprise hybridization sequences (e.g., anchor or intruder hybridization sequences) for sequencing by cPAL chemistry in the 3' to 5' direction and further comprise hybridization sequences (e.g., SBS sequencing primer hybridization sequences) for sequencing by SBS chemistry in the 5' to 3' direction.

For libraries comprising two adapters, the use of both cPAL and SBS chemistries in a sequential manner for sequencing will result in two reads per mate-pair polynucleotide "arm", for a total of four reads per construct or DNB. Thus, the use of multiple sequencing methods on a construct, library, or DNB as described herein can generate more information out of each construct, library, or DNB that is sequenced.

### 11. Kits

In another aspect, kits for practicing the library construction methods described herein are provided.

Described is a kit that comprises a first oligonucleotide and a second oligonucleotide for an adapter as described herein. A kit may comprise a first oligonucleotide and a second oligonucleotide for a bubble adapter. A kit may comprise a first oligonucleotide and a second oligonucleotide for an L-oligo adapter, and optionally further comprises helper oligonucleotides for the L-oligo adapter. A kit may comprise a first oligonucleotide and a second oligonucleotide for a clamp adapter, and optionally further comprises helper oligonucleotides for the clamp adapter.

A kit may comprise oligonucleotides for two or more adapters (e.g., oligonucleotides for a first adapter and oligonucleotides for a second adapter) as described herein. A kit may further comprise a first oligonucleotide and a second oligonucleotide for a first bubble adapter, and further comprises a first oligonucleotide and a second oligonucleotide for a second bubble adapter. A kit may comprise a first oligonucleotide and a second oligonucleotide for a first L-oligo adapter, further comprises a first oligonucleotide and a second oligonucleotide for a second L-oligo adapter, and optionally further comprises helper oligonucleotides for the L-oligo adapter. A kit may comprise a first oligonucleotide and a second oligonucleotide for a first clamp adapter, further comprises a first oligonucleotide and a second oligonucleotide for a second clamp adapter, and optionally further comprises helper oligonucleotides for the clamp adapter. A kit may comprise a first oligonucleotide and a second oligonucleotide for a bubble adapter, further comprises a first oligonucleotide and a second oligonucleotide for a clamp adapter, and optionally further comprises helper oligonucleotides for the clamp adapter.

The kit may further comprise one or more additional components related to features of the adapters as described herein. The kit may further comprise one or more enzymes for carrying out a method described herein (e.g., an enzyme for use in a ligation, amplification, or DNA synthesis reaction as described herein), and optionally may comprise other reagents for performing an enzymatic reaction as described herein (e.g., buffers, nucleotides, etc.). The kit may further comprise one or more primers for carrying out a method described herein (e.g., one or more amplification primers for carrying out an amplification method described herein). The kit may further comprise a splint oligonucleotide. The kit may further comprise one or more reagents for a sequencing method as described herein (e.g., one or more reagents for cPAL and/or SBS sequencing).

A kit may comprise components (e.g., adapter oligonucleotides, enzymes, or enzymes pre-mixed with reaction components) for performing a block of reactions as described herein. Exemplary blocks of reactions are shown in Figure 2. A kit may comprise components for preparing polynucleotide fragments for ligation and/or ligating a first adapter to polynucleotide fragments (e.g., components for modifying polynucleotide fragments and ligating a first adapter, components for modifying polynucleotide fragments, ligating a first adapter, and amplifying the ligation product by PCR; components for fragmenting DNA, modifying polynucleotide fragments, and ligating a first adapter; or components for fragmenting DNA, modifying polynucleotide fragments, ligating a first adapter, and amplifying the ligation product by PCR). A kit may comprise components for forming open double-stranded circular polynucleotide constructs (e.g., components for creating gaps at uracil sites, circularization, and purification). A kit may comprise components for ligating a first adapter and for forming open double-stranded circular polynucleotide constructs (e.g., components for ligating a first adapter, amplifying the ligation product by PCR, creating gaps at uracil sites, circularization, and purification). A kit may comprise components for generating mate-pair polynucleotide arms (e.g., components for performing time and temperature controlled nick translation (TTCNT), components for performing time and temperature controlled extension (TTCE), or components for performing reversible terminator controlled extension (RTCE), such as polymerases, exonucleases, and nucleases; components for TTCNT, TTCE, or RTCE, and components for end-repair of TTCNT, TTCE, or RTCE products, such as polymerases and phosphatases). A kit may comprise components for ligating a second adapter (e.g., components for ligating a first adapter and amplifying the ligation product by PCR). A kit may comprise components for circularizing the mate-pair polynucleotide constructs (e.g., components for denaturing amplification products and circularizing single-stranded polynucleotide constructs). A kit may comprise components for ligating a second adapter and circularizing the mate-pair polynucleotide constructs (e.g., components for ligating a first adapter, amplifying the ligation product by PCR, denaturing amplification products, and circularizing single-stranded polynucleotide constructs). A kit may comprise components for making, loading, and/or pooling DNA nanoballs.

### 12. Examples

The following examples are offered to illustrate, but not to limit the claimed invention.

### Example 1: Construction of Mate-Pair Library Comprising Two Bubble Adapters

Figure 19 depicts a schematic of how a mate-pair library comprising two bubble adapters was constructed.

3 ug of input DNA was fragmented using Covaris to produce 200-1800 bp fragments. The fragmented DNA was then size-selected using magnetic beads to retain 300-1000 bp fragments, with an average size of 650 bp fragments. 500 ng or 1.2 pmol of size-selected DNA was taken forward into the library preparation. End repair was carried out to yield 5' phosphorylated blunt-end fragments using T4 PNK and T4 DNA polymerase enzymes, then a dA tail was added to the fragments. The first bubble adapter Ad203 was ligated to the DNA fragments by A-T ligation. The ligation product was amplified by PCR using uracil-containing primers and PfuCx polymerase, which tolerates the presence of uracils in the template. The amplification product was treated with USER enzyme (Uracil-Specific Excision Reagent Enzyme, a mixture of Uracil DNA glycosylase (UDG) and the DNA glycosylase-lyase Endonuclease VIII) to generate "sticky"-ends with 14-nt overlap, followed by treatment with Plasmid-Safe™ ATP-Dependent DNase ("PS") to allow formation of stable open-dsDNA-circles containing 2-nt gaps. Time and temperature controlled nick translation ("TT-CNT") was carried out on the open-dsDNA circles using Taq polymerase, followed by T7 exonuclease treatment and nuclease treatment. The double-stranded construct was then end-repaired and A-tailed. The second bubble adapter Ad195 was then ligated to the double-stranded construct by A-T ligation and amplified with Q5 polymerase to produce blunt-ended PCR products; one of the primers was 5'-phosphorylated to allow ssDNA circle formation from 2 of the 4 different DNA strands produced by the amplification reaction. The amplification products were then heat denatured into single-stranded DNA constructs. ssDNA circles were formed by ligation with T4 ligase in the presence of a splint oligonucleotide, followed by exonuclease treatment to remove non-circularized linear strands, splint oligonucleotide annealed to the circles, and excess free splint oligonucleotides. DNA nanoballs (DNBs) were then formed from a specific strand of ssDNA circle using a strand-specific RCR primer that is specific for one orientation of the first adapter in the ssDNA circle.

### Example 2: Construction of Mate-Pair Library Comprising Two L-Oligo Adapters

Figure 22 depicts a schematic of a mate-pair library that was constructed comprising two L-oligo adapters.

3 ug of input DNA was fragmented using Covaris to produce 200-1800 bp fragments. The fragmented DNA was then size-selected using magnetic beads to retain 300-1000 bp fragments, with an average size of 650 bp fragments. 500 ng or 1.2 pmol of size-selected DNA was taken forward into the library preparation. End repair was carried out on the fragmented DNA using shrimp alkaline phosphatase and T4 DNA polymerase to yield dephosphorylated blunt-end fragments. The first L-oligo adapter Ad169 was ligated to the DNA fragments in two steps. For the first step, the second oligonucleotide was ligated by blunt-end ligation in the presence of a short helper oligonucleotide with a 3'-end modification. A "heat-kill" step was used to inactivate the ligase and remove the helper oligonucleotide, then a phosphate group was added to the 5'-ends of the DNA fragments using T4 PNK. For the second ligation step, the first oligonucleotide, which has a 3' region of homology to the second oligonucleotide already ligated to the DNA fragment, was annealed and ligated to create symmetrical Y-like structures flanking the DNA fragment. The ligation product was amplified by PCR using uracil-containing primers and PfuCx polymerase, which tolerates the presence of uracils in the template. The amplification product is treated with USER enzyme to generate "sticky"-ends with 14-nt overlap, followed by treatment with Plasmid-Safe™ ATP-Dependent DNase ("PS") to allow formation of stable open-dsDNA-circles containing 2-nt gaps. Time and temperature controlled nick translation ("TT-CNT") was carried out on the open-dsDNA circles using Taq polymerase, followed by T7 exonuclease treatment and nuclease treatment. The double-stranded construct was then end-repaired to generate dephosphorylated blunt ends. The second L-oligo adapter Ad165 was ligated to the double-stranded construct using the same two-step ligation method as was used for ligating the first adapter. The ligation product was amplified with Q5 polymerase to produce blunt-ended PCR products; one of the primers was 5'-phosphorylated to allow ssDNA circle formation from 2 of the 4 different DNA strands produced by the amplification reaction. The amplification products were then heat denatured into single-stranded DNA constructs. ssDNA circles were formed by ligation with T4 ligase in the presence of a splint oligonucleotide, followed by exonuclease treatment to remove non-circularized linear strands, splint oligonucleotide annealed to the circles, and excess free splint oligonucleotides. DNBs were formed from a specific strand of ssDNA circle using a strand-specific RCR primer that is specific for one orientation of the first adapter in the ssDNA circle.

### Example 3: Construction of Mate-Pair Library Comprising Bubble and Clamp Adapters

Figure 23 depicts a schematic of a mate-pair library that was constructed comprising a bubble adapter as the first adapter and a clamp adapter as the second adapter.

3 µg of input DNA was fragmented using Covaris to produce 200-1800 bp fragments. The fragmented DNA was then size-selected using magnetic beads to retain 300-1000 bp fragments, with an average size of 650 bp fragments. 500 ng or 1.2 pmol of size-selected DNA was taken forward into the library preparation. End repair was carried out to yield 5' phosphorylated blunt-end fragments using T4 PNK and T4 DNA polymerase enzymes, then a dA tail was added to the fragments. The first adapter, a bubble adapter Ad201, was ligated to the DNA fragments by A-T ligation. The ligation product was amplified by PCR using uracil-containing primers and PfuCx polymerase, which tolerates the presence of uracils in the template. The amplification product was treated with USER enzyme (Uracil-Specific Excision Reagent Enzyme, a mixture of Uracil DNA glycosylase (UDG) and the DNA glycosylase-lyase Endonuclease VIII) to generate "sticky"-ends with 14-nt overlap, followed by treatment with Plasmid-Safe™ ATP-Dependent DNase ("PS") to allow formation of stable open-dsDNA-circles containing 2-nt gaps. Time and temperature controlled nick translation ("TT-CNT") was carried out on the open-dsDNA circles using Taq polymerase, followed by T7 exonuclease treatment and nuclease treatment. The double-stranded construct was then heat denatured into single strands. The second adapter, a clamp adapter Ad191, comprising 5'-adapter and 3'-adapter parts, was ligated directly to the single-stranded construct using T4 DNA ligase. The ligation template at the ligation junction is represented by combinations of five random nucleotides [(N)₅] plus four universal inosine nucleotides [(I)₄]. The ligation product was amplified with Q5 polymerase to produce blunt-ended PCR products; one of the primers was 5'-phosphorylated to allow ssDNA circle formation from 2 of the 4 different DNA strands produced by the amplification reaction. The amplification products were then heat denatured into single-stranded DNA constructs. ssDNA circles were formed by ligation with T4 ligase in the presence of a splint oligonucleotide, followed by exonuclease treatment to remove non-circularized linear strands, splint oligonucleotide annealed to the circles, and excess free splint oligonucleotides. DNA nanoballs (DNBs) were formed from a specific strand of ssDNA circle using a strand-specific RCR primer that is specific for one orientation of the first adapter in the ssDNA circle.

### Example 4: Improved GC Coverage Using Two-Bubble Adapter System

The GC coverage obtained from mate-pair libraries comprising two bubble adapters was compared to GC coverage obtained from libraries constructed according to other methods (Figure 24). Batch 10000046 (blue line) used NA19238, NA19239, and NA19240 genomic DNA to construct genomic libraries, according to the method described in Example 1 above. Bubble-Adapter 162 was used as the first bubble adapter (Adapter A) and Bubble-Adapter 165 was used as the second bubble adapter (Adapter B). Batch 10000096 (green line) used NA19238, NA19239, NA19240, and NA12878 DNA to construct genomic libraries, according to the method described in Example 1 above. Bubble-Adapter 181 was used as the first bubble adapter (Adapter A) and Bubble-Adapter 194 was used as the second bubble adapter (Adapter B).

As shown in Figure 24, TT-CNT mate-pair libraries comprising two bubble adapters (Batch 10000046 and Batch 10000096) yielded more uniform coverage of the exome including both high AT-rich and high GC-rich sequences, compared to a current library production process (Denali; 26-nt arms are generated by EcoP15) and another method of generating mate-pair library arms. TT-CNT libraries exhibited significantly improved GC coverage across the exome, particularly in the GC-rich region.

### Example 5: Nick Translation Controlled by Nucleotide Amount (ntCNT)

We examined the effect of various dNTP::DNA molar ratios on ntCNT: 17, 8.6, and 5.7. The results are presented in the following table:

**Table 1: Effects of dNTPs::DNA on ntCNT**

| **dNTPs::DNA ratio** | **Temperature, Incubation time** | **Theoretical length/arm (bp) (if all dNTPs are incorporated)** | **Observed ntCNT shift on gel (bp/2 arms)** | **Calculated real shift/arm (bp)** |
|---|---|---|---|---|
| 17 | 10 °C, 20min | ∼68 | 85-160 | ∼60 ± 20 |
| 8.6 | 10 °C, 20min | ∼33 | 40-80 | ∼30 ± 10 |
| 5.7 | 10 °C, 20min | ∼23 | 30-60 | ∼20 ± 8 |

We also examined the effect of various temperatures on the ntCNT and of limited amounts of dNTPs on nick translation, specifically DNA translation distance, using Taq DNA Polymerase. The templates for ntCPE were first amplified with the 5' and 3' adaptor primers that flank a genomic region of 800 bp-3 kb. During ntCPE reactions, the PCR products were first denatured at 96°C, annealed with 5' primers at 56°C and then extended with Taq and titrated amounts of dNTPs at 72°C for 10 minutes. Following ntCPE reactions, ExoVII treatment was used to degrade any single stranded DNA generated from ntCPE, as well as the other PCR strand that couldn't be used as ntCPE templates. Gel in Fig3 demonstrates that the extent of ntCPE with different dNTP amount. The primer extended products from different ntCPE reactions migrated differently and the migration was dependent on the different dNTP titrations. In lane 6, excess dNTPs were added at the polymerization step as a control, which resulted in normal one PCR cycle products with the original PCR size range. Reactions performed with the least dNTPs amounts in lane 5 generated the smallest end-point products. When the dNTPs: :DNA increased (lane 1-5), Taq mediated polymerization can elongate longer. We also tested the relationship between dNTPs amount and other polymerases, such as PfuCx and Pol I. PfuCx probably has the highest Km so that to the same extent of CPE it requires the highest dNTP amount in the reactions. The results showed that we can also combine ntCNT and TTCNT to control the nick translation speed. The results are provided in the following table:

**Table 2: Combined Effects of ntCNT and ttCNT**

| **dNTPs::DNA ratio** | **ntCNT reaction** | **Theoretical length/arm (bp)** | **ntCNT shift on gel (bp/2 arms)** | **Calculated real shift/arm (bp)** |
|---|---|---|---|---|
| 33.5 | 72 °C, 30min | ∼134 bp | 110-185 | ∼75 ± 20 |
| 33.5 | 37 °C, 30min | ∼134 bp | 45-110 | ∼40 ± 20 |
| 335 | 37 °C, 30min | ∼1340 bp | 160-430 | ∼295 ± 70 |
| 335 | 30 °C, 30min | ∼1340 bp | 60-310 | ∼90 ± 60 |

### Example 6: 3' Branch Ligation

After ntCNT, 3' branch ligation is performed to add a 3' arm of the second adapter (AdB_3').

It is well known that nicks in double stranded DNA fragments and double stranded DNA fragments with sticky or blunt ends can be joined at 5' phosphate and 3' hydroxyl groups The ligation of sticky ends or nicks is generally faster and less dependent on enzyme concentration than blunt end ligation. Both processes can be catalyzed by bacteriophage T4 DNA ligase T4 ligase is reported to mediate certain unconventional ligations: it seals dsDNA substrates containing an abasic site or a gap at the ligation junction, joins branched DNA strands, and forms a stem-loop product with partially double stranded DNA (Nilsson and Magnusson, Nucleic Acids Res 10:1425-1437, 1982; Goffin et al., Nucleic Acids Res 15:8755-8771, 1987; Mendel-Hartvig et al., Nucleic Acids Res. 32:e2, 2004; Western and Rose, Nucleic Acids Res., 19:809-813, 1991). We have discovered that T4 ligase can be used to join DNA fragments at dephosphylated nicks, gaps or 5' overhang regions to form an Okazaki fragment-like structure. As illustrated in Figure 20, the insert DNA can be a synthetic linker or adapter DNA consisting of double-stranded DNA with one blunt end and one 3' overhang. Both 3' termini of the adaptors are dideoxynucleotides, which prevents self-ligation of the adapter. The 5' terminus of the long adaptor strand is phosphorylated and ligates to the 3' terminus of the substrate DNA at the gap.

The substrate DNA molecule contains one of the following structures: (1) a nick or (2) gap with a 3'-hydroxyl terminus (i.e., one or more missing nucleotide bases), or (3) a 5' overhang (5'-OH) (that is, 3' branch ligation encompasses nick ligation, gap ligation, and 5' overhang ligation).

By appropriately mixing two or three oligos, we constructed substrates with a nick, a 1-bp gap, an 8-bp gap, and a 5' overhang of 36 bp (Figure 20). The substrates are not phosphorylated and the long strand of the adaptor has a 3' dideoxynucleotide to prevent ligation. T4 ligase joins the 5'-phospharylated adaptor strand to the 3'-hydroxylated substrate DNA strand to form a branched DNA structure. Therefore, we name this novel ligation event a "3' branch ligation."

We examined numerous factors that affect general ligation efficiency including: adaptor: :DNA ratio, the amount of T4 ligase, final ATP concentration, Mg²⁺ concentration, pH, incubation time and various additives. Adding polyethylene glycol (PEG) to a final concentration of 10% substantially increased the ligation efficiency from less than 10% to more than 80%. A variety of ATP concentrations (from 1uM to 1mM) and Mg²⁺ concentration (3mM to 10mM) worked fairly well with the 3' branch ligation. For our optimized conditions, the adaptor: :DNA molar ratio is about 50, and the reactions were performed at pH 7.8 with 10% PEG and 10uM ATP at 37°C for an hour. In a volume of 30 µl, 0.5 pmol of different substrates (1-4) were individually ligated to 25 pmol of adaptor DNA in the presence of 600 units of T4 ligase. A positive control of blunt end ligation and the negative controls of self-ligation of the substrates were also included. To assay for ligation yields, the ligation products were electrophoresed in a 6% polyacrylamide gel. The size shift ratio indicated the efficiency of 3' branch ligation. The data suggested the efficient ligation of 8 bp gap and 5'-OH DNA. The 5'-OH ligation appeared to have been almost 100% complete, even higher than for blunt end ligations. The 1 bp gapped substrates had a ligation efficiency of about 50%. However, the nick ligation efficiency was the lowest, less than 10%, even under optimized conditions.

We also extended our study to different adaptor substrate sequences. Some adaptor sequences resulted in more efficiently ligated products than others on the four substrates mentioned above. However, if the substrate sequences changed, the performance of the adaptors also changed. This is probably due to the nucleotide preferences of T4 ligase. Despite the adaptor sequences, an 8 bp gap and 5'-OH ligations always had the highest ligation efficiency, while a 1 bp gap ligation worked, but not as well as the longer gap or 5'-OH, and the nick ligation worked poorly. This supports our hypothesis (illustrated in Figure 20) that the DNA bends at the point where the nick/gap/OH starts and exposes a 3' hydroxyl group for ligation. The longer ssDNA region makes the 3' termini more accessible in the ligation and therefore results in higher ligation efficiency.

Practically speaking, if the ntCNT reaction uses a DNA polymerase that has 3' exonuclease (exo) activity such as DNA Polymerase I, a 5' arm of a second adapter (AdB) can be added by ligation directly to the 3' end of the resulting gap region. If the CNT reaction uses a DNA polymerase that lacks 3' exo activity (or if ttCNT is used), a less processive exonuclease, e.g., T7 exo or Bst polymerase (Bst polymerase has exonucleoase activity; for this purpose, we use it in the absence of dNTPs), can be used to remove a few nucleotides from the 5' end of the nicks and create a gap region for AdB 3' gap ligation for more effective 3' branch ligation.

The effect of other additives, such as SSB (Single Strand Binding) proteins, was also assayed on those substrates. We titrated the final concentration of ET SSB (New England Biolabs, Ipswich, MA) from 2 ng/µl to 20 ng/µl and discovered that a higher concentration, 10 or 20 ng/µl, of ET SSB can further increase the ligation efficiency for an 8 bp gap and 5'-OH DNA, but has no effect on nicked or 1 bp gapped DNA. It appears that SSB proteins bind to the single stranded region and stabilize ssDNA.

### Example 7: Library Construction using ntCNT, 3' Branch Ligation, and CPE

According to one embodiment of the invention, a method for mate pair library construction is provided as shown in Figure 21. After adding a first adapter (AdA) (e.g., a bubble adapter, L-oligo adapter, clamp adapter, etc.) to genomic DNA and forming a double stranded circle (dsCir) with a nick or a gap, optionally followed by a gapping step to create a gap of several base pairs, CNT moves the nick or gap with a selected length into the genomic DNA. 3' branch ligation is used to ligate a 5' arm of the second adapter at the resulting nick or gap. The two strands of dsCir DNA resulting from 3' branch ligation are optionally separated, and a single stranded DNA (ssDNA) strand is generated that includes an AdA sequence surrounded by genomic DNA (specifically, the ends of a starting genomic DNA fragment) and AdB_5' sequence at the 3' end of the genomic DNA. This ssDNA strand is used as template in a CPE reaction, resulting in a construct with a mate pair derived from the starting genomic DNA fragment. Each arm of the mate pair has a selected length (resulting from the CNT and CPE reactions, respectively), separated by AdA sequence, with AdB_5' sequences at one end of the construct. An AdB_3' sequence (Ad141_3') is then added to the other end of the construct by 3' branch ligation (in this case, a 5' overhang ligation), resulting in an amplifiable template with AdB primers at each end.

Controlled nick translation. One method for performing CNT is controlled nick translation by nucleotide amount (ntCNT), in which a limited amount of one or more nucleotides is used to control the distance that a nick is translated into the genomic sequence, or the length of nick translation. The DNA polymerase stops, either when it runs out of the limiting nucleotide(s) (e.g., polymerases with low dNTP Kₘ such as E. coli DNA Pol I), or when the availability of dNTPs becomes too low to form an enzyme/substrate complex (e.g., high Kₘ DNA polymerases such as Taq DNA polymerase or PfuCx DNA polymerase. This form of CNT is useful for creating mate pair libraries with sequences from the end of a starting DNA fragment of any selected length, permitting sequence reads of 100-150 bp, for example. ntCNT has all the advantages of controlled nick translation: short incubation time, long mate pair read length, and high efficiency. Additionally, ntCNT is not sensitive to temperature or incubation time, resulting in a controllable and easily repeated process with a tight range of read lengths (or mate-pair arm length). The size and range of read lengths are dependent on the selected polymerase type and the corresponding ratio of dNTPs to DNA. Generally, the more dNTPs are used in the reaction, the longer the read length (and the broader the range of read lengths) that results.

Controlled nick translation by nucleotide amount (ntCNT) was carried out in a reaction containing 1.5 pmol Ad142 double stranded circular DNA (300-1000 bp), 6 µl of 10X NEBuffer 2 (New England Biolabs, Ipswich, MA), 5.5 µl of 0.0045 mM dNTPs with 2xAT, 1 µl of 0.91 U/µl DNA Polymerase I (New England Biolabs, Ipswich, MA) and water in a total reaction volume of 60 µl. The reaction mixture was set up on the ice then placed in a thermocycler running at 37 °C for 15 minutes and heat denatured at 65 °C for 15 minutes. Heat lid tracking was set at 5 °C above.

3' branch ligation (gap ligation). 3' branch ligation was performed by mixing 12 µl of 20 µM Adl41_5' adapter (YJ-364 Ad041_5T_04,5'-/5phos/AAGTCGGAGGCCAAGCGGTCGT/3ddC/-3', YJ-365 ON4248 Ad141_5, 5'- TTGGCCTCCGACT/3dT-Q/- 3'), 40 µl of 3x HB buffer (0.05mg/ml BSA, 50mM Tris-Cl pH7.8, 10mM MgCl2, 0.5mM DTT, 1mM ATP, 10% PEG-8000), 3 µl of 600U/µl T4 DNA Ligase (New England Biolabs, Ipswich, MA), 60 µl of CNT product, 2.4 µl of 0.5 µg/µl ET SSB (New England Biolabs, Ipswich, MA) and water in a 120 µl reaction volume. The reaction was then incubated at 37 °C for 1 hour and heat denatured at 65 °C for 15 minutes in a thermocycler, and set heat lid tracking at 5 °C above.

1.5x Axygen beads (Corning, Corning, NY) are used to purify the ligation product following the Axygen beads purification protocol. Then elute in 30 µl pH 8.0 Tris-EDTA (TE) buffer.

Controlled Primer Extension. Controlled primer extension was carried out in a reaction containing 9 ul of 10X ThermoPol buffer(New England Biolabs, Ipswich, MA), 0.5 ul of 0.096 mM dNTPs, 18 ul of 20 uM ON0639 (5'-/52Bio/TCCTAAGACCGCTTGGCCTCCGACT-3'), 30 ul of gap ligation product, 1.5 ul of 5 U/µl Taq and water in a total reaction volume of 90 µl. The reaction mixture is set up on the ice and kept fresh, and then placed in a thermocycler running the program: [96 °C 5 min, 56 °C 1 min, 72 °C 5 min, 4 °C hold]. The reaction was stopped by adding 1.2 µl 0.5 M EDTA.

1.5x Axygen beads (Corning, Corning, NY) are used to purify CPE product which follow Axygen beads purification protocol. Then elute in 40 ul pH 8.0 TE buffer.

3' branch ligation (overhang ligation). Overhang ligation (OH) was performed by mixing 16 ul of 20 uM Ad141_3' adapter (ON3664,5'-/5Phos/GTCTCCAGTCGAAGCCCGACG/3ddC/-3', ON3665, 5'-GCTTCGACTGGAGA/3ddC/-3'), 40 ul of 3x HB buffer, 4 ul of 600 U/µl T4 DNA Ligase (New England Biolabs, Ipswich, MA), 40 ul of CPE product, 2.4 ul of 0.5 ug/µl ET SSB (New England Biolabs, Ipswich, MA) and water in a 120 ul reaction volume. The reaction was then incubated at 37C for 1 hour and heat denatured at 65 °C for 15 minutes in a thermocycler, and set heat lid tracking at 5 °C above.

1.0x Axygen beads (Corning, Corning, NY) were used to purify ligation product which follow Axygen beads purification protocol. Then elute in 90 ul pH 8.0 TE buffer.

AdB PCR. The total purified OH ligation product was PCR amplified using Q5 high-fidelity DNA polymerase (New England Biolabs, Ipswich, MA) in a 240 ul reaction volume with Q5® High GC Enhancer (New England Biolabs, Ipswich, MA). PCR enrichment was carried out by using the program: [98 °C, 30 s (98 °C, 10 s; 65 °C, 30 s; 72 °C, 30 s) 7 cycles, 72 °C 2 min, slow down to 4 °C at 0.1 °C/sec] using the following primers: 5'-/52Bio/TCCTAAGACCGCTTGGCCTCCGACT-3' and 5'-/5phos/AGACAAGCTCGAGCTCGAGCGATCGGGCTTCGACTGGAGAC-3'.

0.8x Axygen beads (Corning, Corning, NY) were used to purify the PCR product which follow Axygen beads purification protocol. The DNA was eluted from the beads in 55 ul pH 8.0 TE buffer. The DNA was then quantified using a dsDNA High-Sensitivity kit (Invitrogen, Waltham, MA) following the manufacturer's instructions.

PCR and PAGE Analysis. In order to assess the quality of the ntCNT and ntCPE arms, the product of gap ligation and OH ligation (1 ul) was amplified using PfuCx DNA polymerase (Agilent Technologies, Santa Clara, CA). Primer sequences were: Cir Control (5'-GTCGAGAACGUCTCGTGCT-3' and 5'-ACGTTCTCGACUCAGCAGA-3'), CNT arm (5'-/52Bio/TCCTAAGACCGCTTGGCCTCCGACT-3' and 5'-ACGTTCTCGACUCAGCAGA-3'), CPE arm (5'-GTCGAGAACGUCTCGTGCT-3' and 5'-/5phos/AGACAAGCTCGAGCTCGAGCGATCGGGCTTCGACTGGAGAC-3'), and Final product (5'-/52Bio/TCCTAAGACCGCTTGGCCTCCGACT-3' and 5'-/5phos/AGACAAGCTCGAGCTCGAGCGATCGGGCTTCGACTGGAGAC-3').

The samples were analyzed on precast 6% TBE polyacrylamide gels (Bio-Rad, Hercules, CA). 5 ul of PCR product was mixed with 2 ul of 6x loading buffer. The sample was then loaded into the gel and run for 10-15 min at 250V. The separated gels were dyed by GelStar and scanned using gel imaging system to get the gel picture which to determine band size and intensity.

### Making ssCir for Rolling Circle Replication to Make DNA Nanoballs.

1. Splint oligo annealing. The AdB PCR product was normalized in 65 ul. 5 ul of 20 uM ON1587 splint oligo (5'- TCGAGCTTGTCTTCCTAAGACCGC-3') was added to each reaction. The reaction was then heat denatured at 95 °C for 3 minutes with heated lid at 105 °C in a thermocycler and immediately snap cooled on ice for 10 minutes.
2. ssDNA splint circularization. Subsequently, 50µl of the following reaction mixture was added, mixed fully by vortex and incubated at 37 °C for 1 hour: 36.4 µl H2O, 12 µl 10X TA buffer (Epicentre, Madison, WI), 1.2 µl 100 mM ATP , 0.4 µl T4 DNA ligase (Enzymatics, Beverly, MA; 120 µl total reaction volume).
3. Exo I and Exo III Tx. The product of the circularization reaction was removed (4 ul). Linear DNA was removed by addition of 8 µl of the following reaction mixture to the circularization product: 0.8 µl 10X TA buffer (Epicentre, Madison, WI), 3.9µl 20 U/ul ExoI (New England Biolabs, Ipswich, MA), 2.0µl H2O, 1.3 µl 100 U/ul ExoIII (New England Biolabs, Ipswich, MA) (total reaction volume 124 µl). The reaction mixture was set up at room temperature and placed in a thermocycler running at 37 °C for 30 min. The reaction was stopped by adding 6 ul 0.5 M EDTA.
4. Purification. Single strand circle DNA (ssCir DNA) was purified by 170 µl PEG32 beads (AMPure XP beads [Beckman Coulter, Inc., Beverley, MA] in 32% PEG 3350 1.6M NaCl, 20mM EDTA 0.09% sodium Azide 0.01% Tween-20), then eluted in 55 ul pH 8.0 TE buffer.
5. Quantitation. 2 µl of the purified ssCir DNA was quantified by ssDNA Oligreen Kit (Invitrogen, Waltham, MA).

### INFORMAL SEQUENCE LISTING

SEQ ID NO:1 – Bubble adapter A Ad203
AACTGCTGACGTACTGATGGGCATGGCGACCTATTCAGBBBBBBBTCTCGACTC
AGCAGTT

SEQ ID NO:2 – Bubble adapter A Ad201

AACTGCTGACGTACTGATGGGCATGGCGACCTATTCAGBBBBBBBAACGATCAC
TCCTCTCGACTCAGCAGTT

SEQ ID NO:3 – Bubble adapter A Ad162

AACTGCTGACGTACTGATGGGCATGGCGACCTATTCAGBBBBBBBBBTCTCGA
CTCAGCAGTT

SEQ ID NO:4 – Bubble adapter A Ad181

AACTGCTGACGTACTGATGGGCATGGCGACCTATTCAGBBBBBBBBBCGATCA
CTCCTCTCCAGCTCAGCAGTT

SEQ ID NO:5 – Bubble adapter B Ad195

AAGTCGGAGGCCAAGCGTGCTTAGGACATGTAGCGTCG(N)6BBBBBBAACGA
GTGATGCGTGTACGATCCGACTT

SEQ ID NO:6 – Bubble adapter B Ad194

AAGTCGGAGGCCAAGCGTGACTTAGGACATGTAGCGACCT(N)6BBBBBBBAAC
GAGTGATGCGTGTACGATCCGACTT

SEQ ID NO:7 - Bubble adapter B Ad165-Bubble
   AAGTCGGAGGCCAAGCGTGCTTAGGACATGTAGTGTACGATCCGACTT 
SEQ ID NO:8 – L-oligo adapter A Ad169

ACTGCTGACGTACTGACTGTAGGGCTGGCGACCTTGACGANNNNNNNNNNTCC
TCAGCTCAGCAGT
SEQ ID NO:9 - L-oligo adapter B Ad165
   AAGTCGGAGGCCAAGCGTGCTTAGGACATGTAGTGTACGATCCGACTT 
SEQ ID NO:10 – Clamp adapter Ad191

AAGTCGGAGGCCAAGCGTGCTTAGGACATGTAGCG(N)6CTCTCTAAACGAGTG
ATGCGTGTACGATCCGACTT
SEQ ID NO:11 - Clamp adapter Ad212
   AAGTCGGAACCGTGGATGCTGAGTGATGGCTGTACGABBBBBBB

## Claims

1. A method of making a mate pair polynucleotide library comprising:
providing a plurality of double-stranded target polynucleotides and a first adapter selected from a bubble adapter, an L-oligo adapter or a clamp adapter, wherein the bubble adapter is formed from a first oligonucleotide and a second oligonucleotide partially complementary to each other at their 5' and 3' ends, such that the 5' end of the first oligonucleotide is complementary to the 3' end of the second oligonucleotide, and the 3' end of the first oligonucleotide is complementary to the 5' end of the second oligonucleotide, and the intervening sequence of each oligonucleotide is not substantially complementary to the other oligonucleotide, such that the middle regions of the oligonucleotides do not hybridize with each other, thus forming a "bubble",
the L-oligo adapter is formed from a first oligonucleotide and a second oligonucleotide partially complementary to each other, such that 3' end of the first oligonucleotide is complementary to the 5' end of the second oligonucleotide, and the remaining 5' sequence of the first oligonucleotide is not substantially complementary to the remaining 3' sequence of the second oligonucleotide, such that these regions do not hybridize with each other, thus the first oligonucleotide forms an L shape, and
the clamp adapter comprises a 3' clamp and a 5' clamp, wherein the 5' clamp comprises a first oligonucleotide and a first helper oligonucleotide, and the 3' clamp comprises a second oligonucleotide and a second helper oligonucleotide, the first helper oligonucleotide hybridizes to a portion of the first oligonucleotide, and the second helper oligonucleotide hybridizes to a portion of the second oligonucleotide to form the clamp adapter in order to facilitate ligation of the first oligonucleotide and the second oligonucleotide to the target polynucleotide, and the helper oligonucleotide is removed following ligation, and thus is not part of the final clamp adapter as viewed in the mate-pair polynucleotide construct, and the helper oligonucleotides comprise a sequence of random nucleotides A, T, C, or G and universal inosine nucleotides that is able to hybridize to the target polynucleotide, thus the helper oligonucleotides facilitate ligation of the first oligonucleotide and second oligonucleotide to the target polynucleotide;
modifying the polynucleotides to make their ends compatible for ligation with the first adapter, then the first adapter is ligated to the modified polynucleotides, then performing an amplification using primers that contain uracil residues and that hybridize within the adapter region, then treating the amplified product with an enzyme that specifically excises uracil bases, which results in the creation of a single nucleotide gap at the location of each uracil in the double-stranded construct, then circularizing the amplified and uracil-specific excising enzyme-treated products to produce circular constructs, each comprising a target polynucleotide, a first adapter, and a nick or gap in the first adapter, and purifying the reaction product of the DNA circularization reaction to remove contaminating non-circularized linear DNA fragments;
performing controlled nick translation to produce nick translation products, each comprising the target polynucleotide, the first adapter, and a nick or gap a first selected distance within the target polynucleotide;
performing 3' branch ligation in the presence of a ligase to ligate a 3' branch adapter to each nick translation product at the nick or gap to produce gap ligation products, wherein the 3' branch adapter comprises a 5' blunt end comprising a 5'-phosphate and a nonligatable 3' end;
performing controlled primer extension to produce primer extension products by hybridizing a primer to the 3' branch adapter of the gap ligation products and extending the primer a second selected distance within the target polynucleotides; and
adding a 5' adapter to a 5' end of the primer extension products to produce a mate pair library, wherein the 5' adapter comprises a 5' blunt end comprising a 5'-phosphate and a nonligatable 3' end, each member of the library comprising: the 5' adapter, a first end portion of a target polynucleotide, the first adapter, a second end portion of the target polynucleotide, and the 3' branch adapter.

2. The method of claim 1 wherein the first adapter comprises a first oligonucleotide and a second oligonucleotide, the method comprising:
ligating respectively the first oligonucleotide and the second oligonucleotide to each end of the target polynucleotides to produce a ligation product; and
ligating the first oligonucleotide and the second oligonucleotide together to produce the circular construct.

3. The method of claim 1 wherein the controlled nick translation is time and temperature controlled nick translation or nick translation controlled by nucleotide amount.

4. The method of claim 1 comprising denaturing the gap ligation products to produce linear single strands and hybridizing the primer to the linear single strands.

5. The method of claim 1 wherein the 3' branch adapter comprises a 5' end comprising a top strand comprising a 5'-phosphate that is ligatable to a 3'-hydroxyl of the nick translation product at the nick or gap and a 3' end that is blocked from ligation.

6. The method of claim 1 wherein the mate pair library is a double-stranded mate pair library, the method comprising producing single strands from the mate pair library and ligating ends of the single strands to produce single-stranded library circles.

7. The method of claim 6 comprising amplifying the library circles by rolling circle replication to produce DNA nanoballs.

8. The method of claim 7 comprising disposing the DNA nanoballs in an array on a solid support to produce a DNA nanoball array.

9. The method of claim 1 wherein the mate pair library is a double-stranded mate pair library, the method comprising:
producing single strands from the mate pair library;
disposing the single strands on a surface of a solid support in an array; and
amplifying the single strands on the array, preferably by bridge PCR, to produce an amplified array.

## Patentansprüche

1. Ein Verfahren zum Herstellen einer Mate-Paar-Polynukleotidbibliothek, das Folgendes beinhaltet:
Bereitstellen einer Vielzahl von doppelsträngigen Zielpolynukleotiden und eines ersten Adapters, ausgewählt aus einem Blasen-Adapter, einem L-Oligo-Adapter oder einem Klammer-Adapter, wobei der Blasen-Adapter aus einem ersten Oligonukleotid und einem zweiten Oligonukleotid gebildet ist, die an ihren 5'- und 3'-Enden teilweise komplementär zueinander sind, sodass das 5'-Ende des ersten Oligonukleotids komplementär zu dem 3'-Ende des zweiten Oligonukleotids ist und das 3'-Ende des ersten Oligonukleotids komplementär zu dem 5'-Ende des zweiten Oligonukleotids ist und die dazwischenliegende Sequenz jedes Oligonukleotids nicht wesentlich komplementär zu dem anderen Oligonukleotid ist, sodass die mittleren Regionen der Oligonukleotide nicht miteinander hybridisieren, wodurch eine "Blase" gebildet wird,
der L-Oligo-Adapter aus einem ersten Oligonukleotid und einem zweiten Oligonukleotid gebildet ist, die teilweise komplementär zueinander sind, sodass das 3'-Ende des ersten Oligonukleotids komplementär zu dem 5'-Ende des zweiten Oligonukleotids ist und die verbleibende 5'-Sequenz des ersten Oligonukleotids nicht wesentlich komplementär zu der verbleibenden 3'-Sequenz des zweiten Oligonukleotids ist, sodass diese Regionen nicht miteinander hybridisieren, wodurch das erste Oligonukleotid eine L-Gestalt bildet, und
der Klammer-Adapter eine 3'-Klammer und eine 5'-Klammer beinhaltet, wobei die 5'-Klammer ein erstes Oligonukleotid und ein erstes Helferoligonukleotid beinhaltet und die 3'-Klammer ein zweites Oligonukleotid und ein zweites Helferoligonukleotid beinhaltet, wobei das erste Helferoligonukleotid an einen Anteil des ersten Oligonukleotids hybridisiert und das zweite Helferoligonukleotid an einen Anteil des zweiten Oligonukleotids hybridisiert, um den Klammer-Adapter zu bilden, um die Ligation des ersten Oligonukleotids und des zweiten Oligonukleotids an das Zielpolynukleotid zu erleichtern, und das Helferoligonukleotid im Anschluss an die Ligation entfernt wird und deshalb nicht Teil des endgültigen Klammer-Adapters bildet, wie dieser in dem Mate-Paar-Polynukleotidkonstrukt zu sehen ist, und die Helferoligonukleotide eine Sequenz von Zufallsnukleotiden A, T, C oder G und Universal-Inosin-Nukleotiden beinhalten, die in der Lage ist, an das Zielpolynukleotid zu hybridisieren, wodurch die Helferoligonukleotide die Ligation des ersten Oligonukleotids und zweiten Oligonukleotid an das Zielpolynukleotid erleichtern;
Modifizieren der Polynukleotide, um ihre Enden für die Ligation mit dem ersten Adapter kompatibel zu machen, danach wird der erste Adapter an die modifizierten Polynukleotide ligiert, danach Durchführen einer Amplifikation unter Verwendung von Primern, die Uracilreste enthalten und die innerhalb der Adapterregion hybridisieren, danach Behandeln des amplifizierten Produkts mit einem Enzym, das spezifisch Uracilbasen exzidiert, was zur Erstellung einer Einzelnukleotidlücke an der Stelle jedes Uracils in dem doppelsträngigen Konstrukt führt, danach Zirkularisieren des amplifizierten und mit uracilspezifischem Exzisionsenzym behandelten Produkts, um ringförmige Konstrukte herzustellen, die jeweils ein Zielpolynukleotid, einen ersten Adapter und eine Bruchstelle oder eine Lücke in dem ersten Adapter beinhalten, und Reinigen des Reaktionsprodukts der DNA-Zirkularisierungsreaktion, um kontaminierende, nicht zirkularisierte lineare DNA-Fragmente zu entfernen;
Durchführen einer gesteuerten Nick-Translation, um Nick-Translationsprodukte herzustellen, die jeweils das Zielpolynukleotid, den ersten Adapter und eine Bruchstelle oder Lücke in einer ersten ausgewählten Distanz innerhalb des Zielnukleotids beinhalten;
Durchführen einer 3'-Verzweigungsligation in Gegenwart einer Ligase, um einen 3'-Verzweigungsadapter an jedes Nick-Translationsprodukt an der Bruchstelle oder Lücke zu ligieren, um Lückenligationsprodukte herzustellen, wobei der 3'-Verzweigungsadapter ein stumpfes 5'-Ende, das ein 5'-Phosphat beinhaltet, und ein nicht ligierbares 3'-Ende beinhaltet;
Durchführen einer gesteuerten Primerverlängerung, um Primerverlängerungsprodukte herzustellen, indem ein Primer an den 3'-Verzweigungsadapter der Lückenligationsprodukte hybridisiert wird und der Primer eine zweite ausgewählte Distanz innerhalb der Zielpolynukleotide verlängert wird; und
Hinzufügen eines 5'-Adapters zu einem 5'-Ende der Primerverlängerungsprodukte, um eine Mate-Paar-Bibliothek herzustellen, wobei der 5'-Adapter ein stumpfes 5'-Ende, das ein 5'-Phosphat beinhaltet, und ein nicht ligierbares 3'-Ende beinhaltet, wobei jedes Mitglied der Bibliothek Folgendes beinhaltet: den 5'-Adapter, einen ersten Endanteil eines Zielpolynukleotids, den ersten Adapter, einen zweiten Endanteil des Zielpolynukleotids und den 3'-Verzweigungsadapter.

2. Verfahren nach Anspruch 1, wobei der erste Adapter ein erstes Oligonukleotid und ein zweites Oligonukleotid beinhaltet, wobei das Verfahren Folgendes beinhaltet:
Ligieren des ersten Oligonukleotids bzw. zweiten Oligonukleotids an jedes Ende der Zielpolynukleotide, um ein Ligationsprodukt herzustellen; und
Ligieren des ersten Oligonukleotids und des zweiten Oligonukleotids miteinander, um das ringförmige Konstrukt zu bilden.

3. Verfahren nach Anspruch 1, wobei die gesteuerte Nick-Translation eine zeit- und temperaturgesteuerte Nick-Translation oder eine durch die Nukleotidmenge gesteuerte Nick-Translation ist.

4. Verfahren nach Anspruch 1, das Folgendes beinhaltet: Denaturieren der Lückenligationsprodukte, um lineare Einzelstränge herzustellen, und Hybridisieren des Primers an die linearen Einzelstränge.

5. Verfahren nach Anspruch 1, wobei der 3'-Verzweigungsadapter Folgendes beinhaltet: ein 5'-Ende, das einen oberen Strang beinhaltet, der ein 5'-Phosphat beinhaltet, das an ein 3'-Hydroxyl des Nick-Translationsprodukts an der Bruchstelle oder Lücke ligierbar ist, und ein 3'-Ende, das von der Ligation blockiert ist.

6. Verfahren nach Anspruch 1, wobei die Mate-Paar-Bibliothek eine doppelsträngige Mate-Paar-Bibliothek ist, wobei das Verfahren das Herstellen von Einzelsträngen aus der Mate-Paar-Bibliothek und das Ligieren von Enden der Einzelstränge beinhaltet, um einzelsträngige Bibliotheksringe zu bilden.

7. Verfahren nach Anspruch 6, das das Amplifizieren der Bibliotheksringe durch Rolling-Circle-Replikation beinhaltet, um DNA-Nanobälle herzustellen.

8. Verfahren nach Anspruch 7, das das Anordnen der DNA-Nanobälle in einem Array auf einem festen Träger beinhaltet, um ein DNA-Nanoball-Array herzustellen.

9. Verfahren nach Anspruch 1, wobei die Mate-Paar-Bibliothek eine doppelsträngige Mate-Paar-Bibliothek ist, wobei das Verfahren Folgendes beinhaltet:
Herstellen von Einzelsträngen aus der Mate-Paar-Bibliothek;
Anordnen der Einzelstränge auf einer Oberfläche eines festen Trägers in einem Array; und
Amplifizieren der Einzelstränge auf dem Array, vorzugsweise durch Brücken-PCR, um ein amplifiziertes Array herzustellen.

## Revendications

1. Procédé de réalisation d'une banque de polynucléotides de type mate pair comprenant :
la fourniture d'une pluralité de polynucléotides cibles double brin et d'un premier adaptateur choisi parmi un adaptateur à bulle, un adapteur L-oligo ou un adapteur à attache, dans lequel l'adaptateur à bulle est formé d'un premier oligonucléotide et d'un deuxième oligonucléotide partiellement complémentaires l'un de l'autre au niveau de leurs extrémités 5' et 3', de sorte que l'extrémité 5' du premier oligonucléotide est complémentaire de l'extrémité 3' du deuxième oligonucléotide, et l'extrémité 3' du premier oligonucléotide est complémentaire de l'extrémité 5' du deuxième oligonucléotide, et la séquence d'intervention de chaque oligonucléotide n'est pas substantiellement complémentaire de l'autre oligonucléotide, de sorte que les régions médianes des oligonucléotides ne s'hybrident pas l'une avec l'autre, formant ainsi une « bulle »,
l'adaptateur L-oligo est formé d'un premier oligonucléotide et d'un deuxième oligonucléotide partiellement complémentaires l'un de l'autre, de sorte que l'extrémité 3' du premier oligonucléotide est complémentaire de l'extrémité 5' du deuxième oligonucléotide, et la séquence 5' restante du premier oligonucléotide n'est pas substantiellement complémentaire de la séquence 3' restante du deuxième oligonucléotide, de sorte que ces régions ne s'hybrident pas l'une avec l'autre, ainsi le premier oligonucléotide a une forme en L, et
l'adaptateur à attache comprend une attache 3' et une attache 5', dans lequel l'attache 5' comprend un premier oligonucléotide et un premier oligonucléotide auxiliaire, et l'attache 3' comprend un deuxième oligonucléotide et un deuxième oligonucléotide auxiliaire, le premier oligonucléotide auxiliaire s'hybride avec une partie du premier oligonucléotide, et le deuxième oligonucléotide auxiliaire s'hybride avec une partie du deuxième oligonucléotide pour former l'adaptateur à attache afin de faciliter la ligature du premier oligonucléotide et du deuxième oligonucléotide avec le polynucléotide cible, et l'oligonucléotide auxiliaire est éliminé après la ligature, et ainsi ne fait pas partie de l'adaptateur à attache final comme on le voit dans le produit de recombinaison de polynucléotides en mate pair, et les oligonucléotides auxiliaires comprennent une séquence de nucléotides aléatoires A, T, C, ou G et des nucléotides d'inosine universels qui sont capables de s'hybrider avec le polynucléotide cible, ainsi les oligonucléotides auxiliaires facilitent la ligature du premier oligonucléotide et du deuxième oligonucléotide avec le polynucléotide cible ;
la modification des polynucléotides pour rendre leurs extrémités compatibles pour la ligature avec le premier adaptateur, puis le premier adaptateur est ligaturé aux polynucléotides modifiés, puis la réalisation d'une amplification en utilisant des amorces qui contiennent des résidus d'uracile et qui s'hybrident au sein de la région d'adaptateur, puis le traitement du produit amplifié avec une enzyme qui excise spécifiquement les bases uracile, ce qui entraîne la création d'une brèche de nucléotide unique au niveau de l'emplacement de chaque uracile dans le produit de recombinaison double-brin, puis la circularisation des produits amplifiés et traités avec une enzyme d'excision spécifique de l'uracile pour produire des produits de recombinaison circulaires, chacune comprenant un polynucléotide cible, un premier adaptateur, et une coupure ou une brèche dans le premier adaptateur, et la purification du produit réactionnel de la réaction de circularisation d'ADN pour éliminer les fragments d'ADN linéaires non circularisés contaminants ;
la réalisation d'une translation de coupure contrôlée pour produire des produits de translation de coupure, chacun comprenant le polynucléotide cible, le premier adaptateur, et une coupure ou une brèche sur une première distance sélectionnée au sein du polynucléotide cible ;
la réalisation d'une ligature de branche 3' en présence d'une ligase pour ligaturer un adaptateur de branche 3' à chaque produit de translation de coupure au niveau de la coupure ou de la brèche pour produire des produits de ligature de brèche, dans lequel l'adaptateur de branche 3' comprend une extrémité émoussée 5' comprenant un phosphate en 5' et une extrémité 3' ne pouvant pas être ligaturée ;
la réalisation d'une extension d'amorce contrôlée pour produire des produits d'extension d'amorce par l'hybridation d'une amorce à l'adaptateur de branche 3' des produits de ligature de brèche et l'extension de l'amorce sur une deuxième distance sélectionnée au sein des polynucléotides cibles ; et
l'ajout d'un adaptateur 5' à une extrémité 5' des produits d'extension d'amorce pour produire une banque de type mate pair, dans lequel l'adaptateur 5' comprend une extrémité émoussée 5' comprenant un phosphate en 5' et une extrémité 3' ne pouvant pas être ligaturée, chaque élément de la banque comprenant : l'adaptateur 5', une première partie d'extrémité d'un polynucléotide cible, le premier adaptateur, une deuxième partie d'extrémité du polynucléotide cible, et l'adaptateur de branche 3'.

2. Procédé selon la revendication 1 dans lequel le premier adaptateur comprend un premier oligonucléotide et un deuxième oligonucléotide, le procédé comprenant :
la ligature respective du premier oligonucléotide et du deuxième oligonucléotide à chaque extrémité des polynucléotides cibles pour produire un produit de ligature ; et
la ligature du premier oligonucléotide et du deuxième oligonucléotide ensemble pour produire le produit de recombinaison circulaire.

3. Procédé selon la revendication 1 dans lequel la translation de coupure contrôlée est une translation de coupure contrôlée par le temps et la température ou une translation de coupure contrôlée par la quantité de nucléotide.

4. Procédé selon la revendication 1 comprenant la dénaturation des produits de ligature de brèche pour produire de simples brins linéaires et l'hybridation de l'amorce aux simples brins linéaires.

5. Procédé selon la revendication 1 dans lequel l'adaptateur de branche 3' comprend une extrémité 5' comprenant un brin supérieur comprenant un phosphate en 5' qui peut être ligaturé à un hydroxyle en 3' du produit de translation de coupure au niveau de la coupure ou de la brèche et une extrémité 3' dont la ligature est bloquée.

6. Procédé selon la revendication 1 dans lequel la banque de type mate pair est une banque de type mate-pair double brin, le procédé comprenant la production de simples brins de la banque de type mate pair et la ligature des extrémités des simples brins pour produire des cercles de banque simples brins.

7. Procédé selon la revendication 6 comprenant l'amplification des cercles de banque par réplication en cercle roulant pour produire des nanobilles d'ADN.

8. Procédé selon la revendication 7 comprenant la disposition des nanobilles d'ADN dans un réseau sur un support solide pour produire un réseau de nanobilles d'ADN.

9. Procédé selon la revendication 1 dans lequel la banque de type mate pair est une banque de type mate pair double brin, le procédé comprenant :
la production de simples brins de la banque de type mate pair ;
la disposition des simples brins sur une surface d'un support solide en un réseau ; et
l'amplification des simples brins sur le réseau, préférablement par PCR en pont, pour produire un réseau amplifié.
